# EUROPEAN PATENT APPLICATION

(11) **EP 2 251 039 A2**
(43) Date of publication of application: **17.11.2010**
(21) Application number: 10168680.6
(22) Date of filing: 28.05.2004
(51) Int. Cl.: A61K 48/00, C12N 15/113, A61K 31/7105

(54) **Oligo double-stranded rna inhibiting the expression of bcl-2 and pharmaceutical composition containing the same**

(30) Priority: 30.05.2003 JP 2003154668; 12.11.2003 JP 2003382639; 31.03.2004 JP 2004104036
(62) Divisional of application: 04745414.5
(71) Applicant: Nippon Shinyaku Co., Ltd., Kyoto-shi, Kyoto 601-8550 (JP)
(72) Inventor: Yano, Junichi, Nara 630-8105 (JP); Hirabayashi, Kazuko, Kyoto 616-8105 (JP); Nakagawa, Shinichiro, Tokyo 170-0003 (JP)
(74) Representative: Vossius & Partner

(57) **Abstract**

The present invention relates to oligo double-stranded RNAs for use in knockdown of the expression of Bcl-2 protein known as a suppressor for apoptosis, and pharmaceutical compositions containing them. It is known that Bcl-2 protein is over-expressed in diseases such as cancer, and by this over-expression, the growth of cancer cells is continued and the drug resistance to anti-cancer agents is caused. The present invention provides highly active oligo double-stranded RNAs cleaving Bcl-2 mRNA and further provides pharmaceutical compositions comprising a complex of the oligo double-stranded RNA and a suitable carrier for inhibiting the expression of Bcl-2 protein.

SEQUENCE LISTING

<110> Nippon Shinyaku Co., LTD.

<120> Oligo double-stranded RNA inhbiting the expression of Bcl-2 and
      pharmaceutical composition containing the same.

<130> L3014 EP/1 S3

<150> JP 2003-154668
<151> 2003-05-30

<150> JP 2003-382639
<151> 2003-11-12

<150> JP 2004-104036
<151> 2004-03-31

<160> 334

<210> 1
<211> 21
<212> DNA
<213> Artificial sequence

<220>
<221> source
<223> /note= "Description of artificial sequence: synthesized DNA/RNA; sense
      strand of B002, wherein 2 nucleotides of 3'-terminus are constructed with
      DNA, and the rest are RNA."

<400> 1
uggcgcacgc ugggagaact t                                                21

<210> 2
<211> 21
<212> DNA
<213> Artificial sequence

<220>
<221> source
<223> /note= "Description of artificial sequence: synthesized DNA/RNA; sense
      strand of B010, wherein 2 nucleotides of 3'-terminus are constructed with
      DNA, and the rest are RNA."

<400> 2
gcugggagaa cgggguacgt t                                                21

<210> 3
<211> 21
<212> DNA
<213> Artificial sequence

<220>
<221> source
<223> /note= "Description of artificial sequence: synthesized DNA/RNA; sense
      strand of B028, wherein 2 nucleotides of 3'-terminus are constructed with
      DNA, and the rest are RNA."

<400> 3
gauaaccggg agauagugat t                                                21

<210> 4
<211> 21
<212> DNA
<213> Artificial sequence

<220>
<221> source
<223> /note= "Description of artificial sequence: synthesized DNA/RNA; sense
      strand of B031, wherein 2 nucleotides of 3'-terminus are constructed with
      DNA, and the rest are RNA."

<400> 4
aaccgggaga uagugaugat t                                                21

<210> 5
<211> 21
<212> DNA
<213> Artificial sequence

<220>
<221> source
<223> /note= "Description of artificial sequence: synthesized DNA/RNA; sense
      strand of B034, wherein 2 nucleotides of 3'-terminus are constructed with
      DNA, and the rest are RNA."

<400> 5
cgggagauag ugaugaagut t                                                21

<210> 6
<211> 21
<212> DNA
<213> Artificial sequence

<220>
<221> source
<223> /note= "Description of artificial sequence: synthesized DNA/RNA; sense
      strand of B037, wherein 2 nucleotides of 3'-terminus are constructed with
      DNA, and the rest are RNA."

<400> 6
gagauaguga ugaaguacat t                                                21

<210> 7
<211> 21
<212> DNA
<213> Artificial sequence

<220>
<221> source
<223> /note= "Description of artificial sequence: synthesized DNA/RNA; sense
      strand of B039, wherein 2 nucleotides of 3'-terminus are constructed with
      DNA, and the rest are RNA."

<400> 7
gauagugaug aaguacauct t                                                21

<210> 8
<211> 21
<212> DNA
<213> Artificial sequence

<220>
<221> source
<223> /note= "Description of artificial sequence: synthesized DNA/RNA; sense
      strand of B040, wherein 2 nucleotides of 3'-terminus are constructed with
      DNA, and the rest are RNA."

<400> 8
auagugauga aguacaucct t                                                21

<210> 9
<211> 21
<212> DNA
<213> Artificial sequence

<220>
<221> source
<223> /note= "Description of artificial sequence: synthesized DNA/RNA; sense
      strand of B041, wherein 2 nucleotides of 3'-terminus are constructed with
      DNA, and the rest are RNA."

<400> 9
uagugaugaa guacauccat t                                                21

<210> 10
<211> 21
<212> DNA
<213> Artificial sequence

<220>
<221> source
<223> /note= "Description of artificial sequence: synthesized DNA/RNA; sense
      strand of B042, wherein 2 nucleotides of 3'-terminus are constructed with
      DNA, and the rest are RNA."

<400> 10
agugaugaag uacauccaut t                                                21

<210> 11
<211> 21
<212> DNA
<213> Artificial sequence

<220>
<221> source
<223> /note= "Description of artificial sequence: synthesized DNA/RNA; sense
      strand of B043, wherein 2 nucleotides of 3'-terminus are constructed with
      DNA, and the rest are RNA."

<400> 11
gugaugaagu acauccaaut t                                                21

<210> 12
<211> 21
<212> DNA

<213> Artificial sequence

<220>
<221> source
<223> /note= "Description of artificial sequence: synthesized DNA/RNA; sense
      strand of B044, wherein 2 nucleotides of 3'-terminus are constructed with
      DNA, and the rest are RNA."

<400> 12
ugaugaagua cauccauuat t                                                21

<210> 13
<211> 21
<212> DNA
<213> Artificial sequence

<220>
<221> source
<223> /note= "Description of artificial sequence: synthesized DNA/RNA; sense
      strand of B045, wherein 2 nucleotides of 3'-terminus are constructed with
      DNA, and the rest are RNA."

<400> 13
gaugaaguac auccauuaut t                                                21

<210> 14
<211> 21
<212> DNA
<213> Artificial sequence

<220>
<221> source
<223> /note= "Description of artificial sequence: synthesized DNA/RNA; sense
      strand of B046, wherein 2 nucleotides of 3'-terminus are constructed with
      DNA, and the rest are RNA."

<400> 14
augaaguaca uccauuauat t                                                21

<210> 15
<211> 21
<212> DNA
<213> Artificial sequence

<220>
<221> source
<223> /note= "Description of artificial sequence: synthesized DNA/RNA; sense
      strand of B055, wherein 2 nucleotides of 3'-terminus are constructed with
      DNA, and the rest are RNA."

<400> 15
auccauuaua agcugucgct t                                                21

<210> 16
<211> 21
<212> DNA
<213> Artificial sequence

<220>

<221> source
<223> /note= "Description of artificial sequence: synthesized DNA/RNA; sense
      strand of B065, wherein 2 nucleotides of 3'-terminus are constructed with
      DNA, and the rest are RNA."

<400> 16
agcugucgca gaggggcuat t                                                21

<210> 17
<211> 21
<212> DNA
<213> Artificial sequence

<220>
<221> source
<223> /note= "Description of artificial sequence: synthesized DNA/RNA; sense
      strand of B073, wherein 2 nucleotides of 3'-terminus are constructed with
      DNA, and the rest are RNA."

<400> 17
cagaggggcu acgagugggt t                                                21

<210> 18
<211> 21
<212> DNA
<213> Artificial sequence

<220>
<221> source
<223> /note= "Description of artificial sequence: synthesized DNA/RNA; sense
      strand of B084, wherein 2 nucleotides of 3'-terminus are constructed with
      DNA, and the rest are RNA."

<400> 18
cgagugggau gcgggagaut t                                                21

<210> 19
<211> 21
<212> DNA
<213> Artificial sequence

<220>
<221> source
<223> /note= "Description of artificial sequence: synthesized DNA/RNA; sense
      strand of B136, wherein 2 nucleotides of 3'-terminus are constructed with
      DNA, and the rest are RNA."

<400> 19
ccgggcaucu ucuccuccct t                                                21

<210> 20
<211> 21
<212> DNA
<213> Artificial sequence

<220>
<221> source

<223> /note= "Description of artificial sequence: synthesized DNA/RNA; sense
      strand of B172, wherein 2 nucleotides of 3'-terminus are constructed with
      DNA, and the rest are RNA."

<400> 20
cauccagccg caucccgggt t                                                21

<210> 21
<211> 21
<212> DNA
<213> Artificial sequence

<220>
<221> source
<223> /note= "Description of artificial sequence: synthesized DNA/RNA; sense
      strand of B199, wherein 2 nucleotides of 3'-terminus are constructed with
      DNA, and the rest are RNA."

<400> 21
gccaggaccu cgccgcugct t                                                21

<210> 22
<211> 21
<212> DNA
<213> Artificial sequence

<220>
<221> source
<223> /note= "Description of artificial sequence: synthesized DNA/RNA; sense
      strand of B207, wherein 2 nucleotides of 3'-terminus are constructed with
      DNA, and the rest are RNA."

<400> 22
cucgccgcug cagaccccgt t                                                21

<210> 23
<211> 21
<212> DNA
<213> Artificial sequence

<220>
<221> source
<223> /note= "Description of artificial sequence: synthesized DNA/RNA; sense
      strand of B253, wherein 2 nucleotides of 3'-terminus are constructed with
      DNA, and the rest are RNA."

<400> 23
gcgcucagcc cggugccact t                                                21

<210> 24
<211> 21
<212> DNA
<213> Artificial sequence

<220>
<221> source
<223> /note= "Description of artificial sequence: synthesized DNA/RNA; sense
      strand of B262, wherein 2 nucleotides of 3'-terminus are constructed with
      DNA, and the rest are RNA."

<400> 24
ccggugccac cuguggucct t                                               21

<210> 25
<211> 21
<212> DNA
<213> Artificial sequence

<220>
<221> source
<223> /note= "Description of artificial sequence: synthesized DNA/RNA; sense
      strand of B280, wherein 2 nucleotides of 3'-terminus are constructed with
      DNA, and the rest are RNA."

<400> 25
caccugaccc uccgccaggt t                                               21

<210> 26
<211> 21
<212> DNA
<213> Artificial sequence

<220>
<221> source
<223> /note= "Description of artificial sequence: synthesized DNA/RNA; sense
      strand of B325, wherein 2 nucleotides of 3'-terminus are constructed with
      DNA, and the rest are RNA."

<400> 26
cgccgcgacu ucgccgagat t                                               21

<210> 27
<211> 21
<212> DNA
<213> Artificial sequence

<220>
<221> source
<223> /note= "Description of artificial sequence: synthesized DNA/RNA; sense
      strand of B352, wherein 2 nucleotides of 3'-terminus are constructed with
      DNA, and the rest are RNA."

<400> 27
cagcugcacc ugacgcccut t                                               21

<210> 28
<211> 21
<212> DNA
<213> Artificial sequence

<220>
<221> source
<223> /note= "Description of artificial sequence: synthesized DNA/RNA; sense
      strand of B397, wherein 2 nucleotides of 3'-terminus are constructed with
      DNA, and the rest are RNA."

<400> 28
gugguggagg agcucuucat t                                               21

<210> 29
<211> 21
<212> DNA
<213> Artificial sequence

<220>
<221> source
<223> /note= "Description of artificial sequence: synthesized DNA/RNA; sense
      strand of B433, wherein 2 nucleotides of 3'-terminus are constructed with
      DNA, and the rest are RNA."

<400> 29
gggaggauug uggccuucut t                                                21

<210> 30
<211> 21
<212> DNA
<213> Artificial sequence

<220>
<221> source
<223> /note= "Description of artificial sequence: synthesized DNA/RNA; sense
      strand of B436, wherein 2 nucleotides of 3'-terminus are constructed with
      DNA, and the rest are RNA."

<400> 30
aggauugugg ccuucuuugt t                                                21

<210> 31
<211> 21
<212> DNA
<213> Artificial sequence

<220>
<221> source
<223> /note= "Description of artificial sequence: synthesized DNA/RNA; sense
      strand of B439, wherein 2 nucleotides of 3'-terminus are constructed with
      DNA, and the rest are RNA."

<400> 31
auuguggccu ucuuugagut t                                                21

<210> 32
<211> 21
<212> DNA
<213> Artificial sequence

<220>
<221> source
<223> /note= "Description of artificial sequence: synthesized DNA/RNA; sense
      strand of B442, wherein 2 nucleotides of 3'-terminus are constructed with
      DNA, and the rest are RNA."

<400> 32
guggccuucu uugaguucgt t                                                21

<210> 33
<211> 21

<212> DNA
<213> Artificial sequence

<220>
<221> source
<223> /note= "Description of artificial sequence: synthesized DNA/RNA; sense
      strand of B451, wherein 2 nucleotides of 3'-terminus are constructed with
      DNA, and the rest are RNA."

<400> 33
uuugaguucg guggggucat t                                                21

<210> 34
<211> 21
<212> DNA
<213> Artificial sequence

<220>
<221> source
<223> /note= "Description of artificial sequence: synthesized DNA/RNA; sense
      strand of B463, wherein 2 nucleotides of 3'-terminus are constructed with
      DNA, and the rest are RNA."

<400> 34
ggggucaugu guguggagat t                                                21

<210> 35
<211> 21
<212> DNA
<213> Artificial sequence

<220>
<221> source
<223> /note= "Description of artificial sequence: synthesized DNA/RNA; sense
      strand of B466, wherein 2 nucleotides of 3'-terminus are constructed with
      DNA, and the rest are RNA."

<400> 35
gucaugugug uggagagcgt t                                                21

<210> 36
<211> 21
<212> DNA
<213> Artificial sequence

<220>
<221> source
<223> /note= "Description of artificial sequence: synthesized DNA/RNA; sense
      strand of B469, wherein 2 nucleotides of 3'-terminus are constructed with
      DNA, and the rest are RNA."

<400> 36
augugugugg agagcgucat t                                                21

<210> 37
<211> 21
<212> DNA
<213> Artificial sequence

<220>
<221> source
<223> /note= "Description of artificial sequence: synthesized DNA/RNA; sense
      strand of B472, wherein 2 nucleotides of 3'-terminus are constructed with
      DNA, and the rest are RNA."

<400> 37
uguguggaca gcgucaacct t                                                21

<210> 38
<211> 21
<212> DNA
<213> Artificial sequence

<220>
<221> source
<223> /note= "Description of artificial sequence: synthesized DNA/RNA; sense
      strand of B475, wherein 2 nucleotides of 3'-terminus are constructed with
      DNA, and the rest are RNA."

<400> 38
guggagagcg ucaaccgggt t                                                21

<210> 39
<211> 21
<212> DNA
<213> Artificial sequence

<220>
<221> source
<223> /note= "Description of artificial sequence: synthesized DNA/RNA; sense
      strand of B478, wherein 2 nucleotides of 3'-terminus are constructed with
      DNA, and the rest are RNA."

<400> 39
gagagcguca accgggagat t                                                21

<210> 40
<211> 21
<212> DNA
<213> Artificial sequence

<220>
<221> source
<223> /note= "Description of artificial sequence: synthesized DNA/RNA; sense
      strand of B516, wherein 2 nucleotides of 3'-terminus are constructed with
      DNA, and the rest are RNA."

<400> 40
caucgcccug uggaugacut t                                                21

<210> 41
<211> 21
<212> DNA
<213> Artificial sequence

<220>
<221> source

<223> /note= "Description of artificial sequence: synthesized DNA/RNA; sense
      strand of B523, wherein 2 nucleotides of 3'-terminus are constructed with
      DNA, and the rest are RNA."

<400> 41
cuguggauga cugaguacct t                                                21

<210> 42
<211> 21
<212> DNA
<213> Artificial sequence

<220>
<221> source
<223> /note= "Description of artificial sequence: synthesized DNA/RNA; sense
      strand of B531, wherein 2 nucleotides of 3'-terminus are constructed with
      DNA, and the rest are RNA."

<400> 42
gacugaguac cugaaccggt t                                                21

<210> 43
<211> 21
<212> DNA
<213> Artificial sequence

<220>
<221> source
<223> /note= "Description of artificial sequence: synthesized DNA/RNA; sense
      strand of B533, wherein 2 nucleotides of 3'-terminus are constructed with
      DNA, and the rest are RNA."

<400> 43
cugaguaccu gaaccggcat t                                                21

<210> 44
<211> 21
<212> DNA
<213> Artificial sequence

<220>
<221> source
<223> /note= "Description of artificial sequence: synthesized DNA/RNA; sense
      strand of B534, wherein 2 nucleotides of 3'-terminus are constructed with
      DNA, and the rest are RNA."

<400> 44
ugaguaccug aaccggcact t                                                21

<210> 45
<211> 21
<212> DNA
<213> Artificial sequence

<220>
<221> source
<223> /note= "Description of artificial sequence: synthesized DNA/RNA; sense
      strand of B536, wherein 2 nucleotides of 3'-terminus are constructed with
      DNA, and the rest are RNA."

<400> 45
aguaccugaa ccggcaccut t                                                21

<210> 46
<211> 21
<212> DNA
<213> Artificial sequence

<220>
<221> source
<223> /note= "Description of artificial sequence: synthesized DNA/RNA; sense
      strand of B539, wherein 2 nucleotides of 3'-terminus are constructed with
      DNA, and the rest are RNA."

<400> 46
accugaaccg gcaccugcat t                                                21

<210> 47
<211> 21
<212> DNA
<213> Artificial sequence

<220>
<221> source
<223> /note= "Description of artificial sequence: synthesized DNA/RNA; sense
      strand of B543, wherein 2 nucleotides of 3'-terminus are constructed with
      DNA, and the rest are RNA."

<400> 47
gaaccggcac cugcacacct t                                                21

<210> 48
<211> 21
<212> DNA
<213> Artificial sequence

<220>
<221> source
<223> /note= "Description of artificial sequence: synthesized DNA/RNA; sense
      strand of B545, wherein 2 nucleotides of 3'-terminus are constructed with
      DNA, and the rest are RNA."

<400> 48
accggcaccu gcacaccugt t                                                21

<210> 49
<211> 21
<212> DNA
<213> Artificial sequence

<220>
<221> source
<223> /note= "Description of artificial sequence: synthesized DNA/RNA; sense
      strand of B558, wherein 2 nucleotides of 3'-terminus are constructed with
      DNA, and the rest are RNA."

<400> 49
caccuggauc caggauaact t                                                21

<210> 50
<211> 21
<212> DNA
<213> Artificial sequence

<220>
<221> source
<223> /note= "Description of artificial sequence: synthesized DNA/RNA; sense
      strand of B576, wherein 2 nucleotides of 3'-terminus are constructed with
      DNA, and the rest are RNA."

<400> 50
cggaggcugg gaugccuuut t                                                21

<210> 51
<211> 21
<212> DNA
<213> Artificial sequence

<220>
<221> source
<223> /note= "Description of artificial sequence: synthesized DNA/RNA; sense
      strand of B586, wherein 2 nucleotides of 3'-terminus are constructed with
      DNA, and the rest are RNA."

<400> 51
gaugccuuug uggaacugut t                                                21

<210> 52
<211> 21
<212> DNA
<213> Artificial sequence

<220>
<221> source
<223> /note= "Description of artificial sequence: synthesized DNA/RNA; sense
      strand of B595, wherein 2 nucleotides of 3'-terminus are constructed with
      DNA, and the rest are RNA."

<400> 52
guggaacugu acggccccat t                                                21

<210> 53
<211> 21
<212> DNA
<213> Artificial sequence

<220>
<221> source
<223> /note= "Description of artificial sequence: synthesized DNA/RNA; sense
      strand of B604, wherein 2 nucleotides of 3'-terminus are constructed with
      DNA, and the rest are RNA."

<400> 53
uacggcccca gcaugcggct t                                                21

<210> 54
<211> 21

<212> DNA
<213> Artificial sequence

<220>
<221> source
<223> /note= "Description of artificial sequence: synthesized DNA/RNA; sense
      strand of B613, wherein 2 nucleotides of 3'-terminus are constructed with
      DNA, and the rest are RNA."

<400> 54
agcaugcggc cucuguuugt t                                                21

<210> 55
<211> 21
<212> DNA
<213> Artificial sequence

<220>
<221> source
<223> /note= "Description of artificial sequence: synthesized DNA/RNA; sense
      strand of B614, wherein 2 nucleotides of 3'-terminus are constructed with
      DNA, and the rest are RNA."

<400> 55
gcaugcggcc ucuguuugat t                                                21

<210> 56
<211> 21
<212> DNA
<213> Artificial sequence

<220>
<221> source
<223> /note= "Description of artificial sequence: synthesized DNA/RNA; sense
      strand of B615, wherein 2 nucleotides of 3'-terminus are constructed with
      DNA, and the rest are RNA."

<400> 56
caugcggccu cuguuugaut t                                                21

<210> 57
<211> 21
<212> DNA
<213> Artificial sequence

<220>
<221> source
<223> /note= "Description of artificial sequence: synthesized DNA/RNA; sense
      strand of B616, wherein 2 nucleotides of 3'-terminus are constructed with
      DNA, and the rest are RNA."

<400> 57
augcggccuc uguuugauut t                                                21

<210> 58
<211> 21
<212> DNA
<213> Artificial sequence

<220>
<221> source
<223> /note= "Description of artificial sequence: synthesized DNA/RNA; sense
      strand of B619, wherein 2 nucleotides of 3'-terminus are constructed with
      DNA, and the rest are RNA."

<400> 58
cggccucugu uugauuucut t                                                21

<210> 59
<211> 21
<212> DNA
<213> Artificial sequence

<220>
<221> source
<223> /note= "Description of artificial sequence: synthesized DNA/RNA; sense
      strand of B622, wherein 2 nucleotides of 3'-terminus are constructed with
      DNA, and the rest are RNA."

<400> 59
ccucuguuug auuucuccut t                                                21

<210> 60
<211> 21
<212> DNA
<213> Artificial sequence

<220>
<221> source
<223> /note= "Description of artificial sequence: synthesized DNA/RNA; sense
      strand of B625, wherein 2 nucleotides of 3'-terminus are constructed with
      DNA, and the rest are RNA."

<400> 60
cuguuugauu ucuccuggct t                                                21

<210> 61
<211> 21
<212> DNA
<213> Artificial sequence

<220>
<221> source
<223> /note= "Description of artificial sequence: synthesized DNA/RNA; sense
      strand of B628, wherein 2 nucleotides of 3'-terminus are constructed with
      DNA, and the rest are RNA."

<400> 61
uuugauuucu ccuggcugut t                                                21

<210> 62
<211> 21
<212> DNA
<213> Artificial sequence

<220>
<221> source

<223> /note= "Description of artificial sequence: synthesized DNA/RNA; sense
      strand of B631, wherein 2 nucleotides of 3'-terminus are constructed with
      DNA, and the rest are RNA."

<400> 62
gauuucuccu ggcugucuct t                                                21

<210> 63
<211> 21
<212> DNA
<213> Artificial sequence

<220>
<221> source
<223> /note= "Description of artificial sequence: synthesized DNA/RNA; sense
      strand of B634, wherein 2 nucleotides of 3'-terminus are constructed with
      DNA, and the rest are RNA."

<400> 63
uucuccuggc ugucucugat t                                                21

<210> 64
<211> 21
<212> DNA
<213> Artificial sequence

<220>
<221> source
<223> /note= "Description of artificial sequence: synthesized DNA/RNA; sense
      strand of B636, wherein 2 nucleotides of 3'-terminus are constructed with
      DNA, and the rest are RNA."

<400> 64
cuccuggcug ucucugaagt t                                                21

<210> 65
<211> 21
<212> DNA
<213> Artificial sequence

<220>
<221> source
<223> /note= "Description of artificial sequence: synthesized DNA/RNA; sense
      strand of B642, wherein 2 nucleotides of 3'-terminus are constructed with
      DNA, and the rest are RNA."

<400> 65
gcugucucug aagacucugt t                                                21

<210> 66
<211> 21
<212> DNA
<213> Artificial sequence

<220>
<221> source
<223> /note= "Description of artificial sequence: synthesized DNA/RNA; sense
      strand of B649, wherein 2 nucleotides of 3'-terminus are constructed with
      DNA, and the rest are RNA."

<400> 66
cugaagacuc ugcucaguut t                                                21

<210> 67
<211> 21
<212> DNA
<213> Artificial sequence

<220>
<221> source
<223> /note= "Description of artificial sequence: synthesized DNA/RNA; sense
      strand of B654, wherein 2 nucleotides of 3'-terminus are constructed with
      DNA, and the rest are RNA."

<400> 67
gacucugcuc aguuuggcct t                                                21

<210> 68
<211> 21
<212> DNA
<213> Artificial sequence

<220>
<221> source
<223> /note= "Description of artificial sequence: synthesized DNA/RNA; sense
      strand of B658, wherein 2 nucleotides of 3'-terminus are constructed with
      DNA, and the rest are RNA."

<400> 68
cugcucaguu uggcccuggt t                                                21

<210> 69
<211> 21
<212> DNA
<213> Artificial sequence

<220>
<221> source
<223> /note= "Description of artificial sequence: synthesized DNA/RNA; sense
      strand of B667, wherein 2 nucleotides of 3'-terminus are constructed with
      DNA, and the rest are RNA."

<400> 69
uuggcccugg ugggagcuut t                                                21

<210> 70
<211> 21
<212> DNA
<213> Artificial sequence

<220>
<221> source
<223> /note= "Description of artificial sequence: synthesized DNA/RNA; sense
      strand of B676, wherein 2 nucleotides of 3'-terminus are constructed with
      DNA, and the rest are RNA."

<400> 70
gugggagcuu gcaucaccct t                                                21

<210> 71
<211> 21
<212> DNA
<213> Artificial sequence

<220>
<221> source
<223> /note= "Description of artificial sequence: synthesized DNA/RNA; sense
      strand of B697, wherein 2 nucleotides of 3'-terminus are constructed with
      DNA, and the rest are RNA."

<400> 71
ggugccuauc ugggccacat t                                                21

<210> 72
<211> 21
<212> DNA
<213> Artificial sequence

<220>
<221> source
<223> /note= "Description of artificial sequence: synthesized DNA/RNA; sense
      strand of B700, wherein 2 nucleotides of 3'-terminus are constructed with
      DNA, and the rest are RNA."

<400> 72
gccuaucugg gccacaagut t                                                21

<210> 73
<211> 21
<212> DNA
<213> Artificial sequence

<220>
<221> source
<223> /note= "Description of artificial sequence: synthesized DNA/RNA; sense
      strand of B703, wherein 2 nucleotides of 3'-terminus are constructed with
      DNA, and the rest are RNA."

<400> 73
uaucugggcc acaagugaat t                                                21

<210> 74
<211> 21
<212> DNA
<213> Artificial sequence

<220>
<221> source
<223> /note= "Description of artificial sequence: synthesized DNA/RNA; sense
      strand of B706, wherein 2 nucleotides of 3'-terminus are constructed with
      DNA, and the rest are RNA."

<400> 74
cugggccaca agugaaguct t                                                21

<210> 75
<211> 21

<212> DNA
<213> Artificial sequence

<220>
<221> source
<223> /note= "Description of artificial sequence: synthesized DNA/RNA; sense
      strand of B709, wherein 2 nucleotides of 3'-terminus are constructed with
      DNA, and the rest are RNA."

<400> 75
ggccacaagu gaagucaact t                                                21

<210> 76
<211> 21
<212> DNA
<213> Artificial sequence

<220>
<221> source
<223> /note= "Description of artificial sequence: synthesized DNA/RNA; sense
      strand of B712, wherein 2 nucleotides of 3'-terminus are constructed with
      DNA, and the rest are RNA."

<400> 76
cacaagugaa gucaacaugt t                                                21

<210> 77
<211> 21
<212> DNA
<213> Artificial sequence

<220>
<221> source
<223> /note= "Description of artificial sequence: synthesized DNA/RNA; sense
      strand of B717, wherein 2 nucleotides of 3'-terminus are constructed with
      DNA, and the rest are RNA."

<400> 77
gugaagucaa caugccugct t                                                21

<210> 78
<211> 21
<212> DNA
<213> Artificial sequence

<220>
<221> source
<223> /note= "Description of artificial sequence: synthesized DNA/RNA; sense
      strand of B719, wherein 2 nucleotides of 3'-terminus are constructed with
      DNA, and the rest are RNA."

<400> 78
gaagucaaca ugccugccct t                                                21

<210> 79
<211> 21
<212> DNA
<213> Artificial sequence

<220>
<221> source
<223> /note= "Description of artificial sequence: synthesized DNA/RNA; sense
      strand of B721, wherein 2 nucleotides of 3'-terminus are constructed with
      DNA, and the rest are RNA."

<400> 79
agucaacaug ccugccccat t                                                21

<210> 80
<211> 21
<212> DNA
<213> Artificial sequence

<220>
<221> source
<223> /note= "Description of artificial sequence: synthesized DNA/RNA; sense
      strand of B724, wherein 2 nucleotides of 3'-terminus are constructed with
      DNA, and the rest are RNA."

<400> 80
caacaugccu gccccaaact t                                                21

<210> 81
<211> 21
<212> DNA
<213> Artificial sequence

<220>
<221> source
<223> /note= "Description of artificial sequence: synthesized DNA/RNA; sense
      strand of B727, wherein 2 nucleotides of 3'-terminus are constructed with
      DNA, and the rest are RNA."

<400> 81
caugccugcc ccaaacaaat t                                                21

<210> 82
<211> 21
<212> DNA
<213> Artificial sequence

<220>
<221> source
<223> /note= "Description of artificial sequence: synthesized DNA/RNA; antisense
      strand of B002, wherein 2 nucleotides of 3'-terminus are constructed with
      DNA, and the rest are RNA."

<400> 82
guucucccag cgugcgccat t                                                21

<210> 83
<211> 21
<212> DNA
<213> Artificial sequence

<220>
<221> source

<223> /note= "Description of artificial sequence: synthesized DNA/RNA; antisense
      strand of B010, wherein 2 nucleotides of 3'-terminus are constructed with
      DNA, and the rest are RNA."

<400> 83
cguaccccgu ucucccagct t                                                21

<210> 84
<211> 21
<212> DNA
<213> Artificial sequence

<220>
<221> source
<223> /note= "Description of artificial sequence: synthesized DNA/RNA; antisense
      strand of B028, wherein 2 nucleotides of 3'-terminus are constructed with
      DNA, and the rest are RNA."

<400> 84
ucacuaucuc ccgguuauct t                                                21

<210> 85
<211> 21
<212> DNA
<213> Artificial sequence

<220>
<221> source
<223> /note= "Description of artificial sequence: synthesized DNA/RNA; antisense
      strand of B031, wherein 2 nucleotides of 3'-terminus are constructed with
      DNA, and the rest are RNA."

<400> 85
ucaucacuau cucccgguut t                                                21

<210> 86
<211> 21
<212> DNA
<213> Artificial sequence

<220>
<221> source
<223> /note= "Description of artificial sequence: synthesized DNA/RNA; antisense
      strand of B034, wherein 2 nucleotides of 3'-terminus are constructed with
      DNA, and the rest are RNA."

<400> 86
acuucaucac uaucucccgt t                                                21

<210> 87
<211> 21
<212> DNA
<213> Artificial sequence

<220>
<221> source
<223> /note= "Description of artificial sequence: synthesized DNA/RNA; antisense
      strand of B037, wherein 2 nucleotides of 3'-terminus are constructed with
      DNA, and the rest are RNA."

<400> 87
uguacuucau cacuaucuct t                                                21

<210> 88
<211> 21
<212> DNA
<213> Artificial sequence

<220>
<221> source
<223> /note= "Description of artificial sequence: synthesized DNA/RNA; antisense
      strand of B039, wherein 2 nucleotides of 3'-terminus are constructed with
      DNA, and the rest are RNA."

<400> 88
gauguacuuc aucacuauct t                                                21

<210> 89
<211> 21
<212> DNA
<213> Artificial sequence

<220>
<221> source
<223> /note= "Description of artificial sequence: synthesized DNA/RNA; antisense
      strand of B040, wherein 2 nucleotides of 3'-terminus are constructed with
      DNA, and the rest are RNA."

<400> 89
ggauguacuu caucacuaut t                                                21

<210> 90
<211> 21
<212> DNA
<213> Artificial sequence

<220>
<221> source
<223> /note= "Description of artificial sequence: synthesized DNA/RNA; antisense
      strand of B041, wherein 2 nucleotides of 3'-terminus are constructed with
      DNA, and the rest are RNA."

<400> 90
uggauguacu ucaucacuat t                                                21

<210> 91
<211> 21
<212> DNA
<213> Artificial sequence

<220>
<221> source
<223> /note= "Description of artificial sequence: synthesized DNA/RNA; antisense
      strand of B042, wherein 2 nucleotides of 3'-terminus are constructed with
      DNA, and the rest are RNA."

<400> 91
auggauguac uucaucacut t                                                21

<210> 92
<211> 21
<212> DNA
<213> Artificial sequence

<220>
<221> source
<223> /note= "Description of artificial sequence: synthesized DNA/RNA; antisense
      strand of B043, wherein 2 nuclectides of 3'-terminus are constructed with
      DNA, and the rest are RNA."

<400> 92
aauggaugua cuucaucact t                                                21

<210> 93
<211> 21
<212> DNA
<213> Artificial sequence

<220>
<221> source
<223> /note= "Description of artificial sequence: synthesized DNA/RNA; antisense
      strand of B044, wherein 2 nucleotides of 3'-terminus are constructed with
      DNA, and the rest are RNA."

<400> 93
uaauggaugu acuucaucat t                                                21

<210> 94
<211> 21
<212> DNA
<213> Artificial sequence

<220>
<221> source
<223> /note= "Description of artificial sequence: synthesized DNA/RNA; antisense
      strand of B045, wherein 2 nucleotides of 3'-terminus are constructed with
      DNA, and the rest are RNA."

<400> 94
auaauggaug uacuucauct t                                                21

<210> 95
<211> 21
<212> DNA
<213> Artificial sequence

<220>
<221> source
<223> /note= "Description of artificial sequence: synthesized DNA/RNA; antisense
      strand of B046, wherein 2 nucleotides of 3'-terminus are constructed with
      DNA, and the rest are RNA."

<400> 95
uauaauggau guacuucaut t                                                21

<210> 96
<211> 21

<212> DNA
<213> Artificial sequence

<220>
<221> source
<223> /note= "Description of artificial sequence: synthesized DNA/RNA; antisense
      strand of BC55, wherein 2 nucleotides of 3'-terminus are constructed with
      DNA, and the rest are RNA."

<400> 96
gcgacagcuu auaauggaut t                                                21

<210> 97
<211> 21
<212> DNA
<213> Artificial sequence

<220>
<221> source
<223> /note= "Description of artificial sequence: synthesized DNA/RNA; antisense
      strand of BC65, wherein 2 nucleotides of 3'-terminus are constructed with
      DNA, and the rest are RNA."

<400> 97
uagccccucu gcgacagcut t                                                21

<210> 98
<211> 21
<212> DNA
<213> Artificial sequence

<220>
<221> source
<223> /note= "Description of artificial sequence: synthesized DNA/RNA; antisense
      strand of B073, wherein 2 nucleotides of 3'-terminus are constructed with
      DNA, and the rest are RNA."

<400> 98
cccacucgua gccccucugt t                                                21

<210> 99
<211> 21
<212> DNA
<213> Artificial sequence

<220>
<221> source
<223> /note= "Description of artificial sequence: synthesized DNA/RNA; antisense
      strand of B084, wherein 2 nucleotides of 3'-terminus are constructed with
      DNA, and the rest are RNA."

<400> 99
aucucccgca ucccacucgt t                                                21

<210> 100
<211> 21
<212> DNA
<213> Artificial sequence

<220>
<221> source
<223> /note= "Description of artificial sequence: synthesized DNA/RNA; antisense
      strand of B136, wherein 2 nucleotides of 3'-terminus are constructed with
      DNA, and the rest are RNA."

<400> 100
gggaggagaa gaugcccggt t                                                21

<210> 101
<211> 21
<212> DNA
<213> Artificial sequence

<220>
<221> source
<223> /note= "Description of artificial sequence: synthesized DNA/RNA; antisense
      strand of B172, wherein 2 nucleotides of 3'-terminus are constructed with
      DNA, and the rest are RNA."

<400> 101
cccgggaugc ggcuggaugt t                                                21

<210> 102
<211> 21
<212> DNA
<213> Artificial sequence

<220>
<221> source
<223> /note= "Description of artificial sequence: synthesized DNA/RNA; antisense
      strand of B199, wherein 2 nucleotides of 3'-terminus are constructed with
      DNA, and the rest are RNA."

<400> 102
gcagcggcga gguccuggct t                                                21

<210> 103
<211> 21
<212> DNA
<213> Artificial sequence

<220>
<221> source
<223> /note= "Description of artificial sequence: synthesized DNA/RNA; antisense
      strand of B207, wherein 2 nucleotides of 3'-terminus are constructed with
      DNA, and the rest are RNA."

<400> 103
cggggucugc agcggcgagt t                                                21

<210> 104
<211> 21
<212> DNA
<213> Artificial sequence

<220>
<221> source

<223> /note= "Description of artificial sequence: synthesized DNA/RNA; antisense
      strand of B253, wherein 2 nucleotides of 3'-terminus are constructed with
      DNA, and the rest are RNA."

<400> 104
guggcaccgg gcugagcgct t                                                21

<210> 105
<211> 21
<212> DNA
<213> Artificial sequence

<220>
<221> source
<223> /note= "Description of artificial sequence: synthesized DNA/RNA; antisense
      strand of B262, wherein 2 nucleotides of 3'-terminus are constructed with
      DNA, and the rest are RNA."

<400> 105
ggaccacagg uggcaccggt t                                                21

<210> 106
<211> 21
<212> DNA
<213> Artificial sequence

<220>
<221> source
<223> /note= "Description of artificial sequence: synthesized DNA/RNA; antisense
      strand of B280, wherein 2 nucleotides of 3'-terminus are constructed with
      DNA, and the rest are RNA."

<400> 106
ccuggcggag ggucaggugt t                                                21

<210> 107
<211> 21
<212> DNA
<213> Artificial sequence

<220>
<221> source
<223> /note= "Description of artificial sequence: synthesized DNA/RNA; antisense
      strand of B325, wherein 2 nucleotides of 3'-terminus are constructed with
      DNA, and the rest are RNA."

<400> 107
ucucggcgaa gucgcggcgt t                                                21

<210> 108
<211> 21
<212> DNA
<213> Artificial sequence

<220>
<221> source
<223> /note= "Description of artificial sequence: synthesized DNA/RNA; antisense
      strand of B352, wherein 2 nucleotides of 3'-terminus are constructed with
      DNA, and the rest are RNA."

<400> 108
agggcgucag gugcagcugt t                                                21

<210> 109
<211> 21
<212> DNA
<213> Artificial sequence

<220>
<221> source
<223> /note= "Description of artificial sequence: synthesized DNA/RNA; antisense
      strand of B397, wherein 2 nucleotides of 3'-terminus are constructed with
      DNA, and the rest are RNA."

<400> 109
ugaagagcuc cuccaccact t                                                21

<210> 110
<211> 21
<212> DNA
<213> Artificial sequence

<220>
<221> source
<223> /note= "Description of artificial sequence: synthesized DNA/RNA; antisense
      strand of B433, wherein 2 nucleotides of 3'-terminus are constructed with
      DNA, and the rest are RNA."

<400> 110
agaaggccac aauccuccct t                                                21

<210> 111
<211> 21
<212> DNA
<213> Artificial sequence

<220>
<221> source
<223> /note= "Description of artificial sequence: synthesized DNA/RNA; antisense
      strand of B436, wherein 2 nucleotides of 3'-terminus are constructed with
      DNA, and the rest are RNA."

<400> 111
caaagaaggc cacaauccut t                                                21

<210> 112
<211> 21
<212> DNA
<213> Artificial sequence

<220>
<221> source
<223> /note= "Description of artificial sequence: synthesized DNA/RNA; antisense
      strand of B439, wherein 2 nucleotides of 3'-terminus are constructed with
      DNA, and the rest are RNA."

<400> 112
acucaaagaa ggccacaaut t                                                21

<210> 113
<211> 21
<212> DNA
<213> Artificial sequence

<220>
<221> source
<223> /note= "Description of artificial sequence: synthesized DNA/RNA; antisense
      strand of B442, wherein 2 nucleotides of 3'-terminus are constructed with
      DNA, and the rest are RNA."

<400> 113
cgaacucaaa gaaggccact t                                                21

<210> 114
<211> 21
<212> DNA
<213> Artificial sequence

<220>
<221> source
<223> /note= "Description of artificial sequence: synthesized DNA/RNA; antisense
      strand of B451, wherein 2 nucleotides of 3'-terminus are constructed with
      DNA, and the rest are RNA."

<400> 114
ugaccccacc gaacucaaat t                                                21

<210> 115
<211> 21
<212> DNA
<213> Artificial sequence

<220>
<221> source
<223> /note= "Description of artificial sequence: synthesized DNA/RNA; antisense
      strand of B463, wherein 2 nucleotides of 3'-terminus are constructed with
      DNA, and the rest are RNA."

<400> 115
ucuccacaca caugacccct t                                                21

<210> 116
<211> 21
<212> DNA
<213> Artificial sequence

<220>
<221> source
<223> /note= "Description of artificial sequence: synthesized DNA/RNA; antisense
      strand of B466, wherein 2 nucleotides of 3'-terminus are constructed with
      DNA, and the rest are RNA."

<400> 116
cgcucuccac acacaugact t                                                21

<210> 117
<211> 21

<212> DNA
<213> Artificial sequence

<220>
<221> source
<223> /note= "Description of artificial sequence: synthesized DNA/RNA; antisense
      strand of B469, wherein 2 nucleotides of 3'-terminus are constructed with
      DNA, and the rest are RNA."

<400> 117
ugacgcucuc cacacacaut t                                                21

<210> 118
<211> 21
<212> DNA
<213> Artificial sequence

<220>
<221> source
<223> /note= "Description of artificial sequence: synthesized DNA/RNA; antisense
      strand of B472, wherein 2 nucleotides of 3'-terminus are constructed with
      DNA, and the rest are RNA."

<400> 118
gguugacgcu cuccacacat t                                                21

<210> 119
<211> 21
<212> DNA
<213> Artificial sequence

<220>
<221> source
<223> /note= "Description of artificial sequence: synthesized DNA/RNA; antisense
      strand of B475, wherein 2 nucleotides of 3'-terminus are constructed with
      DNA, and the rest are RNA."

<400> 119
cccgguugac gcucuccact t                                                21

<210> 120
<211> 21
<212> DNA
<213> Artificial sequence

<220>
<221> source
<223> /note= "Description of artificial sequence: synthesized DNA/RNA; antisense
      strand of B478, wherein 2 nucleotides of 3'-terminus are constructed with
      DNA, and the rest are RNA."

<400> 120
ucucccgguu gacgcucuct t                                                21

<210> 121
<211> 21
<212> DNA
<213> Artificial sequence

<220>
<221> source
<223> /note= "Description of artificial sequence: synthesized DNA/RNA; antisense
      strand of B516, wherein 2 nucleotides of 3'-terminus are constructed with
      DNA, and the rest are RNA."

<400> 121
agucauccac agggcgaugt t                                                21

<210> 122
<211> 21
<212> DNA
<213> Artificial sequence

<220>
<221> source
<223> /note= "Description of artificial sequence: synthesized DNA/RNA; antisense
      strand of B523, wherein 2 nucleotides of 3'-terminus are constructed with
      DNA, and the rest are RNA."

<400> 122
gguacucagu cauccacagt t                                                21

<210> 123
<211> 21
<212> DNA
<213> Artificial sequence

<220>
<221> source
<223> /note= "Description of artificial sequence: synthesized DNA/RNA; antisense
      strand of B531, wherein 2 nucleotides of 3'-terminus are constructed with
      DNA, and the rest are RNA."

<400> 123
ccgguucagg uacucaguct t                                                21

<210> 124
<211> 21
<212> DNA
<213> Artificial sequence

<220>
<221> source
<223> /note= "Description of artificial sequence: synthesized DNA/RNA; antisense
      strand of B533, wherein 2 nucleotides of 3'-terminus are constructed with
      DNA, and the rest are RNA."

<400> 124
ugccgguuca gguacucagt t                                                21

<210> 125
<211> 21
<212> DNA
<213> Artificial sequence

<220>
<221> source

<223> /note= "Description of artificial sequence: synthesized DNA/RNA; antisense
      strand of B534, wherein 2 nucleotides of 3'-terminus are constructed with
      DNA, and the rest are RNA."

<400> 125
gugccgguuc agguacucat t                                                21

<210> 126
<211> 21
<212> DNA
<213> Artificial sequence

<220>
<221> source
<223> /note= "Description of artificial sequence: synthesized DNA/RNA; antisense
      strand of B536, wherein 2 nucleotides of 3'-terminus are constructed with
      DNA, and the rest are RNA."

<400> 126
aggugccggu ucagguacut t                                                21

<210> 127
<211> 21
<212> DNA
<213> Artificial sequence

<220>
<221> source
<223> /note= "Description of artificial sequence: synthesized DNA/RNA; antisense
      strand of B539, wherein 2 nucleotides of 3'-terminus are constructed with
      DNA, and the rest are RNA."

<400> 127
ugcaggugcc gguucaggut t                                                21

<210> 128
<211> 21
<212> DNA
<213> Artificial sequence

<220>
<221> source
<223> /note= "Description of artificial sequence: synthesized DNA/RNA; antisense
      strand of B543, wherein 2 nucleotides of 3'-terminus are constructed with
      DNA, and the rest are RNA."

<400> 128
ggugugcagg ugccgguuct t                                                21

<210> 129
<211> 21
<212> DNA
<213> Artificial sequence

<220>
<221> source
<223> /note= "Description of artificial sequence: synthesized DNA/RNA; antisense
      strand of B545, wherein 2 nucleotides of 3'-terminus are constructed with
      DNA, and the rest are RNA."

<400> 129
caggugugca ggugccggut t                                                21

<210> 130
<211> 21
<212> DNA
<213> Artificial sequence

<220>
<221> source
<223> /note= "Description of artificial sequence: synthesized DNA/RNA; antisense
      strand of B558, wherein 2 nucleotides of 3'-terminus are constructed with
      DNA, and the rest are RNA."

<400> 130
guuauccugg auccaggugt t                                                21

<210> 131
<211> 21
<212> DNA
<213> Artificial sequence

<220>
<221> source
<223> /note= "Description of artificial sequence: synthesized DNA/RNA; antisense
      strand of B576, wherein 2 nucleotides of 3'-terminus are constructed with
      DNA, and the rest are RNA."

<400> 131
aaaggcaucc cagccuccgt t                                                21

<210> 132
<211> 21
<212> DNA
<213> Artificial sequence

<220>
<221> source
<223> /note= "Description of artificial sequence: synthesized DNA/RNA; antisense
      strand of B586, wherein 2 nucleotides of 3'-terminus are constructed with
      DNA, and the rest are RNA."

<400> 132
acaguuccac aaaggcauct t                                                21

<210> 133
<211> 21
<212> DNA
<213> Artificial sequence

<220>
<221> source
<223> /note= "Description of artificial sequence: synthesized DNA/RNA; antisense
      strand of B595, wherein 2 nucleotides of 3'-terminus are constructed with
      DNA, and the rest are RNA."

<400> 133
uggggccgua caguuccact t                                                21

<210> 134
<211> 21
<212> DNA
<213> Artificial sequence

<220>
<221> source
<223> /note= "Description of artificial sequence: synthesized DNA/RNA; antisense
      strand of B604, wherein 2 nucleotides of 3'-terminus are constructed with
      DNA, and the rest are RNA."

<400> 134
gccgcaugcu ggggccguat t                                                21

<210> 135
<211> 21
<212> DNA
<213> Artificial sequence

<220>
<221> source
<223> /note= "Description of artificial sequence: synthesized DNA/RNA; antisense
      strand of B613, wherein 2 nucleotides of 3'-terminus are constructed with
      DNA, and the rest are RNA."

<400> 135
caaacagagg ccgcaugcut t                                                21

<210> 136
<211> 21
<212> DNA
<213> Artificial sequence

<220>
<221> source
<223> /note= "Description of artificial sequence: synthesized DNA/RNA; antisense
      strand of B614, wherein 2 nucleotides of 3'-terminus are constructed with
      DNA, and the rest are RNA."

<400> 136
ucaaacagag gccgcaugct t                                                21

<210> 137
<211> 21
<212> DNA
<213> Artificial sequence

<220>
<221> source
<223> /note= "Description of artificial sequence: synthesized DNA/RNA; antisense
      strand of B615, wherein 2 nucleotides of 3'-terminus are constructed with
      DNA, and the rest are RNA."

<400> 137
aucaaacaga ggccgcaugt t                                                21

<210> 138
<211> 21

<212> DNA
<213> Artificial sequence

<220>
<221> source
<223> /note= "Description of artificial sequence: synthesized DNA/RNA; antisense
      strand of B616, wherein 2 nucleotides of 3'-terminus are constructed with
      DNA, and the rest are RNA."

<400> 138
aaucaaacag aggccgcaut t                                                21

<210> 139
<211> 21
<212> DNA
<213> Artificial sequence

<220>
<221> source
<223> /note= "Description of artificial sequence: synthesized DNA/RNA; antisense
      strand of B619, wherein 2 nucleotides of 3'-terminus are constructed with
      DNA, and the rest are RNA."

<400> 139
agaaaucaaa cagaggccgt t                                                21

<210> 140
<211> 21
<212> DNA
<213> Artificial sequence

<220>
<221> source
<223> /note= "Description of artificial sequence: synthesized DNA/RNA; antisense
      strand of B622, wherein 2 nucleotides of 3'-terminus are constructed with
      DNA, and the rest are RNA."

<400> 140
aggagaaauc aaacagaggt t                                                21

<210> 141
<211> 21
<212> DNA
<213> Artificial sequence

<220>
<221> source
<223> /note= "Description of artificial sequence: synthesized DNA/RNA; antisense
      strand of B625, wherein 2 nucleotides of 3'-terminus are constructed with
      DNA, and the rest are RNA."

<400> 141
gccaggagaa aucaaacagt t                                                21

<210> 142
<211> 21
<212> DNA
<213> Artificial sequence

<220>
<221> source
<223> /note= "Description of artificial sequence: synthesized DNA/RNA; antisense
      strand of B628, wherein 2 nucleotides of 3'-terminus are constructed with
      DNA, and the rest are RNA."

<400> 142
acagccagga gaaaucaaat t                                                21

<210> 143
<211> 21
<212> DNA
<213> Artificial sequence

<220>
<221> source
<223> /note= "Description of artificial sequence: synthesized DNA/RNA; antisense
      strand of B631, wherein 2 nucleotides of 3'-terminus are constructed with
      DNA, and the rest are RNA."

<400> 143
gagacagcca ggagaaauct t                                                21

<210> 144
<211> 21
<212> DNA
<213> Artificial sequence

<220>
<221> source
<223> /note= "Description of artificial sequence: synthesized DNA/RNA; antisense
      strand of B634, wherein 2 nucleotides of 3'-terminus are constructed with
      DNA, and the rest are RNA."

<400> 144
ucagagacag ccaggagaat t                                                21

<210> 145
<211> 21
<212> DNA
<213> Artificial sequence

<220>
<221> source
<223> /note= "Description of artificial sequence: synthesized DNA/RNA; antisense
      strand of B636, wherein 2 nucleotides of 3'-terminus are constructed with
      DNA, and the rest are RNA."

<400> 145
cuucagagac agccaggagt t                                                21

<210> 146
<211> 21
<212> DNA
<213> Artificial sequence

<220>
<221> source

<223> /note= "Description of artificial sequence: synthesized DNA/RNA; antisense
      strand of B642, wherein 2 nucleotides of 3'-terminus are constructed with
      DNA, and the rest are RNA."

<400> 146
cagagucuuc agagacagct t                                                21

<210> 147
<211> 21
<212> DNA
<213> Artificial sequence

<220>
<221> source
<223> /note= "Description of artificial sequence: synthesized DNA/RNA; antisense
      strand of B649, wherein 2 nucleotides of 3'-terminus are constructed with
      DNA, and the rest are RNA."

<400> 147
aacugagcag agucuucagt t                                                21

<210> 148
<211> 21
<212> DNA
<213> Artificial sequence

<220>
<221> source
<223> /note= "Description of artificial sequence: synthesized DNA/RNA; antisense
      strand of B654, wherein 2 nucleotides of 3'-terminus are constructed with
      DNA, and the rest are RNA."

<400> 148
ggccaaacug agcagaguct t                                                21

<210> 149
<211> 21
<212> DNA
<213> Artificial sequence

<220>
<221> source
<223> /note= "Description of artificial sequence: synthesized DNA/RNA; antisense
      strand of B658, wherein 2 nucleotides of 3'-terminus are constructed with
      DNA, and the rest are RNA."

<400> 149
ccagggccaa acugagcagt t                                                21

<210> 150
<211> 21
<212> DNA
<213> Artificial sequence

<220>
<221> source
<223> /note= "Description of artificial sequence: synthesized DNA/RNA; antisense
      strand of B667, wherein 2 nucleotides of 3'-terminus are constructed with
      DNA, and the rest are RNA."

<400> 150
aagcucccac cagggccaat t                                                21

<210> 151
<211> 21
<212> DNA
<213> Artificial sequence

<220>
<221> source
<223> /note= "Description of artificial sequence: synthesized DNA/RNA; antisense
      strand of B676, wherein 2 nucleotides of 3'-terminus are constructed with
      DNA, and the rest are RNA."

<400> 151
gggugaugca agcucccact t                                                21

<210> 152
<211> 21
<212> DNA
<213> Artificial sequence

<220>
<221> source
<223> /note= "Description of artificial sequence: synthesized DNA/RNA; antisense
      strand of B697, wherein 2 nucleotides of 3'-terminus are constructed with
      DNA, and the rest are RNA."

<400> 152
uguggcccag auaggcacct t                                                21

<210> 153
<211> 21
<212> DNA
<213> Artificial sequence

<220>
<221> source
<223> /note= "Description of artificial sequence: synthesized DNA/RNA; antisense
      strand of B700, wherein 2 nucleotides of 3'-terminus are constructed with
      DNA, and the rest are RNA."

<400> 153
acuuguggcc cagauaggct t                                                21

<210> 154
<211> 21
<212> DNA
<213> Artificial sequence

<220>
<221> source
<223> /note= "Description of artificial sequence: synthesized DNA/RNA; antisense
      strand of B703, wherein 2 nucleotides of 3'-terminus are constructed with
      DNA, and the rest are RNA."

<400> 154
uucacuugug gcccagauat t                                                21

<210> 155
<211> 21
<212> DNA
<213> Artificial sequence

<220>
<221> source
<223> /note= "Description of artificial sequence: synthesized DNA/RNA; antisense
      strand of B706, wherein 2 nucleotides of 3'-terminus are constructed with
      DNA, and the rest are RNA."

<400> 155
gacuucacuu guggcccagt t                                                21

<210> 156
<211> 21
<212> DNA
<213> Artificial sequence

<220>
<221> source
<223> /note= "Description of artificial sequence: synthesized DNA/RNA; antisense
      strand of B709, wherein 2 nucleotides of 3'-terminus are constructed with
      DNA, and the rest are RNA."

<400> 156
guugacuuca cuuguggcct t                                                21

<210> 157
<211> 21
<212> DNA
<213> Artificial sequence

<220>
<221> source
<223> /note= "Description of artificial sequence: synthesized DNA/RNA; antisense
      strand of B712, wherein 2 nucleotides of 3'-terminus are constructed with
      DNA, and the rest are RNA."

<400> 157
cauguugacu ucacuugugt t                                                21

<210> 158
<211> 21
<212> DNA
<213> Artificial sequence

<220>
<221> source
<223> /note= "Description of artificial sequence: synthesized DNA/RNA; antisense
      strand of B717, wherein 2 nucleotides of 3'-terminus are constructed with
      DNA, and the rest are RNA."

<400> 158
gcaggcaugu ugacuucact t                                                21

<210> 159
<211> 21

<212> DNA
<213> Artificial sequence

<220>
<221> source
<223> /note= "Description of artificial sequence: synthesized DNA/RNA; antisense
      strand of B719, wherein 2 nucleotides of 3'-terminus are constructed with
      DNA, and the rest are RNA."

<400> 159
gggcaggcau guugacuuct t                                                21

<210> 160
<211> 21
<212> DNA
<213> Artificial sequence

<220>
<221> source
<223> /note= "Description of artificial sequence: synthesized DNA/RNA; antisense
      strand of B721, wherein 2 nucleotides of 3'-terminus are constructed with
      DNA, and the rest are RNA."

<400> 160
uggggcaggc auguugacut t                                                21

<210> 161
<211> 21
<212> DNA
<213> Artificial sequence

<220>
<221> source
<223> /note= "Description of artificial sequence: synthesized DNA/RNA; antisense
      strand of B724, wherein 2 nucleotides of 3'-terminus are constructed with
      DNA, and the rest are RNA."

<400> 161
guuggggca ggcauguugt t                                                 21

<210> 162
<211> 21
<212> DNA
<213> Artificial sequence

<220>
<221> source
<223> /note= "Description of artificial sequence: synthesized DNA/RNA; antisense
      strand of B727, wherein 2 nucleotides of 3'-terminus are constructed with
      DNA, and the rest are RNA."

<400> 162
uuuguuuggg gcaggcaugt t                                                21

<210> 163
<211> 21
<212> DNA
<213> Artificial sequence

<220>
<221> source
<223> /note= "Description of artificial sequence: synthesized DNA/RNA; sense
      strand of B043-15A, wherein 2 nucleotides of 3'-terminus are constructed
      with DNA, and the rest are RNA."

<400> 163
gugaugaagu acauacauut t                                                21

<210> 164
<211> 21
<212> DNA
<213> Artificial sequence

<220>
<221> source
<223> /note= "Description of artificial sequence: synthesized DNA/RNA; antisense
      strand of B043-15A, wherein 2 nucleotides of 3'-terminus are constructed
      with DNA, and the rest are RNA."

<400> 164
aauguaugua cuucaucact t                                                21

<210> 165
<211> 21
<212> DNA
<213> Artificial sequence

<220>
<221> source
<223> /note= "Description of artificial sequence: synthesized DNA/RNA; sense
      strand of B533-18U, wherein 2 nucleotides of 3'-terminus are constructed
      with DNA, and the rest are RNA."

<400> 165
cugaguaccu gaaccgguat t                                                21

<210> 166
<211> 21
<212> DNA
<213> Artificial sequence

<220>
<221> source
<223> /note= "Description of artificial sequence: synthesized DNA/RNA; antisense
      strand of B533-18U, wherein 2 nucleotides of 3'-terminus are constructed
      with DNA, and the rest are RNA."

<400> 166
uaccgguuca gguacucagt t                                                21

<210> 167
<211> 21
<212> DNA
<213> Artificial sequence

<220>
<221> source

<223> /note= "Description of artificial sequence: synthesized DNA/RNA; sense
      strand of B717-10U, wherein 2 nucleotides of 3'-terminus are constructed
      with DNA, and the rest are RNA."

<400> 167
gugaagucau caugccugct t                                                 21

<210> 168
<211> 21
<212> DNA
<213> Artificial sequence

<220>
<221> source
<223> /note= "Description of artificial sequence: synthesized DNA/RNA; antisense
      strand of B717-10U, wherein 2 nucleotides of 3'-terminus are constructed
      with DNA, and the rest are RNA."

<400> 168
gcaggcauga ugacuucact t                                                 21

<210> 169
<211> 21
<212> DNA
<213> Artificial sequence

<220>
<221> source
<223> /note= "Description of artificial sequence: synthesized DNA/RNA; sense
      strand of B533-2A, wherein 2 nucleotides of 3'-terminus are constructed
      with DNA, and the rest are RNA."

<400> 169
cagaguaccu gaaccggcat t                                                 21

<210> 170
<211> 21
<212> DNA
<213> Artificial sequence

<220>
<221> source
<223> /note= "Description of artificial sequence: synthesized DNA/RNA; antisense
      strand of B533-2A, wherein 2 nucleotides of 3'-terminus are constructed
      with DNA, and the rest are RNA."

<400> 170
ugccgguuca gguacucugt t                                                 21

<210> 171
<211> 21
<212> DNA
<213> Artificial sequence

<220>
<221> source
<223> /note= "Description of artificial sequence: synthesized DNA/RNA; sense
      strand of B533-5U, wherein 2 nucleotides of 3'-terminus are constructed
      with DNA, and the rest are RNA."

<400> 171
cugauuaccu gaaccggcat t                                                21

<210> 172
<211> 21
<212> DNA
<213> Artificial sequence

<220>
<221> source
<223> /note= "Description of artificial sequence: synthesized DNA/RNA; antisense
      strand of B533-5U, wherein 2 nucleotides of 3'-terminus are constructed
      with DNA, and the rest are RNA."

<400> 172
ugccgguuca gguaaucagt t                                                21

<210> 173
<211> 21
<212> DNA
<213> Artificial sequence

<220>
<221> source
<223> /note= "Description of artificial sequence: synthesized DNA/RNA; sense
      strand of B533-8U, wherein 2 nucleotides of 3'-terminus are constructed
      with DNA, and the rest are RNA."

<400> 173
cugaguaucu gaaccggcat t                                                21

<210> 174
<211> 21
<212> DNA
<213> Artificial sequence

<220>
<221> source
<223> /note= "Description of artificial sequence: synthesized DNA/RNA; antisense
      strand of B533-8U, wherein 2 nucleotides of 3'-terminus are constructed
      with DNA, and the rest are RNA."

<400> 174
ugccgguuca gauacucagt t                                                21

<210> 175
<211> 21
<212> DNA
<213> Artificial sequence

<220>
<221> source
<223> /note= "Description of artificial sequence: synthesized DNA/RNA; sense
      strand of B533-10A, wherein 2 nucleotides of 3'-terminus are constructed
      with DNA, and the rest are RNA."

<400> 175
cugaguacca gaaccggcat t                                                21

<210> 176
<211> 21
<212> DNA
<213> Artificial sequence

<220>
<221> source
<223> /note= "Description of artificial sequence: synthesized DNA/RNA; antisense
      strand of B533-10A, wherein 2 nuclectides of 3'-terminus are constructed
      with DNA, and the rest are RNA."

<400> 176
ugccgguucu gguacucagt t                                                21

<210> 177
<211> 21
<212> DNA
<213> Artificial sequence

<220>
<221> source
<223> /note= "Description of artificial sequence: synthesized DNA/RNA; sense
      strand of B533-12U, wherein 2 nucleotides of 3'-terminus are constructed
      with DNA, and the rest are RNA."

<400> 177
cugaguaccu guaccggcat t                                                21

<210> 178
<211> 21
<212> DNA
<213> Artificial sequence

<220>
<221> source
<223> /note= "Description of artificial sequence: synthesized DNA/RNA; antisense
      strand of B533-12U, wherein 2 nucleotides of 3'-terminus are constructed
      with DNA, and the rest are RNA."

<400> 178
ugccgguaca gguacucagt t                                                21

<210> 179
<211> 21
<212> DNA
<213> Artificial sequence

<220>
<221> source
<223> /note= "Description of artificial sequence: synthesized DNA/RNA; sense
      strand of B533-15U, wherein 2 nucleotides of 3'-terminus are constructed
      with DNA, and the rest are RNA."

<400> 179
cugaguaccu gaacuggcat t                                                21

<210> 180
<211> 21

<212> DNA
<213> Artificial sequence

<220>
<221> source
<223> /note= "Description of artificial sequence: synthesized DNA/RNA; antisense
      strand of B533-15U, wherein 2 nucleotides of 3'-terminus are constructed
      with DNA, and the rest are RNA."

<400> 180
ugccaguuca gguacucagt t                                                21

<210> 181
<211> 20
<212> DNA
<213> Artificial sequence

<220>
<221> source
<223> /note= "Description of artificial sequence: synthesized DNA/RNA; sense
      strand of B533-20b, wherein 2 nucleotides of 3'-terminus are constructed
      with DNA, and the rest are RNA."

<400> 181
cugaguaccu gaaccggcac                                                  20

<210> 182
<211> 20
<212> DNA
<213> Artificial sequence

<220>
<221> source
<223> /note= "Description of artificial sequence: synthesized DNA/RNA; sense
      strand of B534-20b, wherein 2 nucleotides of 3'-terminus are constructed
      with DNA, and the rest are RNA."

<400> 182
ugaguaccug aaccggcacc                                                  20

<210> 183
<211> 20
<212> DNA
<213> Artificial sequence

<220>
<221> source
<223> /note= "Description of artificial sequence: synthesized DNA/RNA; antisense
      strand of B533-20b, wherein 2 nucleotides of 3'-terminus are constructed
      with DNA, and the rest are RNA."

<400> 183
gccgguucag guacucagtc                                                  20

<210> 184
<211> 20
<212> DNA
<213> Artificial sequence

<220>
<221> source
<223> /note= "Description of artificial sequence: synthesized DNA/RNA; antisense
      strand of B534-20b, wherein 2 nucleotides of 3'-terminus are constructed
      with DNA, and the rest are RNA."

<400> 184
ugccgguuca gguacucagt                                                    20

<210> 185
<211> 21
<212> DNA
<213> Artificial sequence

<220>
<221> source
<223> /note= "Description of artificial sequence: synthesized DNA/RNA; sense
      strand of B043-4nt-D, wherein 4 nucleotides of 3'-terminus are constructed
      with DNA, and the rest are RNA."

<400> 185
gugaugaagu acauccattt t                                                  21

<210> 186
<211> 21
<212> DNA
<213> Artificial sequence

<220>
<221> source
<223> /note= "Description of artificial sequence: synthesized DNA/RNA; DNA/RNA
      of B043-6nt-D sense strand, wherein 6 nucleotides of 3'-terminus are
      constructed with DNA, and the rest are RNA."

<400> 186
gugaugaagu acauccattt t                                                  21

<210> 187
<211> 21
<212> DNA
<213> Artificial sequence

<220>
<221> source
<223> /note= "Description of artificial sequence: synthesized DNA/RNA; DNA/RNA
      of B043-G-dG sense strand, wherein 2 nucleotides of 3'-terminus and G
      nucleotides are constructed with DNA, and the rest are RNA."

<400> 187
gugaugaagu acauccauut t                                                  21

<210> 188
<211> 21
<212> DNA
<213> Artificial sequence

<220>
<221> source

<223> /note= "Description of artificial sequence: synthesized DNA/RNA; DNA/RNA
      of B043-U-dJ sense strand, wherein T nucleotides are constructed with DNA,
      and the rest are RNA."

<400> 188
gtgatgaagt acatccattt t                                                21

<210> 189
<211> 21
<212> DNA
<213> Artificial sequence

<220>
<221> source
<223> /note= "Description of artificial sequence: synthesized DNA/RNA; sense
      strand of B533-4nt-D, wherein 4 nucleotides of 3'-terminus are constructed
      with DNA, and the rest are RNA."

<400> 189
cugaguaccu gaaccggcat t                                                21

<210> 190
<211> 21
<212> DNA
<213> Artificial sequence

<220>
<221> source
<223> /note= "Description of artificial sequence: synthesized DNA/RNA; DNA/RNA
      of B533-6nt-D sense strand, wherein 6 nuclectides of 3'-terminus are
      constructed with DNA, and the rest are RNA."

<400> 190
cugaguaccu gaaccggcat t                                                21

<210> 191
<211> 21
<212> DNA
<213> Artificial sequence

<220>
<221> source
<223> /note= "Description of artificial sequence: synthesized DNA/RNA; DNA/RNA
      of B533-G-dG sense strand, wherein 2 nucleotides of 3'-terminus and G
      nuclectides are constructed with DNA, and the rest are RNA."

<400> 191
cugaguaccu gaaccggcat t                                                21

<210> 192
<211> 21
<212> DNA
<213> Artificial sequence

<220>
<221> source
<223> /note= "Description of artificial sequence: synthesized DNA/RNA; DNA/RNA
      of B533-U-dT sense strand, wherein T nucleotides are constructed with DNA,
      and the rest are RNA."

<400> 192
ctgagtacct gaaccggcat t                                                21

<210> 193
<211> 21
<212> DNA
<213> Artificial sequence

<220>
<221> source
<223> /note= "Description of artificial sequence: synthesized DNA/RNA; antisense
      strand of B043-4nt-D, wherein 4 nucleotides of 3'-terminus are constructed
      with DNA, and the rest are RNA."

<400> 193
aauggaugua cuucaucact t                                                21

<210> 194
<211> 21
<212> DNA
<213> Artificial sequence

<220>
<221> source
<223> /note= "Description of artificial sequence: synthesized DNA/RNA; DNA/RNA
      of B043-2nt2nt-D antisense strand, wherein 2 nucleotides of 3'-terminus
      and 2 nucleotides of 5'-terminus are constructed with DNA, and the rest
      are RNA."

<400> 194
aauggaugua cuucaucact t                                                21

<210> 195
<211> 21
<212> DNA
<213> Artificial sequence

<220>
<221> source
<223> /note= "Description of artificial sequence: synthesized DNA/RNA; antisense
      strand of B533-4nt-D, wherein 4 nucleotides of 3'-terminus are constructed
      with DNA, and the rest are RNA."

<400> 195
ugccgguuca gguacucagt t                                                21

<210> 196
<211> 21
<212> DNA
<213> Artificial sequence

<220>
<221> source
<223> /note= "Description of artificial sequence: synthesized DNA/RNA; DNA/RNA
      of B533-4nt-D antisense strand, wherein 4 nucleotides of 3'-terminus and 2
      nucleotides of 5'-terminus are constructed with DNA, and the rest are
      RNA."

<400> 196
tgccgguuca gguacucagt t                                                21

<210> 197
<211> 2704
<212> DNA
<213> Homo sapiens

<220>
<221> source
<222> 1..2704
<223> /organism="Homo sapiens"
      /db_xref="LocusID:596"
      /db_xref="taxon:9606"
      /clone="MGC:21366 IMAGE:4511027"
      /tissue_type="Testis, embryonal carcinoma"
      /clone_lib="NIH_MGC_92"
      /lab_host="DH10B"
      /note="Vector: pCMV-SPORT6"

<220>
<221> CDS
<222> 390..1109
<223> /product="B-cell CLL/lymphoma 2"
       /protein_id="AAH27258.1"
       /db_xref="GI:20072668"
       /translation="MAHAGRTGYDNREIVMKYIHYKLSQRGYEWDAGDVGAAPPGAAP
       APGIFSSQPGHTPHPAASRDPVARTSPLQTPAAPGAAAGPALSPVPPVVHLTLRQAGD
       DFSRRYRRDFAEMSSQLHLTPFTARGRFATVVEELFRDGVNWGRIVAFFEFGGVMCVE
       SVNREMSPLVDNIALWMTEYLNRHLHTWIQDNGGWDAFVELYGPSMRPLFDFSWLSLK
       TLLSLALVGACITLGAYLGHK"

<400> 197
   ctggagagtg ctgaagattg atgggatcgt tgccttatgc atttgttttg gttttacaaa      60
   aaggaaactt gacagaggat catgctgtac ttaaaaaata caacatcaca gaggaagtag     120
   actgatatta acaatactta ctaataataa cgtgcctcat gaaataaaga tccgaaagga     180
   attggaataa aaatttcctg catctcatgc caagggggaa acaccagaat caagtgttcc     240
   gcgtgattga agacaccccc tcgtccaaga atgcaaagca catccaataa aatagctgga     300
   ttataactcc tcttctttct ctgggggccg tggggtggga gctggggcga gaggtgccgt     360
   tggcccccgt tgcttttcct ctgggaagga tggcgcacgc tgggagaacg gggtacgata     420
   accgggagat agtgatgaag tacatccatt ataagctgtc gcagaggggc tacgagtggg     480
   atgcgggaga tgtgggcgcc gcgcccccgg gggccgcccc cgcaccgggc atcttctcct     540
   cccagcccgg gcacacgccc catccagccg catcccggga cccggtcgcc aggacctcgc     600
   cgctgcagac cccggctgcc cccggcgccg ccgcggggcc tgcgctcagc ccggtgccac     660
   ctgtggtcca cctgaccctc cgccaggccg gcgacgactt ctcccgccgc taccgccgcg     720
   acttcgccga gatgtccagc cagctgcacc tgacgccctt caccgcgcgg ggacgctttg     780
   ccacggtggt ggaggagctc ttcagggacg gggtgaactg ggggaggatt gtggccttct     840
   ttgagttcgg tggggtcatg tgtgtggaga gcgtcaaccg ggagatgtcg cccctggtgg     900
   acaacatcgc cctgtggatg actgagtacc tgaaccggca cctgcacacc tggatccagg     960
   ataacggagg ctgggatgcc tttgtggaac tgtacggccc cagcatgcgg cctctgtttg    1020
   atttctcctg gctgtctctg aagactctgc tcagtttggc cctggtggga gcttgcatca    1080
   ccctgggtgc ctatctgggc cacaagtgaa gtcaacatgc ctgccccaaa caaatatgca    1140
   aaaggttcac taaagcagta gaaataatat gcattgtcag tgatgtacca tgaaacaaag    1200
   ctgcaggctg tttaagaaaa aataacacac atataaacat cacacacaca gacagacaca    1260
   cacacacaca acaattaaca gtcttcaggc aaaacgtcga atcagctatt tactgccaaa    1320
   gggaaatatc atttattttt tacattatta agaaaaaaag atttatttat ttaagacagt    1380
   cccatcaaaa ctcctgtctt tggaaatccg accactaatt gccaagcacc gcttcgtgtg    1440
   gctccacctg gatgttctgt gcctgtaaac atagattcgc tttccatgtt gttggccgga    1500
   tcaccatctg aagagcagac ggatggaaaa aggacctgat cattggggaa gctggctttc    1560
   tggctgctgg aggctgggga gaaggtgttc attcacttgc atttctttgc cctgggggct 1620
   gtgatattaa cagagggagg gttcctgtgg ggggaagtcc atgcctccct ggcctgaaga 1680
   agagactctt tgcatatgac tcacatgatg catacctggt gggaggaaaa gagttgggaa 1740
   cttcagatgg acctagtacc cactgagatt tccacgccga aggacagcga tgggaaaaat 1800
   gcccttaaat catacgaaag tattttttta agctaccaat tgtgccgaga aaagcatttt 1860
   agcaatttat acaatatcat ccagtacctt aagccctgat tgtgtatatt catatatttt 1920
   ggatacgcac ccccccactc ccaatactgg ctctgtctga gtaagaaaca gaatcctctg 1980
   gaacttgagg aagtgaacat ttcggtgact tccgcatcag gaaggctaga gttacccaga 2040
   gcatcaggcc gccacaagtg cctgctttta ggagaccgaa gtccgcagaa cctgcctgtg 2100
   tcccagcttg gaggcctggt cctggaactg agccgggccc ctcactggcc tcctccaggg 2160
   atgatcaaca gggcagtgtg gtctccgaat gtctggaagc tgatggagct cagaattcca 2220
   ctgtcaagaa agagcagtag aggggtgtgg ctgggcctgt caccctgggg ccctccaggt 2280
   aggcccgttt tcacgtggag catgggagcc acgacccttc ttaagacatg tatcactgta 2340
   gagggaagga acagaggccc tgggcccttc ctatcagaag gacatggtga aggctgggaa 2400
   cgtgaggaga ggcaatggcc acggcccatt ttggctgtag cacatggcac gttggctgtg 2460
   tggccttggc ccacctgtga gtttaaagca aggctttaaa tgactttgga gagggtcaca 2520
   aatcctaaaa gaagcattga agtgaggtgt catggattaa ttgacccctg tctatggaat 2580
   tacatgtaaa acattatctt gtcactgtag tttggtttta tttgaaaacc tgacaaaaaa 2640
   aaagttccag gtgtggaata tgggggttat ctgtacatcc tggggcatta aaaaaaaaaa 2700
   aaaa 2704

<210> 198
<211> 19
<212> RNA
<213> Artificial sequence

<220>
<221> source
<223> /note= "Description of artificial sequence: synthesized RNA; sense strand
      of B535-19b."

<400> 198
gaguaccuga accggcacc 19

<210> 199
<211> 19
<212> RNA
<213> Artificial sequence

<220>
<221> source
<223> /note= "Description of artificial sequence: synthesized RNA; antisense
      strand of B535-19b."

<400> 199
ugccgguuca gguacucag 19

<210> 200
<211> 18
<212> RNA
<213> Artificial sequence

<220>
<221> source
<223> /note= "Description of artificial sequence: synthesized RNA; sense strand
      of B536-18b."

<400> 200
aguaccugaa ccggcacc 18

<210> 201
<211> 18
<212> RNA
<213> Artificial sequence

<220>
<221> source
<223> /note= "Description of artificial sequence: synthesized RNA; antisense
      strand of B536-18b."

<400> 201
ugccgguuca gguacuca                                                18

<210> 202
<211> 17
<212> RNA
<213> Artificial sequence

<220>
<221> source
<223> /note= "Description of artificial sequence: synthesized RNA; sense strand
      of B537-17b."

<400> 202
guaccugaac cggcacc                                                 17

<210> 203
<211> 17
<212> RNA
<213> Artificial sequence

<220>
>221> source
<223> /note= "Description of artificial sequence: synthesized RNA; antisense
      strand of B537-17b."

<400> 203
ugccgguuca gguacuc                                                 17

<210> 204
<211> 17
<212> RNA
<213> Artificial sequence

<220>
<221> source
<223> /note= "Description of artificial sequence: synthesized RNA; sense strand
      of B535bl-17b."

<400> 204
gaguaccuga accggca                                                 17

<210> 205
<211> 20
<212> RNA
<213> Artificial sequence

<220>

<221> source
<223> /note= "Description of artificial sequence: synthesized RNA; sense strand
      of B531-20b."

<400> 205
gacugaguac cugaaccggc                                                    20

<210> 206
<211> 21
<212> RNA
<213> Artificial sequence

<220>
<221> source
<223> /note= "Description of artificial sequence: synthesized RNA; sense strand
      of B533-OH-2."

<400> 206
augacugagu accugaaccg g                                                  21

<210> 207
<211> 21
<212> DNA
<213> Artificial sequence

<220>
<221> source
<223> /note= "Description of artificial sequence: synthesized RNA; antisense
      strand of B533-OH-2, wherein 2 nucleotides of 3'-terminus are constructed
      with DNA, and the rest are RNA."

<400> 207
ugccgguuca gguacucagt c                                                  21

<210> 208
<211> 19
<212> RNA
<213> Artificial sequence

<220>
<221> source
<223> /note= "Description of artificial sequence: synthesized RNA; sense strand
      of B043bl-19b."

<400> 208
gugaugaagu acauccauu                                                     19

<210> 209
<211> 19
<212> RNA
<213> Artificial sequence

<220>
<221> source
<223> /note= "Description of artificial sequence: synthesized RNA; sense strand
      of B436bl-19b."

<400> 209
aggauugugg ccuucuuug                                                     19

<210> 210
<211> 19
<212> RNA
<213> Artificial sequence

<220>
<221> source
<223> /note= "Description of artificial sequence: synthesized RNA; sense strand
      of B439bl-19b."

<400> 210
auuguggccu ucuuugagu                                                    19

<210> 211
<211> 19
<212> RNA
<213> Artificial sequence

<220>
<221> source
<223> /note= "Description of artificial sequence: synthesized RNA; sense strand
      of B469bl-19b."

<400> 211
augugugugg agagcguca                                                    19

<210> 212
<211> 19
<212> RNA
<213> Artificial sequence

<220>
<221> source
<223> /note= "Description of artificial sequence: synthesized RNA; sense strand
      of B533bl-19b."

<400> 212
cugaguaccu gaaccggca                                                    19

<210> 213
<211> 19
<212> RNA
<213> Artificial sequence

<220>
<221> source
<223> /note= "Description of artificial sequence: synthesized RNA; sense strand
      of B614bl-19b."

<400> 213
gcaugcggcc ucuguuuga                                                    19

<210> 214
<211> 19
<212> RNA
<213> Artificial sequence

<220>

<221> source
<223> /note= "Description of artificial sequence: synthesized RNA; sense strand
      of B625bl-19b."

<400> 214
cuguuugauu ucuccuggc                                                    19

<210> 215
<211> 19
<212> RNA
<213> Artificial sequence

<220>
<221> source
<223> /note= "Description of artificial sequence: synthesized RNA; sense strand
      of B631bl-19b."

<400> 215
gauuucuccu ggcugucuc                                                    19

<210> 216
<211> 19
<212> RNA
<213> Artificial sequence

<220>
<221> source
<223> /note= "Description of artificial sequence: synthesized RNA; sense strand
      of B634bl-19b."

<400> 216
uucuccuggc ugucucuga                                                    19

<210> 217
<211> 19
<212> RNA
<213> Artificial sequence

<220>
<221> source
<223> /note= "Description of artificial sequence: synthesized RNA; sense strand
      of B717bl-19b."

<400> 217
gugaagucaa caugccugc                                                    19

<210> 218
<211> 18
<212> RNA
<213> Artificial sequence

<220>
<221> source
<223> /note= "Description of artificial sequence: synthesized RNA; sense strand
      of B043bl-18b."

<400> 218
gugaugaagu acauccau                                                     18

<210> 219
<211> 18
<212> RNA
<213> Artificial sequence

<220>
<221> source
<223> /note= "Description of artificial sequence: synthesized RNA; sense strand
      of B044bl-18b."

<400> 219
ugaucaagua cauccauu                                                    18

<210> 220
<211> 18
<212> RNA
<213> Artificial sequence

<220>
<221> source
<223> /note= "Description of artificial sequence: synthesized RNA; sense strand
      of B469bl-18b."

<400> 220
augugugugg agagcguc                                                    18

<210> 221
<211> 18
<212> RNA
<213> Artificial sequence

<220>
<221> source
<223> /note= "Description of artificial sequence: synthesized RNA; sense strand
      of B470bl-18b."

<400> 221
ugugugugga gagcguca                                                    18

<210> 222
<211> 18
<212> RNA
<213> Artificial sequence

<220>
<221> source
<223> /note= "Description of artificial sequence: synthesized RNA; sense strand
      of B534bl-18b."

<400> 222
ugaguaccug aaccggca                                                    18

<210> 223
<211> 18
<212> RNA
<213> Artificial sequence

<220>
<221> source

<223> /note= "Description of artificial sequence: synthesized RNA; sense strand
      of B718bl-18b."

<400> 223
ugaagucaac augccugc                                                18

<210> 224
<211> 19
<212> RNA
<213> Artificial sequence

<220>
<221> source
<223> /note= "Description of artificial sequence: synthesized RNA; antisense
      strand of B043bl-19b."

<400> 224
aauggaugua cuucaucac                                               19

<210> 225
<211> 19
<212> RNA
<213> Artificial sequence

<220>
<221> source
<223> /note= "Description of artificial sequence: synthesized RNA; antisense
      strand of B436bl-19b."

<400> 225
caaagaaggc cacaauccu                                               19

<210> 226
<211> 19
<212> RNA
<213> Artificial sequence

<220>
<221> source
<223> /note= "Description of artificial sequence: synthesized RNA; antisense
      strand of B439bl-19b."

<400> 226
acucaaagaa ggccacaau                                               19

<210> 227
<211> 19
<212> RNA
<213> Artificial sequence

<220>
<221> source
<223> /note= "Description of artificial sequence: synthesized RNA; antisense
      strand of B469bl-19b."

<400> 227
ugacgcucuc cacacacau                                               19

<210> 228

<211> 19
<212> RNA
<213> Artificial sequence

<220>
<221> source
<223> /note= "Description of artificial sequence: synthesized RNA; antisense
      strand of B533bl-19b."

<400> 228
ugccgguuca gguacucag                                                    19

<210> 229
<211> 19
<212> RNA
<213> Artificial sequence

<220>
<221> source
<223> /note= "Description of artificial sequence: synthesized RNA; antisense
      strand of B614bl-19b."

<400> 229
ucaaacagag gccgcaugc                                                    19

<210> 230
<211> 19
<212> RNA
<213> Artificial sequence

<220>
<221> source
<223> /note= "Description of artificial sequence: synthesized RNA; antisense
      strand of B625bl-19b."

<400> 230
gccaggagaa aucaaacag                                                    19

<210> 231
<211> 19
<212> RNA
<213> Artificial sequence

<220>
<221> source
<223> /note= "Description of artificial sequence: synthesized RNA; antisense
      strand of B631bl-19b."

<400> 231
gagacagcca ggagaaauc                                                    19

<210> 232
<211> 19
<212> RNA
<213> Artificial sequence

<220>
<221> source

<223> /note= "Description of artificial sequence: synthesized RNA; antisense
      strand of B634bl-19b."

<400> 232
ucagagacag ccaggagaa                                                19

<210> 233
<211> 19
<212> RNA
<213> Artificial sequence

<220>
<221> source
<223> /note= "Description of artificial sequence: synthesized RNA; antisense
      strand of B717bl-19b."

<400> 233
gcaggcaugu ugacuucac                                                19

<210> 234
<211> 18
<212> RNA
<213> Artificial sequence

<220>
<221> source
<223> /note= "Description of artificial sequence: synthesized RNA; antisense
      strand of B043bl-18b."

<400> 234
auggauguac uucaucac                                                 18

<210> 235
<211> 18
<212> RNA
<213> Artificial sequence

<220>
<221> source
<223> /note= "Description of artificial sequence: synthesized RNA; antisense
      strand of B044bl-18b."

<400> 235
aauggaugua cuucauca                                                 18

<210> 236
<211> 18
<212> RNA
<213> Artificial sequence

<220>
<221> source
<223> /note= "Description of artificial sequence: synthesized RNA; antisense
      strand of B469bl-18b."

<400> 236
gacgcucucc acacacau                                                 18

<210> 237

<211> 18
<212> RNA
<213> Artificial sequence

<220>
<221> source
<223> /note= "Description of artificial sequence: synthesized RNA; antisense
      strand of B470bl-18b."

<400> 237
ugacgcucuc cacacaca                                                18

<210> 238
<211> 18
<212> RNA
<213> Artificial sequence

<220>
<221> source
<223> /note= "Description of artificial sequence: synthesized RNA; antisense
      strand of B534bl-18b."

<400> 238
ugccgguuca gguacuca                                                18

<210> 239
<211> 18
<212> RNA
<213> Artificial sequence

<220>
<221> source
<223> /note= "Description of artificial sequence: synthesized RNA; antisense
      strand of B718bl-18b."

<400> 239
gcaggcaugu ugacuuca                                                18

<210> 240
<211> 21
<212> DNA
<213> Artificial sequence

<220>
<221> source
<223> /note= "Description of artificial sequence: synthesized DNA/RNA; sense
      strand of B434, wherein 2 nucleotides of 3'-terminus are constructed with
      DNA, and the rest are RNA."

<400> 240
ggaggauugu ggccuucuut t                                                21

<210> 241
<211> 21
<212> DNA
<213> Artificial sequence

<220>
<221> source

<223> /note= "Description of artificial sequence: synthesized DNA/RNA; sense
      strand of B435, wherein 2 nucleotides of 3'-terminus are constructed with
      DNA, and the rest are RNA."

<400> 241
gaggauugug gccuucuuut t                                                21

<210> 242
<211> 21
<212> DNA
<213> Artificial sequence

<220>
<221> source
<223> /note= "Description of artificial sequence: synthesized DNA/RNA; sense
      strand of B437, wherein 2 nucleotides of 3'-terminus are constructed with
      DNA, and the rest are RNA."

<400> 242
ggauuguggc cuucuuugat t                                                21

<210> 243
<211> 21
<212> DNA
<213> Artificial sequence

<220>
<221> source
<223> /note= "Description of artificial sequence: synthesized DNA/RNA; sense
      strand of B438, wherein 2 nucleotides of 3'-terminus are constructed with
      DNA, and the rest are RNA."

<400> 243
gauuguggcc uucuuugagt t                                                21

<210> 244
<211> 21
<212> DNA
<213> Artificial sequence

<220>
<221> source
<223> /note= "Description of artificial sequence: synthesized DNA/RNA; sense
      strand of B467, wherein 2 nucleotides of 3'-terminus are constructed with
      DNA, and the rest are RNA."

<400> 244
ucaugugugu ggagagcgut t                                                21

<210> 245
<211> 21
<212> DNA
<213> Artificial sequence

<220>
<221> source
<223> /note= "Description of artificial sequence: synthesized DNA/RNA; sense
      strand of B468, wherein 2 nucleotides of 3'-terminus are constructed with
      DNA, and the rest are RNA."

<400> 245
caugugugug gagagcguct t                                                21

<210> 246
<211> 21
<212> DNA
<213> Artificial sequence

<220>
<221> source
<223> /note= "Description of artificial sequence: synthesized DNA/RNA; sense
      strand of B470, wherein 2 nucleotides of 3'-terminus are constructed with
      DNA, and the rest are RNA."

<400> 246
ugugugugga gagcgucaat t                                                21

<210> 247
<211> 21
<212> DNA
<213> Artificial sequence

<220>
<221> source
<223> /note= "Description of artificial sequence: synthesized DNA/RNA; sense
      strand of B471, wherein 2 nucleotides of 3'-terminus are constructed with
      DNA, and the rest are RNA."

<400> 247
gcguguggag agcgucaact t                                                21

<210> 248
<211> 21
<212> DNA
<213> Artificial sequence

<220>
<221> source
<223> /note= "Description of artificial sequence: synthesized DNA/RNA; sense
      strand of B535, wherein 2 nucleotides of 3'-terminus are constructed with
      DNA, and the rest are RNA."

<400> 248
gaguaccuga accggcacct t                                                21

<210> 249
<211> 21
<212> DNA
<213> Artificial sequence

<220>
<221> source
<223> /note= "Description of artificial sequence: synthesized DNA/RNA; sense
      strand of B629, wherein 2 nucleotides of 3'-terminus are constructed with
      DNA, and the rest are RNA."

<400> 249
uugauuucuc cuggcuguct t                                                21

<210> 250
<211> 21
<212> DNA
<213> Artificial sequence

<220>
<221> source
<223> /note= "Description of artificial sequence: synthesized DNA/RNA; sense
      strand of B630, wherein 2 nucleotides of 3'-terminus are constructed with
      DNA, and the rest are RNA."

<400> 250
ugauuucucc uggcugucut t                                                21

<210> 251
<211> 21
<212> DNA
<213> Artificial sequence

<220>
<221> source
<223> /note= "Description of artificial sequence: synthesized DNA/RNA; sense
      strand of B632, wherein 2 nucleotides of 3'-terminus are constructed with
      DNA, and the rest are RNA."

<400> 251
auuucuccug gcugucucut t                                                21

<210> 252
<211> 21
<212> DNA
<213> Artificial sequence

<220>
<221> source
<223> /note= "Description of artificial sequence: synthesized DNA/RNA; sense
      strand of B633, wherein 2 nucleotides of 3'-terminus are constructed with
      DNA, and the rest are RNA."

<400> 252
uuucuccugg cugucucugt t                                                21

<210> 253
<211> 21
<212> DNA
<213> Artificial sequence

<220>
<221> source
<223> /note= "Description of artificial sequence: synthesized DNA/RNA; sense
      strand of B714, wherein 2 nucleotides of 3'-terminus are constructed with
      DNA, and the rest are RNA."

<400> 253
caagugaagu caacaugcct t                                                21

<210> 254
<211> 21

<212> DNA
<213> Artificial sequence

<220>
<221> source
<223> /note= "Description of artificial sequence: synthesized DNA/RNA; sense
      strand of B715, wherein 2 nucleotides of 3'-terminus are constructed with
      DNA, and the rest are RNA."

<400> 254
aagugaaguc aacaugccut t                                                21

<210> 255
<211> 21
<212> DNA
<213> Artificial sequence

<220>
<221> source
<223> /note= "Description of artificial sequence: synthesized DNA/RNA; sense
      strand of B716, wherein 2 nucleotides of 3'-terminus are constructed with
      DNA, and the rest are RNA."

<400> 255
agugaaguca acaugccugt t                                                21

<210> 256
<211> 21
<212> DNA
<213> Artificial sequence

<220>
<221> source
<223> /note= "Description of artificial sequence: synthesized DNA/RNA; sense
      strand of B718, wherein 2 nucleotides of 3'-terminus are constructed with
      DNA, and the rest are RNA."

<400> 256
ugaagucaac augccugcct t                                                21

<210> 257
<211> 21
<212> DNA
<213> Artificial sequence

<220>
<221> source
<223> /note= "Description of artificial sequence: synthesized DNA/RNA; antisense
      strand of B434, wherein 2 nucleotides of 3'-terminus are constructed with
      DNA, and the rest are RNA."

<400> 257
aagaaggcca caauccucct t                                                21

<210> 258
<211> 21
<212> DNA
<213> Artificial sequence

<220>
<221> source
<223> /note= "Description of artificial sequence: synthesized DNA/RNA; antisense
      strand of B435, wherein 2 nucleotides of 3'-terminus are constructed with
      DNA, and the rest are RNA."

<400> 258
aaagaaggcc acaauccuct t                                                21

<210> 259
<211> 21
<212> DNA
<213> Artificial sequence

<220>
<221> source
<223> /note= "Description of artificial sequence: synthesized DNA/RNA; antisense
      strand of B437, wherein 2 nucleotides of 3'-terminus are constructed with
      DNA, and the rest are RNA."

<400> 259
ucaaagaagg ccacaaucct t                                                21

<210> 260
<211> 21
<212> DNA
<213> Artificial sequence

<220>
<221> source
<223> /note= "Description of artificial sequence: synthesized DNA/RNA; antisense
      strand of B438, wherein 2 nucleotides of 3'-terminus are constructed with
      DNA, and the rest are RNA."

<400> 260
cucaaagaag gccacaauct t                                                21

<210> 261
<211> 21
<212> DNA
<213> Artificial sequence

<220>
<221> source
<223> /note= "Description of artificial sequence: synthesized DNA/RNA; antisense
      strand of B467, wherein 2 nucleotides of 3'-terminus are constructed with
      DNA, and the rest are RNA."

<400> 261
acgcucucca cacacaugat t                                                21

<210> 262
<211> 21
<212> DNA
<213> Artificial sequence

<220>
<221> source

<223> /note= "Description of artificial sequence: synthesized DNA/RNA; antisense
      strand of B468, wherein 2 nucleotides of 3'-terminus are constructed with
      DNA, and the rest are RNA."

<400> 262
gacgcucucc acacacaugt t                                                21

<210> 263
<211> 21
<212> DNA
<213> Artificial sequence

<220>
<221> source
<223> /note= "Description of artificial sequence: synthesized DNA/RNA; antisense
      strand of B470, wherein 2 nucleotides of 3'-terminus are constructed with
      DNA, and the rest are RNA."

<400> 263
uugacgcucu ccacacacat t                                                21

<210> 264
<211> 21
<212> DNA
<213> Artificial sequence

<220>
<221> source
<223> /note= "Description of artificial sequence: synthesized DNA/RNA; antisense
      strand of B471, wherein 2 nucleotides of 3'-terminus are constructed with
      DNA, and the rest are RNA."

<400> 264
guugacgcuc uccacacact t                                                21

<210> 265
<211> 21
<212> DNA
<213> Artificial sequence

<220>
<221> source
<223> /note= "Description of artificial sequence: synthesized DNA/RNA; antisense
      strand of B535, wherein 2 nucleotides of 3'-terminus are constructed with
      DNA, and the rest are RNA."

<400> 265
ggugccgguu cagguacuct t                                                21

<210> 266
<211> 21
<212> DNA
<213> Artificial sequence

<220>
<221> source
<223> /note= "Description of artificial sequence: synthesized DNA/RNA; antisense
      strand of B629, wherein 2 nucleotides of 3'-terminus are constructed with
      DNA, and the rest are RNA."

<400> 266
gacagccagg agaaaucaat t                                                21

<210> 267
<211> 21
<212> DNA
<213> Artificial sequence

<220>
<221> source
<223> /note= "Description of artificial sequence: synthesized DNA/RNA; antisense
      strand of B630, wherein 2 nucleotides of 3'-terminus are constructed with
      DNA, and the rest are RNA."

<400> 267
agacagccag gagaaaucat t                                                21

<210> 268
<211> 21
<212> DNA
<213> Artificial sequence

<220>
<221> source
<223> /note= "Description of artificial sequence: synthesized DNA/RNA; antisense
      strand of B632, wherein 2 nucleotides of 3'-terminus are constructed with
      DNA, and the rest are RNA."

<400> 268
agagacagcc aggagaaaut t                                                21

<210> 269
<211> 21
<212> DNA
<213> Artificial sequence

<220>
<221> source
<223> /note= "Description of artificial sequence: synthesized DNA/RNA; antisense
      strand of B633, wherein 2 nucleotides of 3'-terminus are constructed with
      DNA, and the rest are RNA."

<400> 269
cagagacagc caggagaaat t                                                21

<210> 270
<211> 21
<212> DNA
<213> Artificial sequence

<220>
<221> source
<223> /note= "Description of artificial sequence: synthesized DNA/RNA; antisense
      strand of B714, wherein 2 nucleotides of 3'-terminus are constructed with
      DNA, and the rest are RNA."

<400> 270
ggcauguuga cuucacuugt t                                                21

<210> 271
<211> 21
<212> DNA
<213> Artificial sequence

<220>
<221> source
<223> /note= "Description of artificial sequence: synthesized DNA/RNA; antisense
      strand of B715, wherein 2 nucleotides of 3'-terminus are constructed with
      DNA, and the rest are RNA."

<400> 271
aggcauguug acuucacuut t                                                21

<210> 272
<211> 21
<212> DNA
<213> Artificial sequence

<220>
<221> source
<223> /note= "Description of artificial sequence: synthesized DNA/RNA; antisense
      strand of B716, wherein 2 nucleotides of 3'-terminus are constructed with
      DNA, and the rest are RNA."

<400> 272
caggcauguu gacuucacut t                                                21

<210> 273
<211> 21
<212> DNA
<213> Artificial sequence

<220>
<221> source
<223> /note= "Description of artificial sequence: synthesized DNA/RNA; antisense
      strand of B718, wherein 2 nucleotides of 3'-terminus are constructed with
      DNA, and the rest are RNA."

<400> 273
ggcaggcaug uugacuucat t                                                21

<210> 274
<211> 21
<212> DNA
<213> Artificial sequence

<220>
<221> source
<223> /note= "Description of artificial sequence: synthesized DNA/RNA; sense
      strand of B521, wherein 2 nucleotides of 3'-terminus are constructed with
      DNA, and the rest are RNA."

<400> 274
cccuguggau gacugaguat t                                                21

<210> 275
<211> 21

<212> DNA
<213> Artificial sequence

<220>
<221> source
<223> /note= "Description of artificial sequence: synthesized DNA/RNA; sense
      strand of B557, wherein 2 nucleotides of 3'-terminus are constructed with
      DNA, and the rest are RNA."

<400> 275
acaccuggau ccaggauaat t                                                21

<210> 276
<211> 21
<212> DNA
<213> Artificial sequence

<220>
<221> source
<223> /note= "Description of artificial sequence: synthesized DNA/RNA; sense
      strand of B584, wherein 2 nucleotides of 3'-terminus are constructed with
      DNA, and the rest are RNA."

<400> 276
gggaugccuu uguggaacut t                                                21

<210> 277
<211> 21
<212> DNA
<213> Artificial sequence

<220>
<221> source
<223> /note= "Description of artificial sequence: synthesized DNA/RNA; sense
      strand of B635, wherein 2 nucleotides of 3'-terminus are constructed with
      DNA, and the rest are RNA."

<400> 277
ucuccuggcu gucucugaat t                                                21

<210> 278
<211> 21
<212> DNA
<213> Artificial sequence

<220>
<221> source
<223> /note= "Description of artificial sequence: synthesized DNA/RNA; sense
      strand of B734, wherein 2 nucleotides of 3'-terminus are constructed with
      DNA, and the rest are RNA."

<400> 278
gccccaaaca aauaugcaat t                                                21

<210> 279
<211> 21
<212> DNA
<213> Artificial sequence

<220>
<221> source
<223> /note= "Description of artificial sequence: synthesized DNA/RNA; sense
      strand of B735, wherein 2 nucleotides of 3'-terminus are constructed with
      DNA, and the rest are RNA."

<400> 279
ccccaaacaa auaugcaaat t                                                21

<210> 280
<211> 21
<212> DNA
<213> Artificial sequence

<220>
<221> source
<223> /note= "Description of artificial sequence: synthesized DNA/RNA; sense
      strand of B736, wherein 2 nucleotides of 3'-terminus are constructed with
      DNA, and the rest are RNA."

<400> 280
cccaaacaaa uaugcaaaat t                                                21

<210> 281
<211> 21
<212> DNA
<213> Artificial sequence

<220>
<221> source
<223> /note= "Description of artificial sequence: synthesized DNA/RNA; antisense
      strand of B521, wherein 2 nucleotides of 3'-terminus are constructed with
      DNA, and the rest are RNA."

<400> 281
uacucaguca uccacagggt t                                                21

<210> 282
<211> 21
<212> DNA
<213> Artificial sequence

<220>
<221> source
<223> /note= "Description of artificial sequence: synthesized DNA/RNA; antisense
      strand of B557, wherein 2 nucleotides of 3'-terminus are constructed with
      DNA, and the rest are RNA."

<400> 282
uuauccugga uccaggugut t                                                21

<210> 283
<211> 21
<212> DNA
<213> Artificial sequence

<220>
<221> source

<223> /note= "Description of artificial sequence: synthesized DNA/RNA; antisense
      strand of B584, wherein 2 nucleotides of 3'-terminus are constructed with
      DNA, and the rest are RNA."

<400> 283
aguuccacaa aggcauccct t                                                21

<210> 284
<211> 21
<212> DNA
<213> Artificial sequence

<220>
<221> source
<223> /note= "Description of artificial sequence: synthesized DNA/RNA; antisense
      strand of B635, wherein 2 nucleotides of 3'-terminus are constructed with
      DNA, and the rest are RNA."

<400> 284
uucagagaca gccaggagat t                                                21

<210> 285
<211> 21
<212> DNA
<213> Artificial sequence

<220>
<221> source
<223> /note= "Description of artificial sequence: synthesized DNA/RNA; antisense
      strand of B734, wherein 2 nucleotides of 3'-terminus are constructed with
      DNA, and the rest are RNA."

<400> 285
uugcauauuu guuuggggct t                                                21

<210> 286
<211> 21
<212> DNA
<213> Artificial sequence

<220>
<221> source
<223> /note= "Description of artificial sequence: synthesized DNA/RNA; antisense
      strand of B735, wherein 2 nucleotides of 3'-terminus are constructed with
      DNA, and the rest are RNA."

<400> 286
uuugcauauu uguuuggggt t                                                21

<210> 287
<211> 21
<212> DNA
<213> Artificial sequence

<220>
<221> source
<223> /note= "Description of artificial sequence: synthesized DNA/RNA; antisense
      strand of B736, wherein 2 nucleotides of 3'-terminus are constructed with
      DNA, and the rest are RNA."

<400> 287
uuuugcauau uuguuugggt t                                                21

<210> 288
<211> 27
<212> DNA
<213> Artificial sequence

<220>
<221> source
<223> /note= "Description of artificial sequence: synthesized DNA/RNA; sense
      strand of B037-25, wherein 2 nucleotide of 3'-terminus are constructed
      with DNA, and the rest are RNA."

<400> 288
gagauaguga ugaaguacau ccauutt                                          27

<210> 289
<211> 27
<212> DNA
<213> Artificial sequence

<220>
<221> source
<223> /note= "Description of artificial sequence: synthesized DNA/RNA; sense
      strand of B038-25, wherein 2 nucleotide of 3'-terminus are constructed
      with DNA, and the rest are RNA."

<400> 289
agauagugau gaaguacauc cauuatt                                          27

<210> 290
<211> 27
<212> DNA
<213> Artificial sequence

<220>
<221> source
<223> /note= "Description of artificial sequence: synthesized DNA/RNA; sense
      strand of B039-25, wherein 2 nucleotide of 3'-terminus are constructed
      with DNA, and the rest are RNA."

<400> 290
gauagugaug aaguacaucc auuautt                                          27

<210> 291
<211> 27
<212> DNA
<213> Artificial sequence

<220>
<221> source
<223> /note= "Description of artificial sequence: synthesized DNA/RNA; sense
      strand of B040-25, wherein 2 nucleotide of 3'-terminus are constructed
      with DNA, and the rest are RNA."

<400> 291
auagugauga aguacaucca uuauatt                                          27

<210> 292
<211> 27
<212> DNA
<213> Artificial sequence

<220>
<221> source
<223> /note= "Description of artificial sequence: synthesized DNA/RNA; sense
      strand of B041-25, wherein 2 nucleotide of 3'-terminus are constructed
      with DNA, and the rest are RNA."

<400> 292
uagugaugaa guacauccau uauaatt                                          27

<210> 293
<211> 27
<212> DNA
<213> Artificial sequence

<220>
<221> source
<223> /note= "Description of artificial sequence: synthesized DNA/RNA; sense
      strand of B042-25, wherein 2 nucleotide of 3'-terminus are constructed
      with DNA, and the rest are RNA."

<400> 293
agugaugaag uacauccauu auaagtt                                          27

<210> 294
<211> 27
<212> DNA
<213> Artificial sequence

<220>
<221> source
<223> /note= "Description of artificial sequence: synthesized DNA/RNA; sense
      strand of B043-25, wherein 2 nucleotide of 3'-terminus are constructed
      with DNA, and the rest are RNA."

<400> 294
gugaugaagu acauccauua uaagctt                                          27

<210> 295
<211> 27
<212> DNA
<213> Artificial sequence

<220>
<221> source
<223> /note= "Description of artificial sequence: synthesized DNA/RNA; sense
      strand of B430-25, wherein 2 nucleotide of 3'-terminus are constructed
      with DNA, and the rest are RNA."

<400> 295
   ugggggagga uuguggccuu cuuugtt                                          27

<210> 296
<211> 27

<212> DNA
<213> Artificial sequence

<220>
<221> source
<223> /note= "Description of artificial sequence: synthesized DNA/RNA; sense
      strand of B463-25, wherein 2 nucleotide of 3'-terminus are constructed
      with DNA, and the rest are RNA."

<400> 296
ggggucaugu guguggagag cgucatt                                          27

<210> 297
<211> 27
<212> DNA
<213> Artificial sequence

<220>
<221> source
<223> /note= "Description of artificial sequence: synthesized DNA/RNA; sense
      strand of B527-25, wherein 2 nucleotide of 3'-terminus are constructed
      with DNA, and the rest are RNA."

<400> 297
ggaugacuga guaccugaac cggcatt                                          27

<210> 298
<211> 27
<212> DNA
<213> Artificial sequence

<220>
<221> source
<223> /note= "Description of artificial sequence: synthesized DNA/RNA; sense
      strand of B608-25, wherein 2 nucleotide of 3'-terminus are constructed
      with DNA, and the rest are RNA."

<400> 298
gccccagcau gcggccucug uuugatt                                          27

<210> 299
<211> 27
<212> DNA
<213> Artificial sequence

<220>
<221> source
<223> /note= "Description of artificial sequence: synthesized DNA/RNA; sense
      strand of B625-25, wherein 2 nucleotide of 3'-terminus are constructed
      with DNA, and the rest are RNA."

<400> 299
cuguuugauu ucuccuggcu gucuctt                                          27

<210> 300
<211> 27
<212> DNA
<213> Artificial sequence

<220>
<221> source
<223> /note= "Description of artificial sequence: synthesized DNA/RNA; sense
      strand of B711-25, wherein 2 nucleotide of 3'-terminus are constructed
      with DNA, and the rest are RNA."

<400> 300
ccacaaguga agucaacaug ccugctt                                          27

<210> 301
<211> 27
<212> RNA
<213> Artificial sequence

<220>
<221> source
<223> /note= "Description of artificial sequence: synthesized RNA; sense strand
      of B037SSCH25."

<400> 301
gagauaguga ugaaguacau ccauuau                                          27

<210> 302
<211> 27
<212> RNA
<213> Artificial sequence

<220>
<221> source
<223> /note= "Description of artificial sequence: synthesized RNA; sense strand
      of B037bl25."

<400> 302
gagauaguga ugaaguacau ccauu                                            25

<210> 303
<211> 27
<212> RNA
<213> Artificial sequence

<220>
<221> source
<223> /note= "Description of artificial sequence: synthesized RNA; sense strand
      of B035bl27."

<400> 303
gggagauagu gaugaaguac auccauu                                          27

<210> 304
<211> 27
<212> RNA
<213> Artificial sequence

<220>
<221> source
<223> /note= "Description of artificial sequence: synthesized RNA; sense strand
      of B039bl27."

<400> 304

gauagugaug aaguacaucc auuauaa                                          27

<210> 305
<211> 27
<212> DNA
<213> Artificial sequence

<220>
<221> source
<223> /note= "Description of artificial sequence: synthesized DNA/RNA; sense
      strand of B037-25-dC-S, wherein C nucleotides and 2 nucleotide of 3'-
      terminus are constructed with DNA, and the rest are RNA."

<400> 305
gagauaguga ugaaguacau ccauutt                                          27

<210> 306
<211> 27
<212> DNA
<213> Artificial sequence

<220>
<221> source
<223> /note= "Description of artificial sequence: synthesized DNA/RNA; sense
      strand of B037-25-dG-S, wherein G nucleotides and 2 nucleotide of 3'-
      terminus are constructed with DNA, and the rest are RNA."

<400> 306
gagauaguga ugaaguacau ccauutt                                          27

<210> 307
<211> 27
<212> DNA
<213> Artificial sequence

<220>
<221> source
<223> /note= "Description of artificial sequence: synthesized DNA/RNA; sense
      strand of B037-25-dGC-S, wherein G nucleotides, C nucleotides and 2
      nucleotide of 3'-terminus are constructed with DNA, and the rest are RNA."

<400> 307
gagauaguga ugaaguacau ccauutt                                          27


<210> 308
<211> 27
<212> DNA
<213> Artificial sequence

<220>
<221> source
<223> /note= "Description of artificial sequence: synthesized DNA/RNA; sense
      strand of B037-25-10D, wherein 10 nucleotide of 3'-terminus are
      constructed with DNA, and the rest are RNA."

<400> 308
gagauaguga ugaaguacat ccatttt                                          27

<210> 309
<211> 27
<212> DNA
<213> Artificial sequence

<220>
<221> source
<223> /note= "Description of artificial sequence: synthesized DNA/RNA; sense
      strand of B037-25-14D, wherein 14 nucleotide of 3'-terminus are
      constructed with DNA, and the rest are RNA."

<400> 309
gagauaguga ugaagtacat ccatttt                                          27

<210> 310
<211> 27
<212> DNA
<213> Artificial sequence

<220>
<221> source
<223> /note= "Description of artificial sequence: synthesized DNA/RNA; sense
      strand of B037-25-18D, wherein 18 nucleotide of 3'-terminus are
      constructed with DNA, and the rest are RNA."

<400> 310
gagauaguga tgaagtacat ccatttt                                          27

<210> 311
<211> 27
<212> DNA
<213> Artificial sequence

<220>
<221> source
<223> /note= "Description of artificial sequence: synthesized DNA; sense strand
      of B037DNA25."

<400> 311
gagatagtga tgaagtacat ccatttt                                          27

<210> 312
<211> 27
<212> DNA
<213> Artificial sequence

<220>
<221> source
<223> /note= "Description of artificial sequence: synthesized DNA; sense strand
      of B038DNA25."

<400> 312
agatagtgat gaagtacatc cattatt                                          27

<210> 313
<211> 27
<212> DNA
<213> Artificial sequence

<220>
<221> source
<223> /note= "Description of artificial sequence: synthesized DNA; sense strand
      of B039DNA25."

<400> 313
gatagtgatg aagtacatcc attattt                                          27

<210> 314
<211> 27
<212> DNA
<213> Artificial sequence

<220>
<221> source
<223> /note= "Description of artificial sequence: synthesized DNA; sense strand
      of B040DNA25."

<400> 314
atagtgatga agtacatcca ttatatt                                          27

<210> 315
<211> 27
<212> DNA
<213> Artificial sequence

<220>
<221> source
<223> /note= "Description of artificial sequence: synthesized DNA; sense strand
      of B041DNA25."

<400> 315
tagtgatgaa gtacatccat tataatt                                          27

<210> 316
<211> 27
<212> DNA
<213> Artificial sequence

<220>
<221> source
<223> /note= "Description of artificial sequence: synthesized DNA; sense strand
      of B042DNA25."

<400> 316
agtgatgaag tacatccatt ataagtt                                          27

<210> 317
<211> 27
<212> DNA
<213> Artificial sequence

<220>
<221> source
<223> /note= "Description of artificial sequence: synthesized DNA; sense strand
      of B043DNA25."

<400> 317
gtgatgaagt acatccatta taagctt                                          27

<210> 318
<211> 27
<212> DNA
<213> Artificial sequence

<220>
<221> source
<223> /note= "Description of artificial sequence: synthesized DNA; sense strand
      of B037DNA25PS, wherein all phosphodiester bonds are changed into
      phosphorothioate bonds."

<400> 318
gagatagtga tgaagtacat ccatttt                                          27

<210> 319
<211> 27
<212> DNA
<213> Artificial sequence

<220>
<221> source
<223> /note= "Description of artificial sequence: synthesized DNA/RNA; antisense
      strand of B037-25, wherein 2 nucleotide of 3'-terminus are constructed
      with DNA, and the rest are RNA."

<400> 319
aauggaugua cuucaucacu aucuctt                                          27

<210> 320
<211> 27
<212> DNA
<213> Artificial sequence

<220>
<221> source
<223> /note= "Description of artificial sequence: synthesized DNA/RNA; antisense
      strand of B038-25, wherein 2 nucleotide of 3'-terminus are constructed
      with DNA, and the rest are RNA."

<400> 320
uaauggaugu acuucaucac uaucutt                                          27

<210> 321
<211> 27
<212> DNA
<213> Artificial sequence

<220>
<221> source
<223> /note= "Description of artificial sequence: synthesized DNA/RNA; antisense
      strand of B039-25, wherein 2 nucleotide of 3'-terminus are constructed
      with DNA, and the rest are RNA."

<400> 321
auaauggaug uacuucauca cuauctt                                          27

<210> 322
<211> 27

<212> DNA
<213> Artificial sequence

<220>
<221> source
<223> /note= "Description of artificial sequence: synthesized DNA/RNA; antisense
      strand of B040-25, wherein 2 nucleotide of 3'-terminus are constructed
      with DNA, and the rest are RNA."

<400> 322
uauaauggau guacuucauc acuautt                                          27

<210> 323
<211> 27
<212> DNA
<213> Artificial sequence

<220>
<221> source
<223> /note= "Description of artificial sequence: synthesized DNA/RNA; antisense
      strand of B041-25, wherein 2 nucleotide of 3'-terminus are constructed
      with DNA, and the rest are RNA."

<400> 323
uuauaaugga uguacuucau cacuatt                                          27

<210> 324
<211> 27
<212> DNA
<213> Artificial sequence

<220>
<221> source
<223> /note= "Description of artificial sequence: synthesized DNA/RNA; antisense
      strand of B042-25, wherein 2 nucleotide of 3'-terminus are constructed
      with DNA, and the rest are RNA."

<400> 324
cuuauaaugg auguacuuca ucacutt                                          27

<210> 325
<211> 27
<212> DNA
<213> Artificial sequence

<220>
<221> source
<223> /note= "Description of artificial sequence: synthesized DNA/RNA; antisense
      strand of B043-25, wherein 2 nucleotide of 3'-terminus are constructed
      with DNA, and the rest are RNA."

<400> 325
gcuuauaaug gauguacuuc aucactt                                          27

<210> 326
<211> 27
<212> DNA
<213> Artificial sequence

<220>
<221> source
<223> /note= "Description of artificial sequence: synthesized DNA/RNA; antisense
      strand of B430-25, wherein 2 nucleotide of 3'-terminus are constructed
      with DNA, and the rest are RNA."

<400> 326
caaagaaggc cacaauccuc ccccatt                                          27

<210> 327
<211> 27
<212> DNA
<213> Artificial sequence

<220>
<221> source
<223> /note= "Description of artificial sequence: synthesized DNA/RNA; antisense
      strand of B463-25, wherein 2 nucleotide of 3'-terminus are constructed
      with DNA, and the rest are RNA."

<400> 327
ugacgcucuc cacacacaug acccctt                                          27

<210> 328
<211> 27
<212> DNA
<213> Artificial sequence

<220>
<221> source
<223> /note= "Description of artificial sequence: synthesized DNA/RNA; antisense
      strand of B527-25, wherein 2 nucleotide of 3'-terminus are constructed
      with DNA, and the rest are RNA."

<400> 328
ugccgguuca gguacucagu caucctt                                          27

<210> 329
<211> 27
<212> DNA
<213> Artificial sequence

<220>
<221> source
<223> /note= "Description of artificial sequence: synthesized DNA/RNA; antisense
      strand of B608-25, wherein 2 nucleotide of 3'-terminus are constructed
      with DNA, and the rest are RNA."

<400> 329
ucaaacagag gccgcaugcu ggggctt                                          27

<210> 330
<211> 27
<212> DNA
<213> Artificial sequence

<220>
<221> source

<223> /note= "Description of artificial sequence: synthesized DNA/RNA; antisense
      strand of B625-25, wherein 2 nucleotide of 3'-terminus are constructed
      with DNA, and the rest are RNA."

<400> 330
gagacagcca ggagaaauca aacagtt                                          27

<210> 331
<211> 27
<212> DNA
<213> Artificial sequence

<220>
<221> source
<223> /note= "Description of artificial sequence: synthesized DNA/RNA;antisense
      strand of B711-25, wherein 2 nucleotide of 3'-terminus are constructed
      with DNA, and the rest are RNA."

<400> 331
gcaggcaugu ugacuucacu uguggtt                                          27

<210> 332
<211> 27
<212> RNA
<213> Artificial sequence

<220>
<221> source
<223> /note= "Description of artificial sequence: synthesized RNA; antisense
      strand of B037SSOH25."

<400> 332
aauggaugua cuucaucacu aucuccc                                          27

<210> 333
<211> 25
<212> RNA
<213> Artificial sequence

<220>
<221> source
<223> /note= "Description of artificial sequence: synthesized RNA; antisense
      strand of B037bl25."

<400> 333
aauggaugua cuucaucacu aucuc                                            25

<210> 334
<211> 27
<212> RNA
<213> Artificial sequence

<220>
<221> source
<223> /note= "Description of artificial sequence: synthesized RNA; antisense
      strand of B039bl27."

<400> 334
uuauaaugga uguacuucau cacuauc                                          27

## Description

### Technical Field

The presentinvention relatesto an oligo double-stranded RNAfor use in inhibition ofthe expression (knockdown) of Bcl-2 protein which functions as a suppressor for apoptosis, and pharmaceutical compositions containing the oligo double-stranded RNA which serves to treat and/or prevent diseases for which knockdown of Bcl-2 protein or acceleration of apoptosis is desired.

### Background Art

Bcl-2 is known as an oncogene since the Bcl-2 protein works as a suppressor for apoptosis (programmed cell death)(e.g., see Nobuyuki Tanaka, Molecular Medicine, 2002, vol.39, No.6. p.638-644). Suppression of apoptosis by expression of a large quantity of Bcl-2 protein is considered to cause malignant transformation based on no incidence of apoptotic cells due to damage to DNA, and hematological malignant diseases, for example. In fact, Bcl-2 protein is generated in large quantities at the site of a variety of solid cancers such as lymphosarcoma, prostatic cancer, breast cancer, lung cancer, colon cancer, and rectal cancer (e.g., see Y. Tsujimoto, Proceedings of the National Academy of Sciences USA, 1989, May, vol. 86, no. 6, p.1958-1962; T.J. McDonnell et al., Cancer Research, Dec., 15, 1992, vol.52, no.24, p.6940-6944; H. Joensuu et al., American Journal of Pathology, Nov. 1994, vol.145, no.5, p.1191-1198; N. Ikegaki et al., Cancer Research, Jan. 1, 1994, vol.54, no.1, p.6-8; and M.P. Bronner et al., American Journal of Pathology, Jan. 1995, vol.146, no.1, p.20-26). In addition, in the cells in which Bcl-2 protein is generated in large quantities, no cell death is induced even by an anti-cancer agent because of the suppression of apoptosis, resulting in drug resistance to a variety of anti-cancer agents. Thus, inhibition of the expression of Bcl-2 protein could lead to treatment and/or prevention effective in diseases such as solid cancers and hematological malignant disease, for which acceleration of apoptosis is desired.

In the past, anti-sense techniques have been investigated as methods for inhibiting the expression of Bcl-2 proteins (e.g., see WO 98/56905 pamphlet; JP-T-11-501509; JP-T-2001-505401; JP-T-2001-502172; and WO 02/17852A2 pamphlet). The anti-sense techniques comprise introducing an anti-sense DNA having a strand complementary to a target RNA into a cell to form an RNA-DNA double strand between the target RNA and the anti-sense DNA to cleave the target RNA with the action of RNase H, resulting in inhibition of the expression of the protein. In the anti-sense techniques, however, an anti-sense DNA has to be transferred into the nucleus because the RNase H utilized in this technique is present within the nucleus. In addition, anti-sense DNAs are generally singie-stranded oligo DNAs consisting of about 20 bases and degraded with nucleases in a serum-containing medium or in vivo, particularly in blood. The techniques are complicated, accordingly, in the view that anti-sense DNAs have to be chemically modified in various ways such as displacement of the phosphate-binding site of DNA by a phosphorothioate form, to afford nuclease resistance. Further, the anti-sense DNA has to be used in the order of several hundred nM to several ten µM in order to effectively suppress the cell growth by inhibition of the expression of Bcl-2 protein by anti-sense DNAs.

RNAi (RNA interference) is a phenomenon, wherein a double-stranded RNA (dsRNA) is introduced into a cell, and a mRNA complementary to the introduced dsRNA is specifically degraded, resulting in inhibition of synthesis of the gene product coded by the mRNA. Though RNAi was a phenomenon first found in C. elegans (see, e.g., A. Fire et al., Nature, 1998, vol.391, p.806-811), thereafter this was also observed in Drosophila, Trypanosoma, hydra, plants (A. thaliana), and Xenopus, as well as in mammalian cells including mouse embryonic stem cells (ES cells), egg cells and early embryos (e.g., WO02/44321A2 pamphlet; WO01/68836A2 pamphlet; JP-T-2002-516062).

On the other hand, in the differentiated mammalian cells, no effect of RNAi was observed, though dsRNA of 30 bp or more was introduced in order to obtain an inhibitory effect on the expression of a protein by RNAi as in C. elegans. This is due to the activation of two pathways known as interferon response, wherein the invasion of dsRNA of 30 bp or more was recognized as viral infection. In one pathway, interferon induces a dsRNA-deppndent protein kinase (PKR), which is activated binding dsRNA and phosphorylate a translation initiator elF2α to inhibit translation, In the other pathway, interferon induces 2'-5'-oligoadenylate synthase (2-5A synthase), which activates RNase L to cleave a single-stranded RNA such as mRNA. The activation of these pathways reduces the expression of entire genes in cells. Thus, introduction of dsRNA of 30 bp or more did not exert a specific inhibitory (knockdown) effect for the expression of the target gene since the introduction reduced the expression of entire genes in cells through interferon responses (see, e.g , K. Ui-Tei et al., FEBS Letters, 2000, vol,479, p.79-82).

A research group of Tuschl et al. found that an added dsRNA was cleft into dsRNAs of 21 to 23 base pairs in an in vitro system using a cell extract from Drosphila, suggesting that the cleft RNAs worked as guides for recognition of the target mRNA sequence (see, e.g., P.D. Zamore et al., Cell, March 31, 2000, vol.101, no.1, p.25-33). And, it was confirmed practically that the target mRNA was cleft by addition of the 21 to 22 bases of double-stranded RNAs to the above reaction medium. The Tuschl's group designated such a short double-stranded RNA as siRNA (short interfering RNA), which had an activity inhibiting the expression of a protein by RNAi (see, e.g., S.M. Elbashir et al., Genes & Development, Jan. 15, 2001, vol.15, no.2, p.188-200). In addition, it has also been reported that siRNA has a single-stranded portion of 2 to 3 bases at the 3'-terminal of the respective RNA strands constituting siRNA, that is, a so-called overhang, and those having such an overhang exhibit a strong inhibitory activity on the expression of a protein. Further, the Tuschl's group has reported that siRNA shows a sequence-specific inhibitory activity on the gene expression also in the differentiated mammal cells without any problem of the above-mentioned interferon response (see, e.g., WO 01/75164A2 pamphlet; and S.M. Elbashir et al., Nature, May 24, 2001, vol.411, p.494-498). Thus, the use of siRNA allows the inhibition of the expression of a protein coded by a target gene in mammals, by means of knockdown of the target gene utilizing RNAi.

As a protein necessary for the cleavage of a target mRNA in inhibition of the protein expression utilizing RNAi, a protein complex designated as RISC (RNA-induced silencing complex; nucleotidase complex) has been considered. RISC is considered to incorporate siRNA into the complex through recognition of overhang at the 3'-terminal of siRNA having double strand and thereby to recognize the target mRNA by using the incorporated siRNA as a guide. That is, the complex seems to recognize and bind mRNA having the same sequence as siRNA and cleave the mRNA in the middle portion of siRNA by an RNase III-like enzymatic activity.

Since RISC is formed in cytoplasm, in a method of knockdown of the target gene utilizing RNAi, there is an advantage that it is sufficient to introduce siRNA simply into cytoplasm, that is, transferring it into the nucleus is not necessary. siRNA is not necessarily modified since siRNA exerts a sufficient effect in the form of a naturally occurring nucleotide constitution. Moreover, the amount of siRNA to be introduced to attain a sufficient effect is extremely small as compared with that of an anti-sense DNA to be introduced in an anti-sense technique; thus, this has attracted a great deal of attention as a convenient and effective method. RNAi techniques using siRNA have been reviewed in Takashi Morita and Kayo Yoshida, Tanpakusitsu/Kakusan/Kohso (Proteins, Nucleic Acids and Enzymes), 2002, vol.47, p.1939-1945; Senri Ushida, Tanpakusitsu/Kakusan/Kohso, 2001, vol.46, p.1381-1386; Hiroaki Tahara, Tanpakusitsu/Kakusan/ Kohso, 2001, vol.46, p.2017-2024; Tatsushi Igaki and Masayuki Miura, Idenshi Igaku, 2002, vol.6, p.455-460; and J. Martinez et al., Cell, Sept. 6, 2002, vol.110, no.5, p.563-574. In addition, there is a report indicating that the RNAi effect has been shown by an anti-sense strand alone as well (see, e.g. J. Martinez et al., Cell, Sept. 6, 2002, vol.110, no.5, p.563-574) .

Methods for introducing siRNA into cytoplasm include a method using as a vehicle a carrier such as cationic liposome or other cationic carrier, and a method of direct introduction into a cell such as calcium phosphate method, electroporation or microinjection, in addition, a method of introducing into a cell an expression vector incorporated with an siRNA-coding sequence so as to express siRNA in the cell has also been investigated (see, e.g., Miyagishi et al., Nature Biotechnology, May 2002, vol.20, no.5, p.497-500: N.S. Lee et al., Nature Biotechnology, May 2002, vol.20, no. 5, p.500-505; and T.R. Brummelkamp et al.. Science, April 19, 2002, vol.296, p.550-553).

There are several reports on siRNA directed to bcl-2 mRNA (see, e.g., WO02/055692A2 pamphlet; WO02/055693A2 pamphlet; T. Futami et al., Nucleic Acids Research, Supplement, 2002, no.2, p.251-252; D.P. Cioca and Y. Aoki, Cancer Gene Therapy, 2003, no.10, p.125-133; and M. Jiang and J. Milner, Genes and Development, 2003, no.17, p.832-837); these reports disclose siRNA in which the sequences of oligo double-strand are different from that of the present invention, or no specific sequence is disclosed.

### Disclosure of Invention

The present invention provides an oligo double-stranded RNA which functions to inhibit the expression of Bcl-2 protein acting as a suppressor for apoptosis. In addition, the present invention provides a pharmaceutical composition for treatment and/or prevention of diseases for which acceleration of apoptosis is desired, including cancers or hematological malignant diseases.

### Brief Description of Drawings

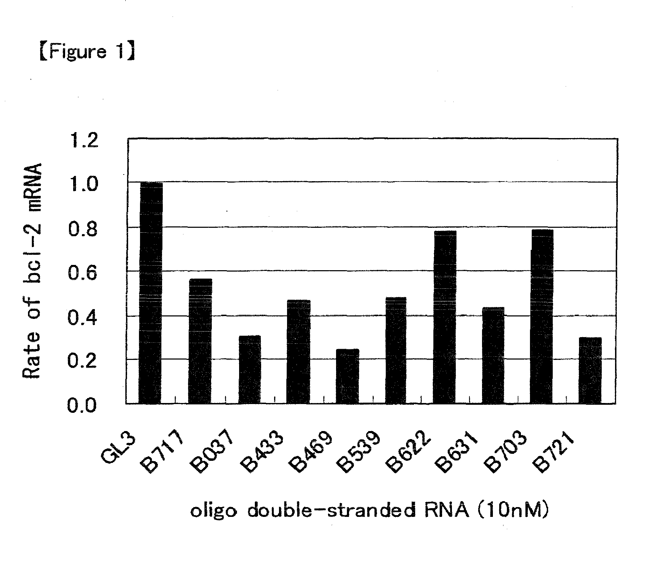

Fig. 1 shows the result of semi-quantification by RT-PCR of the bcl-2 mRNA in A431 cells, wherein the cells were transfected with the respective oligo double-stranded RNAs obtained by a primary screening, and incubated for 24 hours. The vertical axis indicates the relative amount of bcl-2 mRNA in the cells transfected with the respective oligo double-stranded RNAs, wherein the amount of bcl-2 mRNA in transfection of a negative control GL3 was regarded as 1. Under the horizontal axis are indicated the names of the olig double-stranded RNAs examined.

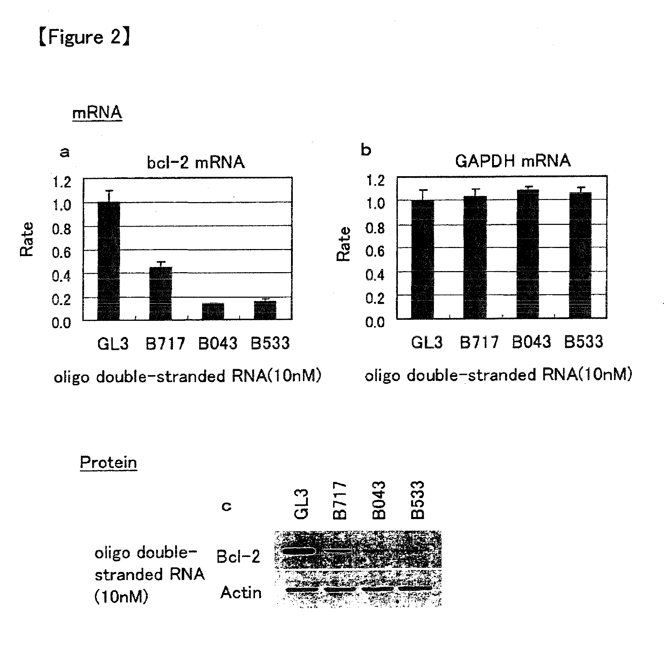

Fig. 2a and b show the results of semi-quantification of the bcl-2 mRNA (Fig. 2a) and the constitutively expressed gene GADPH mRNA (Fig. 2b) in A431 cells by RT-PCR, wherein the cells were transfected with the respective oligo double-stranded RNAs of B717, B043 and B533, and incubated for 24 hours. Fig. 2c shows the result of an evaluation of the expression of Bcl-2 protein in A431 cells by Western blotting, wherein the cells were transfected with the respective B717, B043 and B533 at a final concentration of 10 nM and incubated for 72 hours.

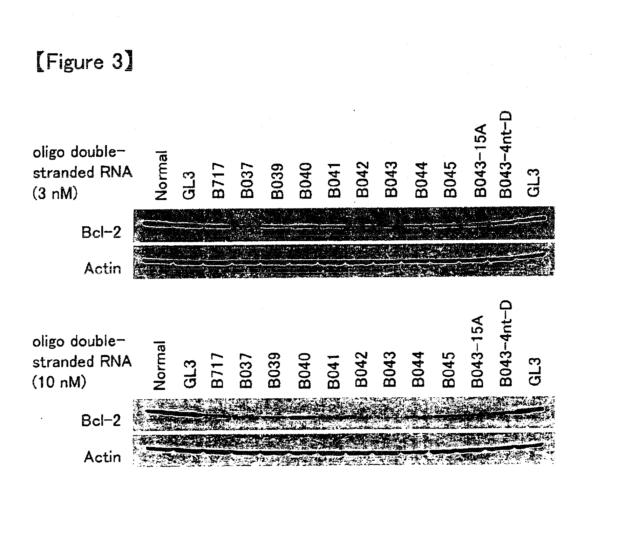

Fig. 3 shows the result of an evaluation performed by Western blotting of the inhibitory activity of oligo double-stranded RNA on the expression of Bcl-2 protein. The upper case indicates the result wherein A431 cells were transfected with the respective RNAs at a final concentration of 3 nM and incubated for 72 hours, and the lower case, at a final concentration of 10 nM.

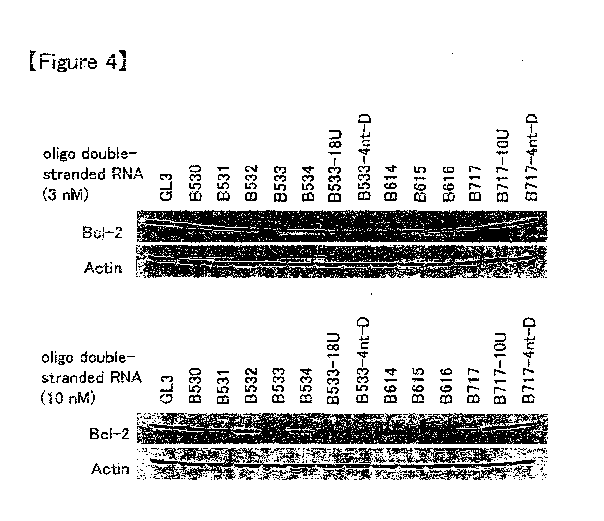

Fig. 4 shows the result of an evaluation performed by Western blotting of the inhibitory activity of oligo double-stranded RNA on the expression of Bcl-2 protein. The upper case indicates the result wherein A431 cells were transfected with the respective RNAs at a final concentration of 3 nM and incubated for 72 hours, and the lower case, at a final concentration of 10 nM.

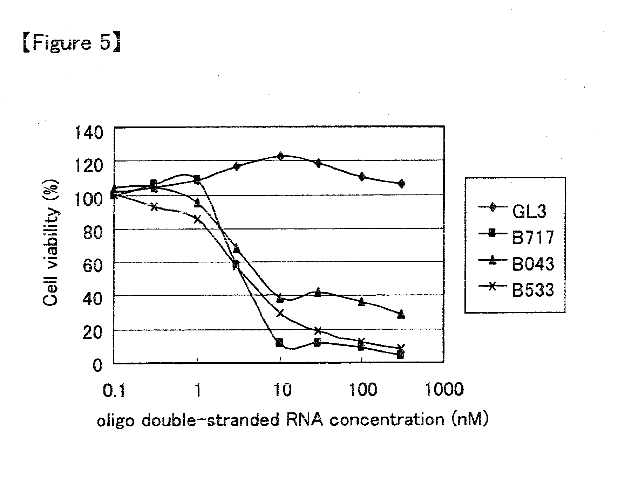

Fig. 5 shows the result of an evaluation performed by a cell counting assay of the effect on the cell growth of the selected oligo double-stranded RNAs. A431 cells were transfected with an oligo double-stranded RNA, then incubated for 6 days, and measured for cell numbers. The vertical axis indicates the rate of the cell number when transfected with the respective oligo double-stranded RNAs. The cell number of the non-transfected A431 cells was regarded as 100%. The horizontal axis indicates the final concentration after transfection.

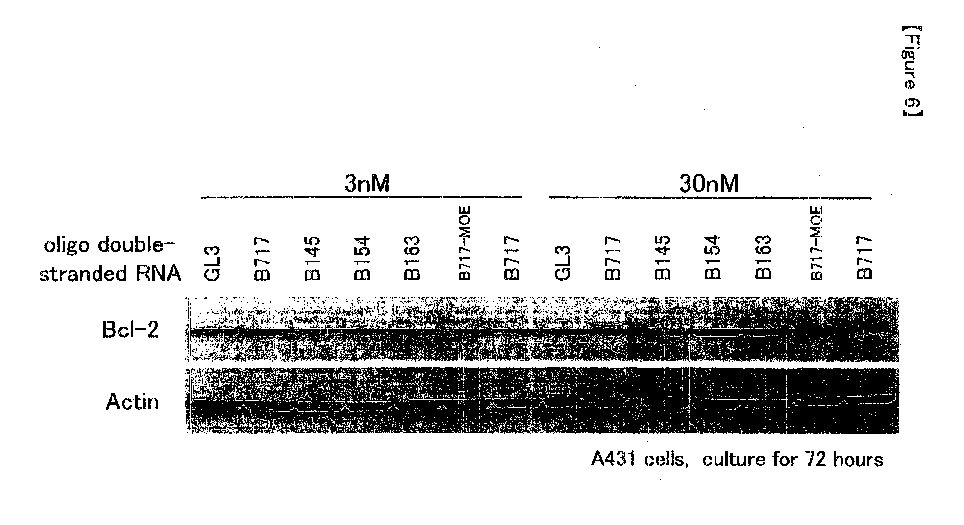

Fig. 6 shows the result of an evaluation performed by Western blotting of an inhibitory activity of oligo double-stranded RNA on the expression of Bcl-2 protein. The result was obtained by transfection of the cells the oligo double-stranded RNA at a final concentration of 3 nM or 10 nM, followed by incubation for 72 hours.

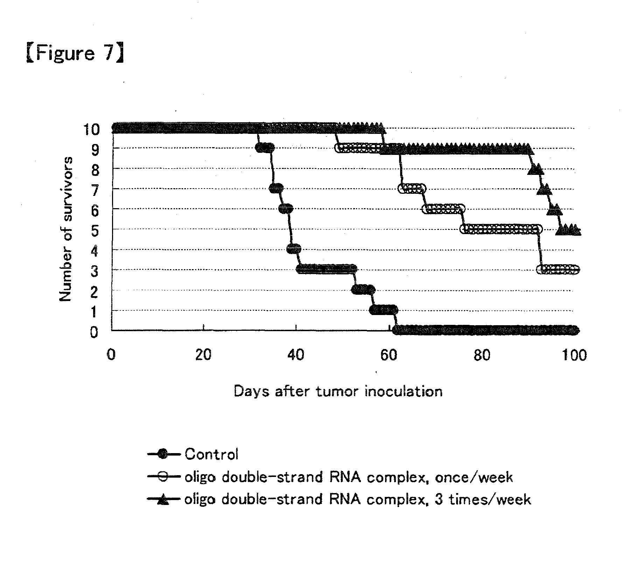

Fig. 7 shows the survival number of mice up to 100 days after inoculation of A549 cells. The symbol •, indicates a control group; o, a group to which B717 of oligo double-stranded RNA was administered once a week; and ▲, a group to which B717 of oligo double-stranded RNA was administered 3 times a week.

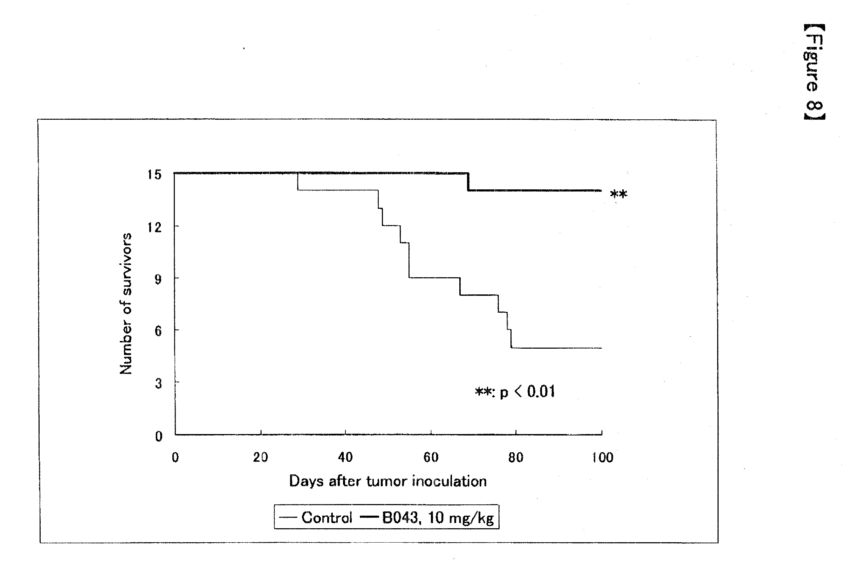

Fig. 8 shows the survival number of mice up to 100 days after inoculation of A549 cells. The fine line indicates a control group; and the bold line indicates a group to which B043 of oligo double-stranded RNA was administered.

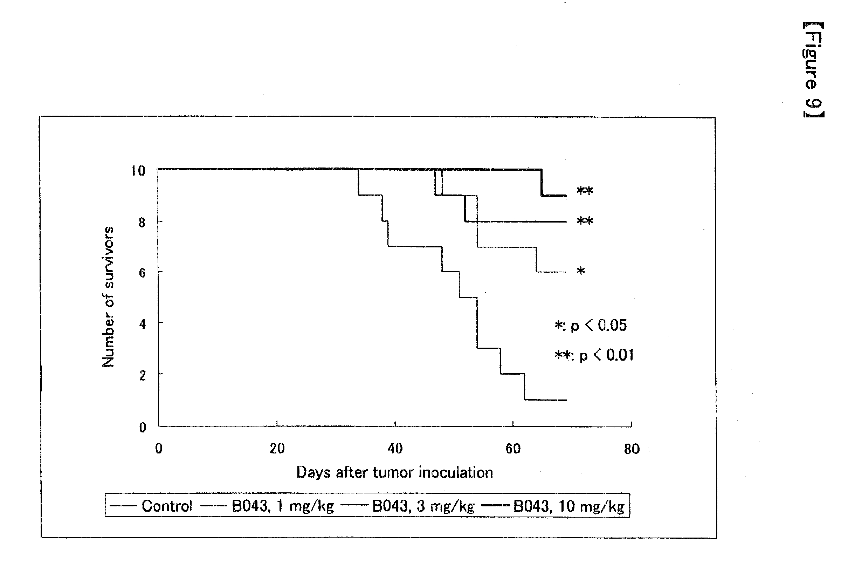

Fig. 9 shows the survival number of mice up to 69 days after inoculation of A549 cells. The fine line indicates a control group: the dotted line, a group to which B043 of oligo double-stranded RNA was administered at a dose of 1 mg/kg body weight; the gray line, a group to which B043 of oligo double-stranded RNA was administered at a dose of 3 mg/kg bodyweight; and the bold line, a group to which B043 of oligo double-stranded RNAwas administered at a dose of 10 mg/kg body weight.

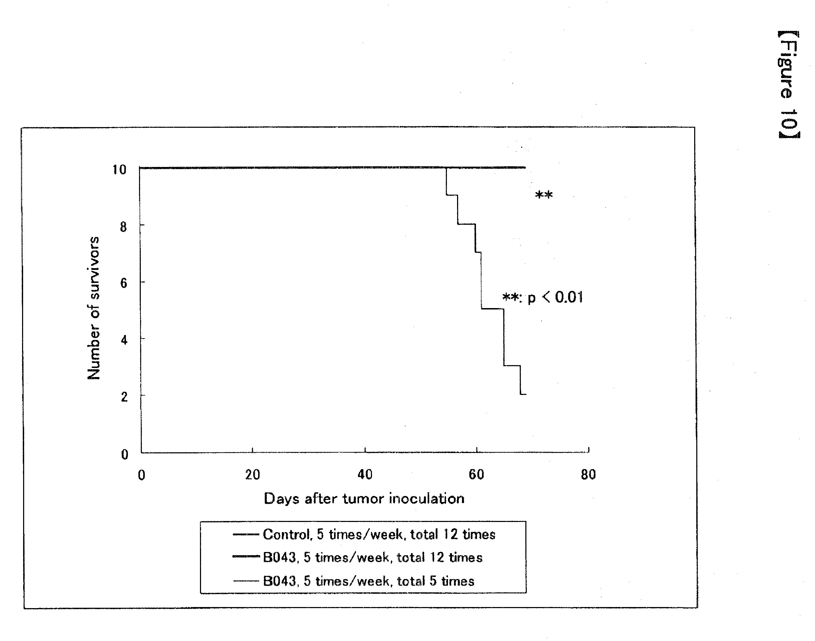

Fig. 10 shows the survival number of mice up to 69 days after inoculation of A549 cells. The fine line indicates a control group; the bold line, a group to which B043 of oligo double-stranded RNA was administered 5 times a week, a total of 12 times; and the dotted line, a group to which B043 of oligo double-stranded RNA was administered 5 times a week, a total of 5 times. In this figure, the dotted line is overlapping with the bold line.

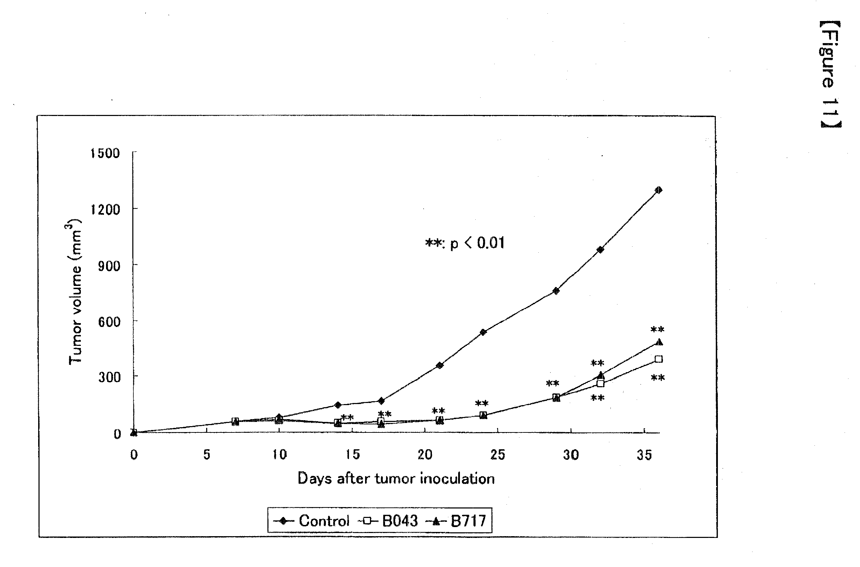

Fig. 11 shows the tumor volume of mice up to 36 days after inoculation of PC-3 cells. The symbol ◆ , indicates a control group □, a group to which B043 of oligo double-stranded RNA was administered; and ▲, a group to which B717 of oligo double-stranded RNA was administered.

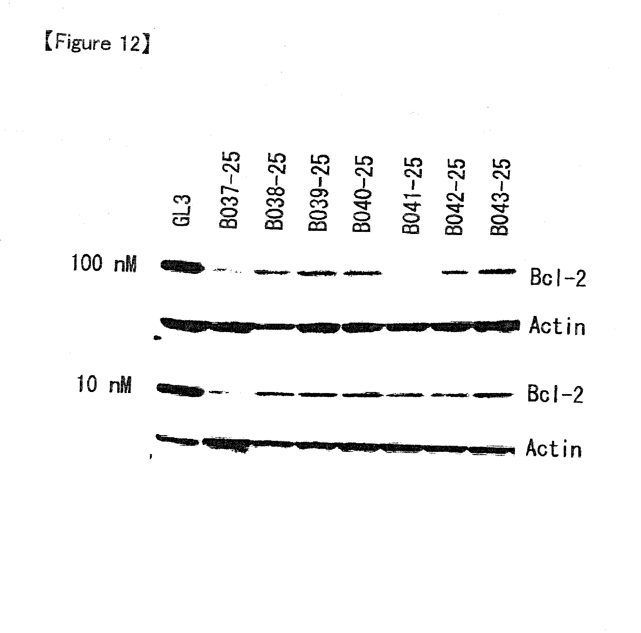

Fig.12 shows the result of an evaluation performed by Western blotting of the inhibitory activity of oligo double-stranded RNAs on the expression of Bcl-2 protein. The upper case indicates the result wherein A431 cells were transfected with the respective RNAs at a final concentration of 100 nM, and the lower case, at a final concentration of 10 nM.

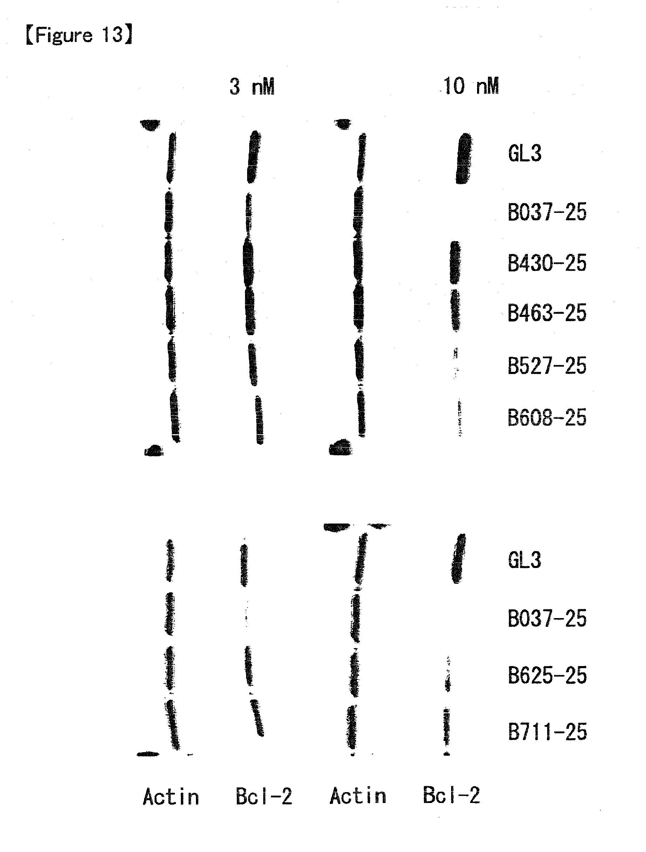

Fig.13 shows the result of an evaluation performed by Western blotting ofthe inhibitory activity of oligo double-stranded RNAs on the expression of Bcl-2 protein. The upper case indicates the result wherein A431 cells were transfected with the respective RNAs at a final concentration of 10 nM, and the lower case, at a final concentration of 3 nM.

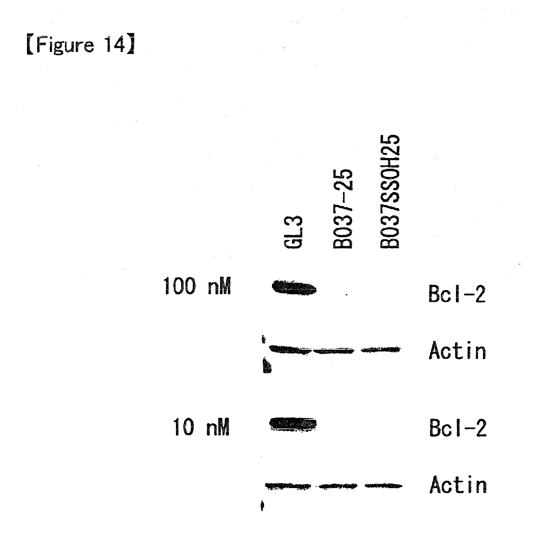

Fig.14 shows the result of an evaluation performed by Western blotting ofthe inhibitory activity of oligo double-stranded RNAs on the expression of Bcl-2 protein. The upper case indicates the result wherein A431 cells were transfected with the respective RNAs at a final concentration of 100 nM. and the lower case, at a final concentration of 10 nM.

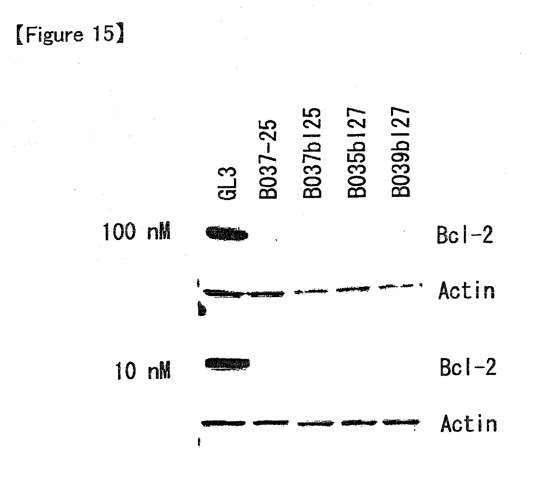

Fig. 15 shows the result of an evaluation performed by Western blotting of the inhibitory activity of oligo double-stranded RNAs on the expression of Bcl-2 protein. The upper case indicates the result wherein A431 cells were transfected with the respective RNAs at a final concentration of 100 nM, and the lower case, at a final concentration of 10 nM.

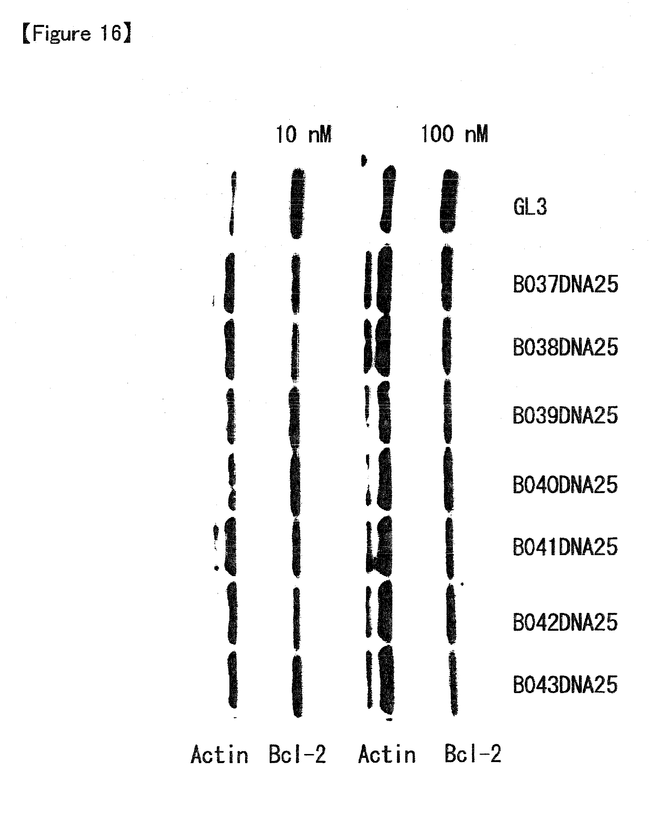

Fig.16 shows the result of an evaluation performed by Western blotting of the inhibitory activity of oligo double-stranded RNAs on the expression of Bcl-2 protein. The upper case indicates the result wherein A431 cells were transfected with the respective RNAs at a final concentration of 100 nM, and the lower case, at a final concentration of 10 nM.

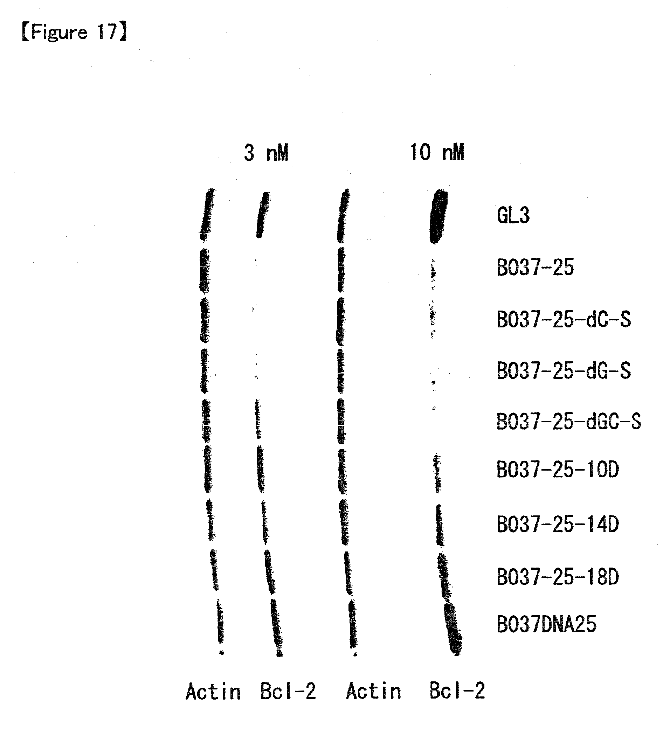

Fig. 17 shows the result of an evaluation performed by Western blotting ofthe inhibitory activity of oligo double-stranded RNAs on the expression of Bcl-2 protein. The upper case indicates the result wherein A431 cells were transfected with the respective RNAs at a final concentration of 10 nM, and the lower case, at a final concentration of 3 nM.

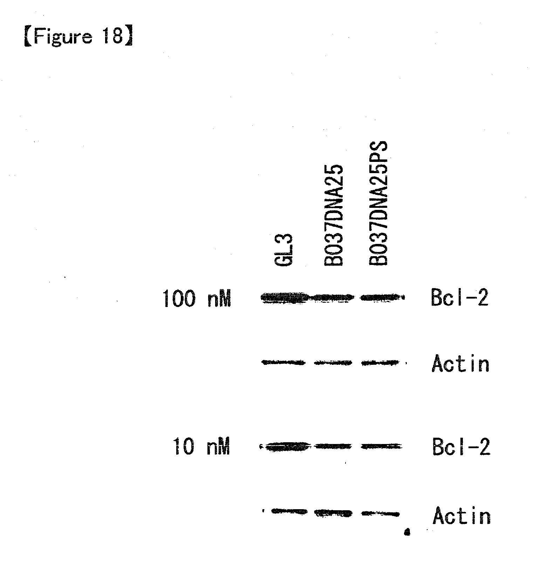

Fig.18 shows the result of an evaluation performed by Western blotting ofthe inhibitory activity of oligo double-stranded RNAs on the expression of Bcl-2 protein. The upper case indicates the result wherein A431 cells were transfected with the respective RNAs at a final concentration of 100 nM, and the lower case, at a final concentration of 10 nM.

### Best Mode for Carrying Out the Invention

### Oligo double-stranded RNAs inhibiting the expression of Bcl-2

The present inventors investigated the sequences registered as bcl-2 mRNA to a gene database for comparison and confirmed that the full length mRNA sequence of bcl-2 registered as GenBank Accession No. BC027258 was the consensus. Based on the sequence (SEQ ID NO:197) of GenBank Accession No. BC027258, primary, secondary and tertiary screenings were conducted to obtain an oligo double-stranded RNA having an inhibitory activity on the expression of Bcl-2 protein.

The oligo double-stranded RNA in the description is defined as a pair of RNAs of 15 to 31 nucleotides (which may contain partially deoxylribonucleotide(s) or modified nucleotide(s); hereinafter, the same in this paragraph) which have a double-strand forming portion of 15 to 27 base pairs, the pair of RNAs being introduced in a cell and cleaving a mRNA complementary to the oligo double-stranded RNA to inhibit synthesis of the gene product coded by the mRNA.

The oligo double-stranded RNA in the description is not limited to siRNAs described in the Tuschl's report. For example, the oligo double-stranded RNA of the present invention does not necessarily have the overhang at the 3'-terminal, and the number of base in the respective counterpart strand is not limited to 19 to 25 bases. The oligo double-stranded RNA of the present invention is a pair of 15 to 31 nucleotides, preferably a pair of 17 to 23 nucleotide, as well as a pair of 25 to 27 nucleotides.

The double-strand forming portion in the description means the portion in the oligo double-stranded RNA in which a pair of nucleic acids constituting the oligo double-stranded RNA forms a double strand, and which contains a sense strand and an anti-sense strand corresponding to bcl-2 mRNA. In this connection, when there is a single-strand at the 3'- or 5'-terminal side following the double-strand forming portion in the respective RNA strands, this is designated as an overhang.

Details of screening will be shown in Examples. Briefly, the primary screening was carried out on a pool composed of oligo double-stranded RNAs, each comprising a double-strand forming portion which is formed by pairing a sense RNA strand of 19 bases beginning from every 9 bases, in principle, in the ORF (open reading frame) of bcl-2 mRNA and its complementary RNA strand. The secondary screening was carried out on oligo double-stranded RNAs, each having as a sense sequence a sequence which shifts by every 3 bases (either upstream or downstream) from the sequence of bcl-2 mRNA corresponding to the oligo double-stranded RNA in which a high inhibitory activity on the expression of Bcl-2 protein was observed in the primary screening. The tertiary screening was carried out on oligo double-stranded RNAs, each having as a sense sequence a sequence which shifts by every 1 base from the sequence of bcl-2 mRNA corresponding to the oligo double-stranded RNA in which a high inhibitory activity on the expression of Bcl-2 protein was observed in the secondary screening. In addition, screening was also performed to evaluate an inhibitory activity on the expression of Bcl-2 of the sequences of oligo double-stranded RNAs which have various overhangs different in length and those which have a small number of bases.

As shown in detail in Examples mentioned below, the inhibitory activity of oligo double-stranded RNAs having the selected sequence on the expression of Bcl-2 protein was evaluated by introducing the oligo double-stranded RNA into culture cancer cells using a cationic liposome, etc" culturing it for a given period of time, and determining the amount and the decrease of expressed Bcl-2 protein in the cancer cells by Western blotting. In addition, the amount of bcl-2 mRNA was semi-quantified by RT-PCR and/or the inhibitory effect on cell growth was evaluated by a cell-counting assay,

On the basis of the above screening results, the present inventors found that the use of some oligo double-stranded RNAs could greatly decrease the expression of Bcl-2 protein, wherein the oligo double-stranded RNAs are a pair of a nucleic acid of a sequence selected from SEQ ID NO. 1 to SEQ ID NO:81, SEQ ID NO:240 to SEQ ID NO: 256, and SEQ ID NO:274 to SEQ ID NO:280, and a complementary nucleic acid of a sequence selected from SEQ ID NO:82 to SEQ ID NO:162, SEQ ID NO:257 to SEQ ID NO:273, and SEQ ID NO:281 to SEQ ID NO:287. in addition, it was found that the oligo double-stranded RNAs having the above-mentioned sequence, regardless of the presence or absence of the overhang and even if the double-strand forming portion is short, can inhibit the expression of Bcl-2 protein.

It has been elucidated by the present inventors that the sequence of the overhang at the 3'-terminal has no effect on the activity. Therefore, the present invention includes an oligo double-stranded RNA capable of inhibiting the expression of Bcl-2 protein, which comprises a double-strand forming portion of 15 to 19 base pairs which is a pair of a sense RNA strand of one sequence selected from SEQ ID NO:1 to SEQ ID NO:81, SEQ ID NO:240 to SEQ !D NO:256, and SEQ ID NO:274 to SEQ ID NO:280 from which 2 dT bases at the 3'-terminal are excluded, and a complementary anti-sense RNA strand of one sequence selected from SEQ ID NO:82 to SEQ ID NO:162, SEQ ID NO:257 to SEQ ID NO:273, and SEQ ID NO:281 to SEQ ID NO:287 from which 2 dT bases at the 3'-terminal are excluded, of which pair a total of up to 4 base pairs may further be excluded from either one or both terminals.

In one mode of the present invention, the oligo double-stranded RNA of the present invention may contain one or more bases of deletion, substitution, insertion or addition in a part of the bases in either or both sequences of an RNA pair of double-strand forming portion. In another mode of the present invention, the oligo double-stranded RNA of the present invention may be substituted by a deoxyribonucleotide(s) ora modified nucleotide(s) in a part(s) of ribonucleotides from either or both strands of an RNA pair of double-strand forming portion or in the whole sense RNA strand.

In a desirable mode of the present invention, a pair of a sense RNA strand with an anti-sense RNA strand includes an oligo double-stranded RNA comprising a double-strand forming portion which is a pair of the sequences represented by SEQ ID NO:11 and SEQ ID NO:92, SEQ ID NO:30 and SEQ ID NO:111, SEQ ID NO:36 and SEQ ID NO:117, SEQ ID NO:43 and SEQ ID NO:124, SEQ ID NO:55 and SEQ ID NO:136, SEQ ID NO:62 and SEQ ID NO: 143, or SEQ ID NO:77 and SEQ ID NO:158 from which 2 dT bases at the respective 3'-terminals are excluded.

The oligo double-stranded RNA of the present invention may or may not contain as an overhang a 1-base to 4-base nucleotide at the 3'-terminal or 5'-terminal of at least one strand of the RNAs. In particular, the 7 oligo double-stranded RNAs represented by the pairs according to the above-mentioned sequence identification numbers have a strong inhibitory activity on the expression of Bcl-2 protein even though no overhang exists.

When an overhang exists, the nucleotide constituting the overhang may be either ribonucleotide or deoxyribonucleotide. A particularly desirable mode is an oligo double-stranded RNA having 2 bases of nucleotide as an overhang. A preferable overhang is dTdT, UU, a sequence identical with a part of bcl-2 mRNA following the double-strand forming portion, or a sequence complementary to a part of bcl-2 mRNA following the double-strand forming portion.

In order to enhance in viva stability such as nuclease resistance, the oligo double-stranded RNA of the present invention may be modified at least partially in the riboses or a phosphate backbone constituting the nucleotide, Preferred modification, if modified, includes modification in 2'-position of sugar, modification of other parts of sugar, modification of phosphate backbone in the oligo double-stranded RNA, and so on. The modification in 2'-position of sugar includes substitution of 2'-hydroxyl group of ribose with H, OR, R, R', OR, SH, SR, NH₂, NHR, NR₂, N₃, CN, F, Cl, Br, I, etc. Wherein, "R" represents alkyl or aryl, preferably alkyl group of 1 to 6 carbons, and "R'" represents alkylene, preferably alkylene of 1 to 6 carbons. The modified derivative of other parts of sugar includes 4' thio derivatives. The modified derivative of phosphate backbone includes phosphorothioate, phosphorodithioate, alkyl phosphonate, phosphoroamidate, and the like.

The particularly desirable oligo double-stranded RNA of the present invention includes B043, B436, B469, B533, B614, B631 and B717; the followings show their sequences.
B043:
   sense strand
      5'-GUGAUGAAGUACAUCCAUU-dTdT-3' (SEQ ID NO:11)
   anti-sense strand
      5'-AAUGGAUGUACUUCAUCAC-dTdT-3' (SEQ ID NO:92)
B436:
   sense strand
      5'-AGGAUUGUGGCCUUCUUUG-dTdT-3' (SEQ ID NO:30)
   anti-sense strand
      5'-CAAAGAAGGCCACAAUCCU-dTdT-3'' (SEQ ID NO:111)
B469:
   sense strand
      5'-AUGUGUGUGGAGAGCGUCA-dTdT-3' (SEQ ID NO:36)
   anti-sense strand
      5'-UGACGCUCUCCACACACAU-dTdT-3' (SEQ ID NO:117)
B533:
   sense strand
      5'-CUGAGUACCUGAACCGGCA-dTdT-3' (SEQ ID NO:43)
   anti-sense strand
      5`-UGCCGGUUCAGGUACUCAG-dTdT-3° (SEQ ID NO:124)
B614:
   sense strand
      5'-GCAUGCGGCCUCUGUUUGA-dTdT-3' (SEQ ID NO:55)
   anti-sense strand
      5'-UCAAACAGAGGCCGCAUGC-dTdT-3' (SEQ ID NO:136)
B631:
   sense strand
      5'-GAUUUCUCCUGGCUGUCUC-dTdT-3' (SEQ ID NO:62)
   anti-sense strand
      5'-GAGACAGCCAGGAGAAAUC-dTdT-3' (SEQ I D NO:143)
B717:
   sense strand
      5'-GUGAAGUCAACAUGCCUGC-dTdT-3' (SEQ ID NO:77)
   anti-sense strand
      5'-GCAGGCAUGUUGACUUCAC-dTdT-3' (SEQ ID NO:158)

The oligo double-stranded RNA of the present invention, when introduced into a cell, can inhibit the expression of Bcl-2 protein in comparison with the case in which the oligo double-stranded RNA is not present. In inhibiting the expression of Bcl-2 protein using an anti-sense DNA. several hundred nM to several ten µM of the anti-sense DNA is generally required, while the oligo double-stranded RNA of the present invention can inhibit the expression of Bcl-2 protein even at a concentration of several nM to several hundred nM through transfection to a cell and subsequent incubation for 24 hours or more, for example, 72 hours, in comparison with no oligo double-stranded RNA. Preferably, A431 cell (epithelial cancer cell) is transfected with the oligo double-stranded RNA at a concentration of 3-10 nM, followed by incubation for 72 hours, at which stage the expression of Bcl-2 protein is inhibited in comparison with no oligo double-stranded RNA.

A single-stranded nucleic acid of the sense strand alone or the anti-sense strand alone constituting the oligo double-stranded RNA of the present invention also falls within the scope of the present invention. That is, the nucleic acid comprises an RNA of one sequence selected from SEQ ID NO:1 to SEQ ID NO:162 from which 2 dT bases at the 3'-terminal are excluded, as a double-strand forming portion.

In one mode of the present invention, the nucleic acid of the present invention may have one or more bases of deletion, substitution, insertion or addition in a part of the bases in the RNA sequence.

The nucleic acid of the present invention may or may not contain as an overhang 1-base to 4-base nucleotide at the 3'-terminal. The nucleotide of the overhang may be either ribonucleotide or deoxyribonucleotide. A particularly desirable mode is a nucleic acid having 2 nucleotide bases as an overhang. A preferable overhang is dTdT, UU, a sequence identical with a part of bcl-2 mRNAfollowing the double-strand forming portion, or a sequence complementary to a part of bcl-2 mRNA following the double-strand forming portion.

in addition, the nucleic acid of the present invention includes a nucleic acid which has one sequence selected from SEX ID NO:163 to SEQ in NO:196 and SEQ in NO:198 to SEQ ID NO:239.

In another mode of the present invention, the nucleic acid of the present invention may be substituted by a eoxyribonucleotide(s) or a modified nucleotide(s) in a part of ribonucleotides of the RNA sequence of the double-strand forming portion.

A nucleic acid which has one sequence selected from SEA ID NO:1 to SENT ID NO:162 and SET ID NO:240 to SEQ ID NO:287 from which 2 dT bases at the 3'-terminal are excluded, and in which uridine (U) is thymine (T), as a deoxyribonucleotide, falls within the scope of the present invention. The nucleic acid may be incorporated into a plasmid to generate an oligo double-stranded RNA of the present invention, or may be used as template DNAs to obtain an oligo double-stranded RNA of the present invention by in vitro transcription reaction. Alternatively, this may be used as an anti-sense probe. In addition, the above-mentioned nucleic acid may be that characterized in that it contains bases having one or more bases of deletion, substitution, insertion or addition in its sequence, and it has an inhibitory activity on the expression of Bcl-2 protein when it works as a template for transcription to generate RNA which constitutes an oligo double-stranded RNA.

Further, the present invention provides a method for screening an oligo double-stranded RNA, which has an inhibitory activity on the expression of Bcl-2 protein, from oligo double-stranded RNAs having double-strand forming portions of 25 to 27 base pairs including 19 base pairs according to the particularly preferred oligo double-stranded RNAs in the present invention: B043 (SEQ ID NO:11 and SEQ ID NO:92), B436 (SEQ ID NO:30 and SEQ ID NO:111), B469 (SEQ ID NO:36 and SEQ ID NO:117), B533 (SEQ ID NO:43 and SEQ ID NO:124), B614 (SEQ ID NO:55 and SEQ ID NO:136), B631 (SEQ ID NO:62 and SEQ ID NO:143), and B717 (SEQ ID NO:77 and SEQ ID NO:158).

In addition, the present invention provides oligo double-stranded RNAs, which have an inhibitory activity on the expression of Bcl-2 protein, comprising a double-strand forming portion of 25 to 27 base pairs including 19 base pairs of an oligo double-stranded RNA selected from the group consisting of B043, B436, B469, B533, B614, B631, and B717.

As a result of measurement of the inhibitory activity on the expression of Bcl-2 protein, the present inventors found that the expression of Bcl-2 protein greatly decreased when an oligo double-stranded RNA comprising a double-strand forming portion which is a pair of a nucleic acid corresponding to a part of bcl-2 mRNA which has one sequence selected from SEQ ID NO:288 and SEQ ID NO:295 to SEQ ID NO:300 from which 2 dT bases at the 3'-terminal and 6 bases at the 5'-terminal are excluded and to which a total of 6 bases are added at the 3'-terminal and/or 5'-terminal: and a nucleic acid complementary to the part of bcl-2 mRNA which has one sequence selected from SEQ ID NO:319 and SEQ ID NO:326 to SEQ ID NO:331 from which 2 dT bases and the subsequent 6 bases at the 3'-terminal are excluded and to which a total of 6 bases are added at the 3'-terminal and/or 5'-terminal.

In a desirable mode of the present invention, a pair of a sense RNA strand and an anti-sense RNA strand includes an oligo double-stranded RNA of 25 base pairs comprising a double-strand forming portion which is a pair of sequences represented by SEQ ID NO:288 and SEQ ID NO:319, SEQ ID NO:289 and SEQ ID NO:320, SEQ ID NO: 290 and SEQ ID NO:321, SEQ ID NO:291 and SEQ ID NO:322, SEQ ID NO:292 and SEQ ID NO:323, SEQ ID NO:293 and SEQ ID NO:324. SEQ ID NO:294 and SEQ ID NO:325, SEQ ID NO:295 and SEQ ID NO:326, SEQ ID NO:296 and SEQ ID NO:327, SEQ ID NO:297 and SEQ ID NO:328, SEQ ID NO:298 and SEQ ID NO:329, SEQ ID NO:299 and SEQ ID NO:330, or SEQ ID NO:300 and SEQ ID NO:331 from which 2 dT bases at the respective 3'-terminals are excluded.

When an overhang exists, the nucleotide constituting the overhang may be either ribonucleotide or deoxyribonucleotide. A particularly desirable mode is an oligo double-stranded RNA having 2 nucleotide bases as an overhang. A more preferred mode is an oligo double-stranded RNA having dTdT, UU or a sequence identical with a part of bcl-2 mRNA following the double-strand forming portion as an overhang at the 3'-terminal of a sense RNA strand, and having dTdT, UU or a sequence complementary to a part of bcl-2 mRNA following the double-strand forming portion as an overhang at the 3'-terminal of an anti-sense RNA strand.

In addition, as one mode of the present invention, the present inventors found that the expression of Bcl-2 protein greatly decreased when an oligo double-stranded RNA of 27 base pairs which is a pair of a nucleic acid corresponding to a part of bcl-2 mRNA which has one sequence selected from SEQ ID NO:288 to SEO ID NO:300 from which 2 dT bases at the 3'-terminal are excluded and to which a total of 2 bases are added at the 3'-terminal and/or 5'-terminal; and a nucleic acid complementary to the part of bcl-2 mRNA which has one sequence selected from SEQ ID NO:319 to SEQ ID NO:331 from which 2 dT bases at the 3'-terminal are excluded and to which a total of 2 bases are added at the 3'-terminal and/or 5'-terminal.

In a desirable mode of the present invention, a pair of a sense RNA strand and an anti-sense RNA strand includes an oligo double-stranded RNA of 27 base pairs of sequences represented by SEQ ID NO:303 and SEQ ID NO: 332, or SEQ ID NO:304 and SEQ ID NO:334.

In one mode of the present invention, the oligo double-stranded RNA of the present invention may contain one or more bases of deletion, substitution, insertion or addition in a part of bases in either or both sequences in a RNA pair of a double-strand forming portion.

Further, in another embodiment of the present invention, the oligo double-stranded RNA of the present invention may be substituted by a deoxyribonucleotide(s) or a modified nucleotide(s) in a part(s) of one or both of the ribonucleotides from either or both strands of an RNA pair of a double-strand forming portion or in the whole sense RNA strand.

In a desirable mode of the present invention, a pair of a sense RNA strand and an anti-sense RNA strand includes an oligo double-stranded RNA comprising a double-strand forming portion which is a pair of sequences represented by SEQ ID NO:305 and SEQ ID NO:319, SEQ ID NO:306 and SEQ ID NO:319, SEQ ID NO:307 and SEQ ID NO:319, SEQ ID NO:308 and SEQ ID NO:319, SEQ ID NO:309 and SEQ ID NO:319, SEQ ID NO:310 and SEQ ID NO: 319, SEQ ID NO:311 and SEQ ID NO:319, SEQ ID NO:312 and SEQ ID NO:320, SEQ ID NO:313 and SEQ ID NO:321, SEQ ID NO:314 and SET ID NO:322, SEQ ID NO:31 and SEQ ID NO:323, SEQ ID NO:316 and SEQ ID NO:324, or SEQ ID NO:317 and SEQ ID NO:325 from which 2 dT bases at the respective 3'-terminals are excluded.

In order to enhance in vivo stability such as nuclease resistance, the oligo double-stranded RNA of the present invention may be modified at least partially in the ribose or phosphate backbone constituting the nucleotide. Preferred modification, if modified, includes modification in 2'-position of sugar, modification of other parts of sugar, modification of phosphate backbone in the oligo double-stranded RNA, and so on. The modification in 2'-position of sugar includes substitution of 2'-hydroxyl group of ribose with H, OR, R, R', OR, SH, SR, NH₂, NHR, NR₂, N₃, CN, F, Cl, Br, I, etc. Wherein, "R" represents alkyl or aryl, preferably alkyl group of 1 to 6 carbons, and "R' represents alkylene, preferably alkylene of 1 to 6 carbons. The modified derivative of other parts of sugar includes 4' thio derivatives. The modified derivative of phosphate backbone includes phosphorothioate, phosphorodithioate, alkyl phosphonate, phosphoroamidate, and the like.

The particularly desirable oligo double-stranded RNA of the present invention includes B037-25, B038-25, B039-25, B040-25, B041-25, B042-25, B043-25, B430-25, B463-25, B527-25, B608-25, B625-25 and B711-25; the followings show their sequences.
B037-25:
   sense strand
      5'-GAGAUAGUGAUGA-AGUACAUCCAUU-dTdT-3' (SEQ ID NO:288)
   anti-sense strand
      5'-AAUGGAUGUACUUCAUCACUAUCUC-dTdT-3' (SEQ ID NO:319)
B038-25:
   sense strand
      5'-AGAUAGUGAUGAAGUACAUCCAUUA-dTdT-3' (SEQ ID NO:289)
   anti-sense strand
      5'-UAAUGGAUGUACUUCAUCACUAUCU-dTdT-3' (SEQ ID NO:320)
B039-25:
   sense strand
      5'-GAUAGUGAUGAAGUACAUCCAUUAU-dTdT-3' (SEQ ID NO:290)
   anti-sense strand
      5'-AUAAUGGAUGUACUUCAUCACUAUC-dTdT-3' (SEQ ID NO:321)
B040-25:
   sense strand
      5'-AUAGUGAUGAAGUACAUCCAUUAUA-dTdT-3' (SEQ ID NO:291)
   anti-sense strand
      5'-UAUAAUGGAUGUACUUCAUCACUAU-dTdT-3' (SEQ ID NO:322)
B041-25:
   sense strand
      5'-UAGUGAUGAAGUACAUCCAUUAUAA-dTdT-3' (SEQ ID NO:292)
   anti-sense strand
      5'-UUAUAAUGGAUGUACUUCAUCACUA-dTdT-3' (SEQ ID NO:323)
B042-25:
   sense strand
      5'-AGUGAUGAAGUACAUCCAUUAUAAG-dTdT-3' (SEQ ID NO:293)
   anti-sense strand
      5'-CUUAUAAUGGAUGUACUUCAUCACU-dTdT-3' (SEQ ID NO:324)
B043-25:
   sense strand
      5'-GUGAUGAAGUACAUCCAUUAUAAGC-dTdT-3' (SEQ ID NO:294)
   anti-sense strand
      5'-GCUUAUAAUGGAUGUACUUCAUCAC-dTdT-3' (SEQ ID NO:325)
B430-25:
   sense strand
      5'-UGGGGGAGGAUUGUGGCCUUCUUUG-dTdT-3' (SEQ ID NO:295)
   anti-sense strand
      5'-CAAAGAAGGCCACAAUCCUCCCCCA-dTdT-3' (SEQ ID NO:326)
B463-25:
   sense strand
      5'-GGGGOCAOGUGOGUGGAGAGCGUCA-dTdT-3' (SEQ ID NO:296)
   anti-sense strand
      5'-UGACGCUCUCCACACACAUGACCCC-dTdT-3' (SEQ ID NO:327)
B527-25:
   sense strand
      5'-GGAUGACUGAGUACCUGAACCGGCA-dTdT-3' (SEQ ID NO:297)
   anti-sense strand
      5'-UGCCGGUUCAGGUACUCAGUCAUCC-dTdT-3' (SEQ ID NO:328)
B608-25:
   sense strand
      5'-GCCCCAGCAUGCGGCCUCUGUUUGA-dTdT-3' (SEQ ID NO:298)
   anti-sense strand
      5'-UCAAACAGAGGCCGCAUGCUGGGGC-dTdT-3' (SEQ ID NO:329)
B625-25:
   sense strand
      5'-CUGUUUGAUUUCUCCUGGCUGUCUC-dTdT-3' (SEQ ID NO:299)
   anti-sense strand
      5'-GAGACAGCCAGGAGAAAUCAAACAG-dTdT-3' (SEQ ID NO:330)
B711-25:
   sense strand
      5'-CCACAAGUGAAGUCAACAUGCCUGC-dTdT-3' (SEQ ID NO:300)
   anti-sense strand
      5'-GCAGGCAUGUUGACUUCACUUGUGG-dTdT-3' (SEQ ID NO:331)

The oligo double-stranded RNA of the present invention, when introduced into a cell, can inhibit the expression of Bcl-2 protein in comparison with the case in which the oligo double-stranded RNA is not present. In inhibiting the expression of Bcl-2 protein using an anti-sense DNA, several hundred nM to several ten µM of the anti-sense DNA is generally required, while the oligo double-stranded RNA of the present invention can inhibit the expression of Bcl-2 protein even at a concentration of several nM to several hundred nM through transfection of a cell and subsequent incubation for 24 hours or more, for example, 72 hours, in comparison with no oligo double-stranded RNA. Preferably A431 cell (epithelial cancer cell) is transfected with the oligo double-stranded RNA at a concentration of 3-10 nM, followed by incubation for 72 hours, at which stage the expression of Bcl-2 protein is inhibited in comparison with no oligo double-stranded RNA.

A single-stranded nucleic acid of the sense strand alone or the anti-sense strand alone constituting the nucleic acids of the present invention, falls within the scope of the present invention. That is, the nucleic acid includes a nucleic acid corresponding to a part of bcl-2 mRNA which has one sequence selected from SEQ ID NO:288 and SEQ ID NO: 295 to SEQ ID NO:300 from which 2 dT bases at the 3'-terminal and 6 bases at the 5'-terminal are excluded and to which a total of 6 bases are added at the 3'-terminal and/or 5'-terminal; or a nuclei acid complementary to the part of bcl-2 mRNA which has one sequence selected from SEQ ID NO:319 and SEQ ID NO:326 to SEQ ID NO:331 from which 2 dT bases and the subsequent 6 bases at the 3'-terminal are excluded and to which a total of 6 bases are added at the 3'-terminal and/or 5'-terminal.

In one mode of the present invention, the nucleic acid of the present invention may contain one or more bases of deletion, substitution, insertion or addition in a part of the bases in the RNA sequence.

In the nucleic acids of the present invention, the nucleotide constituting the overhang may be either ribonucleotide or deoxyribonucleotide. A particularly desirable mode is a nucleic acid having 2 nucleotide bases as an overhang. A preferable overhang is dTdT, UU, a sequence identical with a part of bcl-2 mRNA following a double-strand forming portion, or a sequence complementary to a part of bcl-2 mRNA following a double-strand forming portion.

Preferably, the nucleic acid of the present invention includes a nucleic acid of a sequence selected from SEQ ID NO:288 to SEQ ID NO:300 and SEQ ID NO:319 to SEQ ID NO:331.

In addition, the nucleic acid of the present invention include a nucleic acid corresponding to a part of bcl-2 mRNAwhich has one sequence selected from SEQ ID NO:288 to SEQ ID NO:300 from which 2 dT bases at the 3'-terminal are excluded and to which a total of 2 bases are added at the 3'-terminal and/or 5'-terminal; or a nucleic acid complementary to the part of bcl-2 mRNA which has one sequence selected from SEQ ID NO:319 to SEQ ID NO:331 from which 2 dT bases at the 3'-terminal are excluded and to which a total of 2 bases are added to the 3'-terminal and/or 5'-terminal.

Preferably, the nucleic acid of the present invention includes a nucleic acid of one sequence selected from SEQ ID NO:303 to SEQ ID NO:304 and SEQ ID NO:332 to SEQ ID NO:334.

In another mode of the present invention, a nucleic acid which has one sequence selected from SEQ ID NO: 288 to SEQ ID NO:300 and SEQ ID NO:319 to SEQ ID NO:331 from which 2 dT bases at the 3'-terminal are excluded and in which a part of the ribonucleotides is replaced with a deoxyribonucleotide(s) or a modified nucleotide(s), falls within the scope of the present invention.

In addition, a nucleic acid which has one sequence selected from SEQ ID NO:288 to SEQ ID NO:300 and SEQ ID NO:319 to SEQ ID NO:331 from which 2 dT bases at the 3'-terminal are excluded and in which uridine (U) thymine (T), as a deoxyribonucleotide, falls within the scope of the present invention. The nucleic acids may be incorporated into a plasmid to generate an oligo double-stranded RNA of the present invention, or may be used as a template DNA to obtain an oligo double-stranded RNA of the present invention by in vitro transcription reaction. Alternatively, it may be used as an anti-sense probe. In addition, the above-mentioned nucleic acid may be those characterized in that it contains one or more bases of deletion, substitution, insertion or addition in its sequence, and it has an inhibitory activity on the expression of Bcl-2 protein when it is used as a template for transcription to generate RNA which constitutes an oligo double-stranded RNA.

RNAs and DNAs of the present invention can be synthesized in a solid or liquid phase by a phosphoramidite method or triester method as well known by persons skilled in the art. In the most typical mode, a solid phase synthetic method by a phosphoramidite method is employed using an automatic synthesizer for nucleic acid or in manual. After termination of the synthesis on a solid phase, the objective product is released from the solid phase, and after removing a protecting group by deprotection, purified. In purification, it is desirable to obtain the nucleic acid in 90% or more purity, preferably 95% or more. When used as an oligo double-stranded RNA, a synthesized and purified sense strand may be mixed and annealed with an anti-sense strand in a molar ratio of 0 to 10 equivalents for 1 equivalent of the anti-sense strand, preferably in 0.5 to 2 equivalents, more preferably in 0.9 to 1.1 equivalents, and most preferably in an equimolar ratio, or alternatively, mixed strands may be used directly without a step of annealing. Annealing typically may be carried out by mixing an approximately equimolar amount of sense and anti-sense strands, heating the mixture at about 94°C for about 5 minutes, and the slowly cooling it down to room temperature, though another condition well-known by persons skilled in the art may be employed.

The oligo double-stranded RNAs of the present invention may be transfected into a cell using a carrier for transfection such as cationic liposome and other vectors. In another embodiment of the present invention, the RNAs may be introduced directly into a cell by a calcium phosphate method, electroporation or microinjeciton, or the like.

### Pharmaceutical Compositions

The invention provides a pharmaceutical composition comprising a complex of an oligo double-stranded RNA of the present invention and a carrier which is effective in introducing the oligo double-stranded RNA into a cell. The pharmaceutical composition of the present invention can be used in treatment and/or prevention of diseases caused by over-expression of Bcl-2 protein, diseases for which acceleration of apoptosis is desired, or hematological malignant diseases. Specifically, these diseases are hematological malignant diseases including both of lymphoma and leukemia, and solid tumors, for example, liver cancer, skin cancer, breast cancer, lung cancer, cancers of digestive organs, prostate cancer, uterus cancer, bladder cancer, or the like.

As for the carrier for forming a complex with an oligo double-stranded RNA, cationic carriers such as a cationic liposome and a cationic polymer, or a carrier utilizing virus envelope, which are effective in introducing an oligo double-stranded RNA into a cell, may be used. The preferred cationic liposome includes a liposome comprising 2-O-(2-diethylaminoethyl)carbamoyl-1,3-O-dioleoylglycerol (hereinafter, referred to as "liposome A"), Oligofectamine (Invitrogen), Lipofectin (Invitrogen), Lipofectamine (Invitrogen), Lipofectamine 2000 (Invitrogen), DMRIE-C (Invitrogen), Gene Silencer (Gene Therapy Systems), Trans Messenger (QIAGEN), Trans IT TKO (Mirus), or the like. The preferred cationic polymer includes Jet SI (Qbiogene), Jet-PEI (polyethylenimine; Qbiogene), or the like. The preferred carrier utilizing virus envelope is Genome One (HVJ-E liposome; Ishihara Sangyo) or the like.

The complex of an oligo double-stranded RNA and a carrier contained in the pharmaceutical composition of the present invention can be prepared according to a method well-known by persons skilled in the art. Briefly, the complex can be prepared by mixing a carrier-dispersed solution with an oligo double-stranded RNA-containing solution in a suitable concentration. When a cationic carrier is used, the complex can be readily formed by mixing an oligo double-stranded RNA, which is charged negatively in an aqueous solution with the carrier in an aqueous solution in a conventional way. The aqueous medium used in formation of the complex includes water for injection, distilled water for injection, electrolyte such as saline, sugar solution such as glucose solution, maltose solution. In addition, such mixing conditions as pH and temperature can be determined by persons skilled in the art. For example, in the case of liposome A, the complex can be prepared by slowly adding an oligo double-stranded RNA in 10% maltose solution into a liposome A-dispersed solution of 16 mg/ml in 10% maltose solution at pH 7.4 and 25°C with stirring.

The complex, if required, may be treated with a supersonic dispersing apparatus, high pressure-emulsifying apparatus or the like to give a homogeneous composition. Persons skilled in the art will be able to choose an optimal method and condition for preparing a complex of a carrier with an oligo double-stranded RNA according to the carrier used, without being bound by the above-mentioned method.

As for the formulating ratio of an oligo double-stranded RNA to a carrier in the complex contained in the pharmaceutical composition of the present invention, the carrier can be used in an amount of 1 to 200 parts by weight for 1 part by weight of oligo double-stranded RNA. Preferably, the carrier is used in 2.5 to 100 parts by weight, more preferably 10 to 20 parts by weight, for 1 part by weight of oligo double-stranded RNA.

The pharmaceutical composition of the present invention may contain a pharmaceutically acceptable carrier or a diluent in addition to the complex of oligo double-stranded RNA and carrier. The pharmaceutically acceptable carrier or diluent is essentially chemically inactive and innocuous compositions and give totally no effect on a biological activity of the pharmaceutical composition of the present invention. Such carrier or diluent includes, but not limited to, salt solution, sugar solution, glycerol solution, ethanol, and the like.

The pharmaceutical composition of the present invention contains an amount of complex effective in treating and/or preventing and is provided in a formulation properly applicable to a subject. The formulation of the pharmaceutical composition of the present invention includes, for example, liquid preparations such as injections, infusions, etc., external preparations such as ointment, lotion, and lyophilized preparations.

The liquid preparations suitably contain the complex in the range of 0.001 to 25% (w/v) in concentration, preferably in the range of 0.01 to 5% (w/v) in concentration, and more preferably in the range of 0.1 to 2% (w/v) in concentration. The pharmaceutical composition of the present invention may optionally contain a proper amount of pharmaceutically acceptable additives, for example, emulsifying auxiliary agents, stabilizers, tonicity adjusting agents, pH controllers, or the like. Specifically, the additives include fatty acids of 6 to 22 carbons (e.g., caprylic acid, capric acid, lauric acid, myristic acid, palmitic acid, stearic acid, oleic acid, linoleic acid, arachidonic acid, docosahexaenoic acid) and their pharmaceutically acceptable salts (e.g., sodium salts, potassium salts, calcium salts); emulsifying auxiliary agents such as albumin, dextran; stabilizers such as cholesterol, phosphatidic acid; tonicity adjusting agents such as sodium chloride, glucose, maltose, lactose, sucrose, trehalose; pH controllers such as hydrochloric acid, nitric acid, phosphoric acid, acetic acid, sodium hydroxide, potassium hydroxide, triethanolamine; and the like. The pharmaceutically acceptable additives can be added at a proper step before or after preparation of the complex.

The lyophilized preparations can be prepared by freeze-drying a complex of an oligo double-stranded RNA and a carrier after preparation of the complex. The lyophilization can be carried out in a conventional method. For example, a predetermined amount of the above complex-containing solution after treatment of dispersion is dispensed into vials under sterile condition, then preliminarily dried at about -40 to -20°C for about 2 hours, then primarily dried at about 0 to 10°C under reduced pressure, and secondarily dried at about 15 to 25°C for lyophilization. Thereafter, in general, the vials are filled with nitrogen gas and closed with stoppers to give the lyophilized preparations of the pharmaceutical composition of the present invention.

In general, the lyophilized preparation of the present invention can be reconstituted in an optional suitable solution (reconstituting solution) and used. The reconstituting solution includes water for injection, electrolyte solution such as saline, glucose solution, and other conventional infusions. The amount of the reconstituting solution depends on the purpose of use and suitably is, but is not limited, in 0.5 to 2 fold for the liquid amount before lyophilization, or 500 ml or less.

The pharmaceutical composition of the present invention is preferably administered in a form of dosage unit and may be administered to animals including humans intravenously, intra-arterially, orally, interstitially, percutaneously, transmucosally or rectally, suitably according to the condition of a subject. Particularly preferred is intravenous, percutaneous or trasmucosal administration. In addition, local application such as direct application into cancers may be accepted. The formulation suitable to these ways of administration includes, for example, a variety of injections, oral preparations, drip infusions, absorbents, eye lotions, ointments, lotions, and suppositories.

For example, it is desirable to determine the dose of the pharmaceutical composition of the present invention in consideration of the drug, formulation, subject's condition such as age and body weight, route for administration, property and degree of disease, or the like, and usually the dose is in the range of 0.1 mg to 10 g/day/subject as an oligo double-stranded RNA for an adult, preferably in the range of 1 mg to 500 mg/day/subject. In some cases, the dose may be lower than that or must be increased in other cases. The composition may be administered once or several times a day and at intervals of one to several days.

In another mode of the present invention, it is possible to incorporate a DNA into the pharmaceutical composition together with pharmaceutically acceptable additives, wherein the DNA is used in generating an oligo double-stranded RNA of the present invention. In this context, the DNA used in generating an oligo double-stranded RNA of the present invention means a plasmid or the like for generating the oligo double-stranded RNA of the present invention, which contains a DNA having as deoxyribonucleotides a sequence of the double-strand forming portion in the oligo double-stranded RNA of the ptesent invention, wherein uridine in the nucleotide sequence is replaced with thymine. When such a pharmaceutical composition is administered to a subject, the oligo double-stranded RNA of the present invention is generated in the body and exerts the same effect as the above-mentioned pharmaceutical composition containing an oligo double-stranded RNA and a suitable carrier, that is, the pharmaceutical composition is effective in treatment and/or prevention of diseases caused by over-expression of Bcl-2. Dosage form and route can be determined in the same manner as in the pharmaceutical composition of the present invention containing a complex of an oligo double-stranded RNA and a carrier, and the administration is achieved depending on subject's condition, and the dose can be determined similarly in consideration of the drug, formulation, subject's condition such as age and body weight, route for administration, property and degree of disease, or the like.

The present invention will be explained by the following examples which are not intended as a limitation of the scope thereof.

### Examples

### Example 1: Screening and evaluation thereof

### i) Preparation of an oligo double-stranded RNA

Nucleic acids constituting oligo double-stranded RNAs were synthesized by a standard solid phase phosphoramidite method using an automatic nucleic acid synthesizer. The synthesis was relied on Dharmacon Co. (Colorado, USA) or Japan Bioservice (Saitama Pref., Japan), or achieved by the present inventors.

Briefly, the present inventors performed the synthesis according to the following procedure. Using an automatic DNA synthesizer (Applied Biosystems, Expedite 8909), monomers were condensed one by one by a standard phosphoramidite method to form a desired sequence. Using a concentrated ammonium hydroxide-ethanol (3: 1) mixture, the nucleotide chain was cleaved from CPG (controlled pore glass) and deprotected in the same solution kept at 55°C for 18 hours. The mixture was then treated with 1 M tetrabutylammonium fluoride in tetrahydrofuran solution for 20 hours to remove the 2'-silyl group by deprotection. The resulting oligo-ribonucleotide was purified by reverse-phase chromatography. The product was further treated with 80% acetic acid solution at room temperature for 30 minutes to remove the 5'-DMTr group for deprotection, followed by re-purification by ion-exchange chromatography. After desalination, the resulting oligonucleotide was proved to be the objective full length oligonucleotide in 90% or more purity by means of capillary gel electrophoresis.

Thus, a variety of nucleic acids constituting oligo double-stranded RNAs were synthesized. Oligo double-stranded RNAs were prepared by mixing two nucleic acids of which the double-strand forming portion was complementary, in an equimolar amount as mentioned below.

### ii) Method of evaluation in screening

Evaluation in screening was performed by introducing an oligo double-stranded RNA together with a carrier into a various type of cancer cells and determining the amount of expressed protein by Western blotting and by quasi-determining the mRNA amount by RT-PCR (reverse transcription-polymerase chain reaction).

### Preparation of a complex of an oligo double-stranded RNA and a carrier

Using liposome A comprising 2-O-(2-diethylaminoethyl)carbamoyl-1,3-O-dioleoylglycerol and purified egg yolk lecithin as a carrier, a complex with an oligo double-stranded RNA was prepared. One part by weight of oligo double-stranded RNA was mixed with 16 parts by weight of liposome A to give a complex. The followings indicate the preparation of 2 ml of complex solution containing 10 µM final concentration of the oligo double-stranded RNA. The concentration of the oligo double-stranded RNA indicates the molar concentration of oligo double-stranded RNA contained in the complex assuming that the oligo double-stranded RNA forms a double-strand completely.

A sense strand and an anti-sense strand were respectively dissolved in water for injection so as to be 300 µM, each of which 66.6 µl was mixed in a test tube. One ml of solution containing each strand in a concentration of 20 µM was prepared by adding 866.8 µl of 10% maltose solution to this mixture. This was used as an oligo double-stranded RNA solution.

One ml of a liposome A-dispersed solution at 4.3 mg/ml was prepared by adding 732 µl of 10% maltose solution to 268 µl of the liposome A-dispersed solution of 16 mg/ml. One ml of the above oligo double-stranded RNA-containing solution was slowly added to 1 ml of the prepared liposome A-dispersed solution with stirring. By the above procedure, a solution containing a complex of liposome A with oligo double-stranded RNA in which the final concentration of oligo double-stranded RNA was 10 µM was prepared. The particles of the complex were homogenized by dispersing with a 600 W bath-type ultra-sonicator for 2 minutes.

### Western Blotting

Using the above-mentioned complex of liposome A with oligo double-stranded RNA, it was evaluated whether the expression of Bcl-2 protein was inhibited by transfection of the cells with the oligo double-stranded RNA, by evaluating change of the amount of Bcl-2 protein by Western blotting.

On a Petri dish of 6 cm in diameter, A431 cell (epithelial cancer cell), A375 cell (melanoma cell), MDA-MB-231 cell (breast cancer), or A549 cell (lung cancer) was seeded at 2×10⁵ cells/dish, and incubated in a DMEM medium (Sigma, D6046) containing 10% FBS (fetal bovine serum) overnight at 37°C under5% CO₂. Nextday, the culture medium was removed from the dish under suction, and 2.7 ml of 10 % FBS-DMEM medium (Sigma, D6046) was added for substitution of the medium. There was added 0.3 ml of the solution containing the complex of oligo double-stranded RNA- liposome A (oligo double-stranded RNA : liposome A = 1 : 16 by weight) mixed in 10% maltose solution, so that the final volume was 3 ml. At this point, the final concentration of oligo double-stranded RNA is 3 nM or 10 nM. This was incubated at 37°C in a 5% CO₂ incubator for 72 hours. The cells were washed twice with PBS (phosphate buffered saline) and moved with a cell scraper into a 1.5 ml tube. After centrifugation at 1000xg for 2 minutes and removal of the supernatant, the cells were dissolved in 20-100µl of lysis buffer (50 mM Tris-HCl, pH 7.5, 150 mM NaCl, 1% NP-40). The mixture was allowed to stand on ice for 30 minutes, centrifuged at 100,000xg for 15 minutes, and the supernatant was moved into a fresh tube and kept as a sample for electrophoresis.

Electrophoresis was carried out on polyacrylamide gel (ATTO NPG-520L or E-T520L), on which a sample was applied at 15 µg of total protein for one lane. After termination of electrophoresis, the protein in the gel was transferred on a polyvinylidene fluoride (PVDF) membrane, and blocked in 5% skim milk-containing PBST (PBST-MLK; herein, the composition of PBST was 0.1% Tween 20-containing PBS) at room temperature for 1 hour. First, Bcl-2 protein was detected. The PVDF membrane after blocking was soaked in a mouse anti-human Bcl-2 monoclonal antibody (DAKO M0887) diluted 500 times with PBST-MLK and shaken overnight at 4°C for binding with a primary antibody. The PVDF membrane was washed wit PBST, and shaken with HRP (horse radish peroxidase) labeled anti-mouse Ig antibody (DAKO P0447) diluted 1500 times with PBST-MLK at room temperature for 2 hours to bind a secondary antibody. After washing with PBST, light was emitted from the membrane with Western Lightening Chemiluminescence Reagent Plus (Perkin Elmer) and exposed on a film.

After detection of Bcl-2 protein, the same PVDF membrane was washed with distilled water, and Actin protein was detected in the same manner as Bcl-2. Goat anti-human actin antibody (Santa Cruz sc-1616) was used as a primary antibody, and HRP-labeled anti-goat Ig antibody (DAKO P0449) was used as a secondary antibody. The antibodies were diluted 2500 times and 3000 times, respectively, with PBST-MLK.

The density of band was determined visually by using as a negative control a band density of Bcl-2 protein where transfection with an oligo double-stranded RNA (GL3) inhibiting the expression of luciferase was made, and as a positive control a band density of Bcl-2 protein where transfection with an oligo double-stranded RNA B717 inhibiting the expression of Bcl-2 protein was made. As a result of visual observation, the detection when the density was the same as that of the positive control was designated as "+"; when the density was lighter than that of the positive control, as "++"; and when the density was lighter than that of the negative control, and deeper than that of the positive control, designated as "+/-". In this connection, when the double-strand forming portion was an oligo double-stranded RNA of 25 or more base pairs, the case where the density was much lighter than that of the negative control in a treating concentration of 10 nM was designated as "++"; slightly lighter, "+"; and the case where the density in a treating concentration of 100 nM was slightly lighter than that of the negative control was designated as "+/-".

The term "retention of activity" in the description means that, similarly as above, the band density (activity) of Bcl-2 protein where transfection with an objective oligo double-stranded RNA has been made, is "+/-", "+" or "++" or more.

### Semi-quantification of mRNA by RT-PCR

In order to elucidate whether the inhibitory activity on the expression of Bcl-2 protein by transfection with an oligo double-stranded RNA is caused by inhibition of mRNA expression, the amount of expressed bcl-2 mRNA was semi-quantified by RT-PCR.

On a Petri dish of 6 cm in diameter, A431 cell (epithelial cell) was seeded at a rate of 2×10⁵ cells/dish, and incubated in a DMEM medium (Sigma, D6046) containing 10% FBS (fetal bovine serum) overnight at 37°C under 5% CO₂. Next day, the culture medium was removed from the dish under suction, and 2.7 ml of 10% FBS-DMEM medium (Sigma, D6046) was added for substitution of the medium. There was added 0.3 ml of the solution containing the complex of oligo double-stranded RNA-liposome A (oligo double-stranded RNA. liposome A = 1 : 16 by weight) mixed in 10% maltose solution, so that the final volume was 3 ml. At this point, the final concentration of oligo double-stranded RNA is 10 nM. This was incubated in a CO₂ incubator for 24 hours. The cells were washed twice with PBS, then lysed with 0.5 ml of Isogen (Nippon Gene) and moved into a 1.5 ml tube. Chloroform (200 µl) was added, and the aqueous layer was separated from the organic layer, and the total RNA was isolated from the aqueous layer.

Reverse transcription reaction was conducted using 2 µg of total RNA as a template and Thermoscript RT-PCR System (Gibco BRL 11146-016), and cDNA was prepared. Using this cDNA contained in the reaction mixture of reverse transcription as a template in PCR reaction, PCR amplification specific for bcl-2 and a constitutive expression gene GADPH (D-glyceraldehyde-3-phosphate dehydrogenase) was conducted using a capillary PCR detection/quantification system (Lightcycler, Roche Diagnostics), and the amount of mRNA was determined by semi-puantification. In PCR amplification of bcl-2, 100 ng of cDNA derived from total RNA was used as a template, and in PCR amplification of GADPH, 0.5 ng of cDNA derived from total RNA was used as a template. As a negative control, cells transfected with an oligo double-stranded RNA (GL3) inhibiting the expression of luciferase was used. The amount of sample mRNA was evaluated as a relative ratio when the amount of bcl-2 mRNA or GADPH mRNA in the negative control was regarded as 1.

### iii) Screening

The sequences registered as a mRNA sequence coding for Bcl-2 protein were compared and investigated, and as a result, it was confirmed that the full length mRNA of bcl-2 registered as GenBank Accession NO. BC027258 was the consensus. On the basis of the sequence of this GenBank Accession NO. BC027258 (hereinafter, referred to as bcl-2 mRNA; SEQ ID NO:197), an oligo double-stranded RNA having an inhibitory activity on the expression of Bcl-2 protein was searched by primary, secondary and tertiary screenings as shown in Examples below.

The oligo double-stranded RNAs used in screening were prepared to have a double-strand forming portion formed from a sense RNA strand of 19 bases and an anti-sense RNA strand complementary thereto and have a dTdT as an overhang at the 3'-terminal. The oligo double-stranded RNAs used in screening were represented by "Bxxx", wherein "xxx" was indicated the position number from the initiation point of translation in bcl-2 mRNA of the 5'-teminal base of sense strand in the oligo double-stranded RNA. Primary screening

The oligo double-stranded RNA pool in primary screening was composed of oligo double-stranded RNAs, each comprising a double-strand forming portion consisting of a sense RNA strand of 19 bases beginning every 9 bases in the ORF (open reading frame) of bcl-2 mRNA, and its complementary anti-sense RNA strand. However, in case the result of BRAST search showed that an oligo double-stranded RNA used for screening contained a sequence of 17 successive bases or more complementary to a mRNA other than bcl-2 mRNA, the sequence of the oligo double-stranded RNA was properly shifted by one or two bases to use an oligo double-stranded RNA containing a sequence of no more than 16 successive bases complementary to a mRNA other than bcl-2 mRNA.

As a result of primary screening, the following oligo double-stranded RNAs were recognized to have an inhibitory activity on the expression of Bcl-2 protein: B002 (SEQ ID NO:1 and SEQ ID NO:82), B010 (SEQ ID NO:2 and SEQ ID NO:83), B028 (SEQ ID NO:3 and SEQ ID NO:84), B037 (SEQ ID NO:6 and SEQ ID NO:87), B046 (SEQ ID NO:14 and SEQ ID NO:95), B055 (SEQ ID NO:15 and SEQ ID NO:96), B065 (SEQ ID NO:16 and SEQ ID NO:97), B073 (SEQ ID NO:17 and SEQ ID NO:98), B084 (SEQ ID NO:18 and SEQ ID NO:99), B136 (SEQ ID NO:19 and SEQ ID NO:100), B172 (SEQ ID NO:20 and SEQ ID NO:101), B199 (SEQ ID NO:21 and SEQ ID NO:102), B207 (SEQ ID NO:22 and SEQ ID NO:103), B253 (SEQ ID NO:23 and SEQ ID NO:104), B262 (SEQ DNO:24 and SEQ ID NO:105), B280 (SEQ ID NO:25 and SEQ ID NO:106), B325 (SEQ ID NO:26 and SEQ ID NO:107), B352 (SEQ ID NO:27 and SEQ ID NO: 108), B397 (SEQ ID NO:28 and SEQ ID NO:109), B433 (SEQ ID NO:29 and SEQ ID NO:110), B442 (SEQ ID NO:32 and SEQ ID NO:113), B451 (SEQ ID NO:33 and SEQ ID NO:114), B469 (SEQ ID NO:36 and SEQ ID NO:117), B478 (SEQ ID NO:39 and SEQ ID NO:120), B516 (SEQ ID NO:40 and SEQ ID NO:121), B523 (SEQ ID NO:41 and SEQ ID NO:122), B539 (SEQ ID NO:46 and SEQ ID NO:127), B558 (SEQ ID NO:49 and SEQ ID NO:130), B576 (SEQ !D NO: 50 and SEO ID NO:131), B586 (SEQ ID NO:51 and SEQ ID NO:132), B595 (SEQ ID NO:52 and SEQ ID NO:133), B604 (SEQ ID NO:53 and SEQ ID NO:134), B613 (SEQ ID NO:54 and SEQ ID NO:135), B622 (SEQ ID NO:59 and SEQ ID NO:140), B631 (SEQ ID NO:62 and SEQ ID NO:143), B642 (SEQ ID NO:65 and SEQ ID NO:146), B649 (SEQ ID NO: 66 and SEQ ID NO:147), B654 (SEQ ID NO:67 and SEQ ID NO:148), B658 (SEQ ID NO:68 and SEQ ID NO:149), B667 (SEQ !D NO:69 and SEQ ID NO:150), B676 (SEQ ID NO:70 and SEQ ID NO:151), B703 (SEQ ID NO:73 and SEQ ID NO:154), B712 (SEQ ID NO:76 and SEQ ID NO:157), B717 (SEQ ID NO:77 and SEQ ID NO:158), and B721 (SEQ ID NO:79 and SEQ ID NO:160). These oligo double-stranded RNAs were evaluated by Western blotting for an inhibitory activity of the expression of Bcl-2 protein; Table 1 provided in the last of this section shows the results.

Among the oligo double-stranded RNAs which had an inhibitory activity on the expression of Bcl-2 protein in the primary screening, the following 9 oligo double-stranded RNAs showed a particularly high activity.
B037:
   sense strand
      5'-GAGAUAGUGAUGAAGUACA-dTdT-3' (SEQ ID NO:6)
   anti-sense strand
      5'-UGUACUUCAUCACUAUCUC-dTdT-3' (SEQ ID NO:87)
B433:
   sense strand
      5'-GGGAGGAUUGUGGCCUUCU-dTdT-3' (SEQ ID NO:29)
   anti-sense strand
      5'-AGAAGGCCACAAUCCUCCC-dTdT-3' (SEQ ID NO:110)
B469:
   sense strand
      5'-AUGUGUGUGGAGAGCGUCA-dTdT-3' (SEQ ID NO:36)
   anti-sense strand
      5'-UGACGCUCUCCACACACAU-dTdT-3' (SEQ ID NO:117)
B539:
   sense strand
      5'-ACCUGAACCGGCACCUGCA-dTdT-3' (SEQ ID NO:46)
   anti-sense strand
      5'-UGCAGGUGCCGGUUCAGGU-dTdT-3' (SEQ ID NO:127)
B622:
   sense strand
      5'-CCUCUGUUUGAUUUCUCCU-dTdT-3' (SEQ ID NO:59)
   anti-sense strand
      5'-AGGAGAAAUCAAACAGAGG-dTdT-3' (SEQ ID NO:140)
B631:
   sense strand
      5'-GAUUUCUCCUGGCUGUCUC-dTdT-3' (SEQ ID NO:62)
   anti-sense strand
      5'-GAGACAGCCAGGAGAAAUC-dTdT-3' (SEQ ID NO:143)
B703:
   sense strand
      5'-UAUCUGGGCCACAAGUGAA-dTdT-3' (SEQ ID NO:73)
   anti-sense strand
      5'-UUCACUUGUGGCCCAGAUA-dTdT-3' (SEQ ID NO:154)
B717:
   sense strand
      5'-GUGAAGUCAACAUGCCUGC-dTdT-3' (SEQ ID NO:77)
   anti-sense strand
      5'-GCAGGCAUGUUGACUUCAC-dTdT-3' (SEQ ID NO:158)
B721:
   sense strand
      5'-AGUCAACAUGCCUGCCCCA-dTdT-3' (SEQ ID NO:79)
   anti-sense strand
      5'-UGGGGCAGGCAUGUUGACU-dTdT-3' (SEQ ID NO:160)

For the 9 oligo double-stranded RNAs (B037, B433, B469, B539, B622, B631, B703, B717 and B721) showing a high inhibitory activity on the expression of Bcl-2 protein, the amount of bcl-2 mRNA was evaluated by RT-PCR. Fig. 1 shows the results. In the figure, the vertical axis means the relative amount of bcl-2 mRNA expressed, where the amount of bcl-2 mRNA generated in A431 cells transfected with the oligo double-stranded RNA inhibiting the expression of GL3 (firefly luciferase) as a negative control and incubated for 24 hours is scored as 1, and the relative amounts of bcl-2 mRNA generated in the cells similarly transfected with the respective oligo double-stranded RNAs are shown. In all of the cells transfected with the 9 oligo double-stranded RNAs obtained in the primary screening, decrease of the amount of bcl-2 mRNA was observed in comparison with the negative control. In subsequent experiments, B717 was used as an oligo double-stranded RNA of a positive control. Secondary screening

The oligo double-stranded RNA pool in the secondary screening was composed of 8 oligo double-stranded RNAs (B037, B433, B469, B539, B622, B631, B703 and B721) excluding B717 from the above 9 oligo double-stranded RNAs which showed a high inhibitory activity on the expression of Bcl-2 protein in the primary screening; and of such oligo double-stranded RNAs that comprise a double-strand forming portion consisting of a sense RNA strand of 19 bases beginning from the point distant by 3 bases or 6 bases toward upstream or downstream along the bcl-2 mRNA from the 5'-terminal of the sense RNA strand of the respective 8 oligo double-stranded RNAs, and its complementary RNA strand. However, in case the result of BRAST search showed that an oligo double-stranded RNA used for screening contained a sequence of 17 successive bases or more complementary to a mRNA other than bcl-2 mRNA, the sequence of the oligo double-stranded RNA was properly shifted by one or two bases to use an oligo double-stranded RNA containing a sequence of no more than 16 successive bases complementary to a mRNA other than bcl-2 mRNA.

As a result of secondary screening, the following oligo double-stranded RNAs were recognized to have an inhibitory activity on the expression of Bcl-2 protein: B031 (SEQ ID NO:4 and SEQ ID NO:85), B034 (SEQ ID NO:5 and SEQ ID NO:86), B037 (SEQ ID NO:6 and SEQ ID NO:87), B040 (SEQ ID NO:8 and SEQ ID NO:89), B043 (SEQ ID NO:11 and SEQ ID NO:92), B433 (SEQ ID NO:29 and SEQ ID NO:110), B436 (SEQ ID NO:30 and SEQ ID NO:111), B439 (SEQ ID NO:31 and SEQ ID NO:112), B463 (SEQ ID NO:34 and SEQ ID NO:115), B466 (SEQ ID NO:35 and SEQ ID NO:116), B469 (SEQ ID NO:36 and SEQ ID NO:117), B472 (SEQ ID NO:37 and SEQ ID NO:118), B475 (SEQ ID NO:38 and SEQ ID NO:119), B533 (SEQ ID NO:43 and SEQ ID NO:124), B536 (SEQ ID NO:45 and SEQ ID NO: 126), B539 (SEQ ID NO:46 and SEQ ID NO:127), B543 (SEQ ID NO:47 and SEQ ID NO:128), B545 (SEQ ID NO:48 and SEQ ID NO:129), B616 (SEQ ID NO:57 and SEQ ID NO:138), B619 (SEQ ID NO:58 and SEQ ID NO:139), B622 (SEQ ID NO:59 and SEQ ID N0:140),B625 (SEQ ID NO:60 and SEQ ID NO:141), B628 (SEQ ID NO:61 and SEQ ID NO;142), B631 (SEQ ID NO: 62 and SEQ ID NO: 143), B634 (SEQ ID NO: 63 and SEQ ID NO: 144), B636 (SEQ !D NO:64 and SEQ ID NO:145), B697 (SEQ ID NO:71 and SEQ ID NO:152), B700 (SEQ ID NO:72 and SEQ ID NO:153), B703 (SEQ ID NO:73 and SEQ ID NO:154), B706 (SEQ ID NO:74 and SEQ ID NO:155), B709 (SEQ ID NO:75 and SEQ ID NO:156), B719 (SEQ ID NO:78 and SEQ ID NO:159), B721 (SEQ ID NO:79 and SEQ ID NO:160), B724 (SEQ ID NO:80 and SEQ ID N0:161), and B727 (SEQ ID NO:81 and SEQ ID NO:162). These oligo double-stranded RNAs were evaluated by Western blotting for an inhibitory activity of the expression of Bcl-2 protein; Table 1 provided in the last of this section shows the results.

Among the oligo double-stranded RNAs which had an inhibitory activity on the expression of Bcl-2 protein in the secondary screening, the following 7 oligo double-stranded RNAs showed a particularly high activity.
8037:
   sense strand
      5` -GAGAUAGUGAUGAAGUACA-dTdT-3' (SEQ I D NO:6)
   anti-sense strand
      5`-UGUACUUCAUCACUAUCUC-dTdT-3' (SEQ SD NO:87)
B043:
   sense strand
      5'-GUGAUGAAGUACAUCCAUU-dTdT-3' (SEQ ID NO:11)
   anti-sense strand
      5'-AAUGGAUGUACUUCAUCAC-dTdT-3' (SEQ ID NO:92)
B436:
   sense strand
      5'-AGGAUUGUGGCCUUCUUUG-dTdT-3` (SEQ ID NO:30)
   anti-sense strand
      5'-CAAAGAAGGCCACAAUCCU-dTdT-3' (SEQ I D NO:111)
B469:
   sense strand
      5'-AUGUGUGUGGAGAGCGUCA-dTdT-3' (SEQ ID NO:36)
   anti-sense strand
      5'-UGACGCUCUCCACACACAU-dTdT-3' (EEQ ID NO:117)
B533:
   sense strand
      5'-CUGAGUACCUGAACCGGCA-dTdT-3' (SEQ ID NO:43)
   anti-sense strand
      5'-UGCCGGUUCAGGUACUCAG-dTdT-3' (SEQ I D NO:124)
8616:
   sense strand
      5'-AUGCGGCCUCUGUUUGAUU-dTdT-3' (SEQ ID NO:57)
   anti-sense strand
      5'-AAUCAAACAGAGGCCGCAU-dTdT-3' (SEQ ID NO:138)
B631:
   sense strand
      5'-GAUUUCUCCUGGCUGUCUC-dTdT-3' (SEQ ID NO:62)
   anti-sense strand
      5'-GAGACAGCCAGGAGAAAUC-dTdT-3' (SEQ ID NO:143)

Among them, the cells transfected with the respective oligo double-stranded RNAs of B043 and B533 were evaluated for the amount of bcl-2 mRNA by RT-PCR; Fig. 2a shows the results. In Figure, the vertical axis indicates the ratio of the amount of bcl-2 mRNA generated after transfection of the respective oligo double-stranded RNAs in the same manner as below, wherein the oligo double-stranded RNA inhibiting the expression of GL3 (firefly luciferase) as a negative control was introduced into A431 cell, followed by incubation for 24 hours, and the amount of bol-2 mRNA generated in the cell was regarded as 1. It was observed that the amount of bcl-2 mRNA was reduced in the cells transfected with either of the oligo double-stranded RNAs as compared to the negative control. Further, it was observed that the amount of bcl-2 mRNA was reduced more than in transfection with an oligo double-stranded RNA of the positive control B717 (Fig. 2a). On the other hand, no influence was observed in the amount of mRNA of a house keeping gene GAPDH (Fig. 2b). In addition. A431 cell was transfected with the respective oligo double-stranded RNAs of B043 and B533, and incubated for 72 hours; the expressed amount of Bcl-2 protein in the resulting cells was evaluated by Western blotting; Fig. 2c shows a photograph of the result. When transfection with an oligo double-stranded RNA at 10 nM was made, it was observed that the selected oligo double-stranded RNA markedly inhibited the expression of Bcl-2 protein (Fig. 2c; upper case). On the other hand, no influence was observed in the amount of a control Actin protein (Fig. 2c: lower case).

Therefore, it was demonstrated that all of the oligo double-stranded RNAs obtained in the secondary screening specifically cleft bcl-2 mRNA and specifically inhibited the expression of Bcl-2 protein.

### Tertiary screening

The oligo double-stranded RNA pool in the tertiary screening was composed of 4 oligo double-stranded RNAs (B037, B043, B533 and B616) which showed a high inhibitory activity on the expression of Bcl-2 protein in the secondary screening, and of such oligo double-stranded RNAs that comprise a double-strand forming portion consisting of a sense RNA strand of 19 bases beginning from the point shifted one by one in the range of about 3 bases toward upstream or downstream along the bcl-2 mRNA from the 5'-terminal of the sense RNA strand of the respective 4 oligo double-stranded RNAs, and its complementary RNA strand. In this operation, however, the sequences of high GC content, which were expected to decrease the synthetic yield, were excluded from the targets for screening.

The results of evaluation of the inhibitory activity on the expression of Bcl-2 protein by Western blotting are shown by photographs in Fig. 3 and Fig. 4, according to which a screening process of screening procedure will be explained as follows. In Fig. 3, "Normal" indicates A431 cell which has not been transfected with an oligo double-stranded RNA; and in Fig. 3 and Fig. 4, "GL3" indicates a cell which has been transfected with an oligo double-stranded RNA inhibiting the expression of firefly luciferase used as a negative control. Actin protein shown in the lower case in each photograph exhibits approximately the same degree of coloration in any lane, and the result indicates that there is no large difference in the sample amount applied to electrophoresis among the respective lanes. Therefore, the degree of coloration of Bcl-2 protein was directly compared, and when the band density of Bcl-2 protein was markedly reduced, the inhibitory activity on the expression of Bod-2 protein in the corresponding oligo double-stranded RNA was determined to be high.

As a result of tertiary screening, the following oligo double-stranded RNAs were recognized to have an inhibitory activity on the expression of Bcl-2 protein: B037 (SEQ ID NO:6 and SEQ ID NO:87), B039 (SEQ ID NO:7 and SEQ ID NO:88), B040 (SEQ ID NO:8 and SEQ ID NO:89), B041 (SEQ ID NO:9 and SEQ ID NO:90), B042 (SEQ ID NO:10 and SEQ ID NO:91), B043 (SEQ ID NO:11 and SEQ ID NO:92), B044 (SEQ ID NO:12 and SEQ ID NO:93), B045 (SEQ ID NO:13 and SEQ ID NO:94), B531 (SEQ ID NO:4 and SEQ ID NO:123), B533 (SEQ ID NO:43 and SEQ ID NO:124), B534 (SEQ ID NO:44 and SEQ ID NO:125), B614 (SEQ ID NO:55 and SEQ ID NO:136), B015 (SEQ ID NO:56 and SEQ ID NO:137), and B616 (SEQ ID NO:57 and SEQ ID NO:138). These oligo double-stranded RNAs were evaluated by Western blotting for an inhibitory activity of the expression of Bcl-2 protein; Table 1 provided in the last of this section shows the results.

The following 3 oligo double-stranded RNAs having a particularly high inhibitory activity on the expression of Bcl-2 protein were selected.
B043:
   sense strand
      5'-GUGAUGAAGUACAUCCAUU-dTdT-3' (SEQ ID NO:11)
   anti-sense strand
      5'-AAUGGAUGUACUUCAUCAC-dTdT-3' (SEQ ID NO:92)
B533:
   sense strand
      5'-CUGAGUACCUGAACCGGCA-dTdT-3' (SEQ I D NO:43)
   anti-sense strand
      5'-UGCCGGUUCAGGUACUCAG-dTdT-3' (SEQ ID NO:124)
B614:
   sense strand
      5'-GCAUGCGGCCUCuGUUUGA-dTdT-3-' (SEQ ID NO:55)
   anti-sense strand
      5'-UCAAACAGAGGCCGCAUGC-dTdT-3' (SEQ ID NO:136)

It was found that these 3 oligo double-stranded RNAs (B043, B533 and B614), when transfected at 10 nM, almost perfectly inhibited the expression of Bcl-2 protein, and even in transfection at 3 nM, the expression of Bcl-2 protein was significantly inhibited (Fig. 3 and Fig. 4).

Table 1, lists the oligo double-stranded RNAs which have been recognize to have an inhibitory activity on the expression of proteins in the course of the primary to tertiary screening.

**[Table 1-a]**

| Table 1: the oligo double-stranded RNA confirmed activity by screening | | | |
|---|---|---|---|
| oligo double-stranded RNA | sense strand / anti-sense strand | sequence | activity |
| B002 | sense strand | UGGCGCACGCUGGGAGAAC-dTdT (SEQ ID NO:1) | +- |
| | anti-sense strand | GUUCUCCCAGCGUGCGCCA-dTdT (SEQ ID NO:82) | |
| B010 | sense strand | GCUGGGAGAACGGGGUACG-dTdT (SEQ ID NO:2) | + |
| | anti-sense strand | CGUACCCCGUUCUCCCAGC-dTdT (SEQ ID NO:83) | |
| B028 | sense strand | GAUAACCGGGAGAUAGUGA-dfidT (SEQ ID NO:3) | + |
| | anti-sense strand | UCACUAUCUCCCGGUUAUC-dTdT (SEQ ID NO:84) | |
| B031 | sense strand | AACCGGGAGAUAGUGAUGA-dTdT (SEQ ID NO:4) | + |
| | anti-sense strand | UCAUCACUAUCUCCCGGUU-dTdT (SEQ ID NO:85) | |
| B034 | sense strand | CGGGAGAUAGUGAUGAAGU-dTdT (SEQ ID NO:5) | + |
| | anti-sense strand | ACUUCAUCACUAUCUCCCG-dTdT (SEQ ID NO:86) | |
| B037 | sense strand | GAGAUAGUGAUGAAGUACA-dTdT (SEQ ID NO:6) | ++ |
| | anti-sense strand | UGUACUUCAUCACUAUCUC-dTdT (SEQ ID NO:87) | |
| B039 | sense strand | GAUAGUGAUGAAGUACAUC-dTdT (SEQ ID NO:7) | + |
| | anti-sense strand | GAUGUACUUCAUCACUAUC-dTdT (SEQ ID NO:86) | |
| B040 | sense strand | AUAGUGAUGAAGUACAUCC-dTdT (SEQ ID NO:8) | +- |
| | anti-sense strand | GGAUGUACUUCAUCACUAU-dTdT (SEQ ID NO:89) | |
| B041 | sense strand | UAGUGAUGAAGUACAUCCA-dTdT (SEQ ID NO:9) | + |
| | anti-sense strand | UGGAUGUACUUCAUCACUAdTdT (SEQ ID NO:90) | |
| B042 | sense strand | AGUGAUGAAGUACAUCCAU-dTdT (SEQ ID NO:10) | + |
| | anti-sense strand | AUGGAUGUACUUCAUCACU-dTdT (SEQ ID NO:91) | |
| B043 | sense strand | GUGAUGAAGUACAUCCAUU-dTdT (SEQ ID NO:11) | ++ |
| | anti-sense strand | AAUGGAUGUACUUCAUCAC-dTdT (SEQ ID NO:92) | |
| B044 | sense strand | UGAUGAAGUACAUCCAUUA-dTdT (SEQ ID NO:12) | ++ |
| | anti-sense strand | UAAUGGAUGUACUUCAUCA-dTdT (SEQ ID NO:93) | |
| B045 | sense strand | GAUGAAGUACAUCCAUUAU-dTdT (SEQ ID NO:13) | + |
| | anti-sense strand | AUAAUGGAUGUACUUCAUC-dTdT (SEQ ID NO:94) | |
| B046 | sense strand | AUGAAGUACAUCCAUUAUA-dTdT (SEQ ID NO:14) | + |
| | anti-sense strand | UAUAAUGGAUGUACUUCAU-dTdT (SEQ ID NO:95) | |
| B055 | sense strand | AUCCAUUAUAAGCUGUCGC-dTdT (SEQ ID N0:15) | +- |
| | anti-sense strand | GCGACAGCUUAUAAUGGAU-dTdT (SEQ ID NO:96) | |
| B065 | sense strand | AGCUGUCGCAGAGGGGCUA-dTdT (SEQ ID NO:16) | + |
| | anti-sense strand | UAGCCCCUCUGCGACAGCU-dTdT (SEQ ID NO:97) | |
| B073 | sense strand | CAGAGGGGCUACGAGUGGG-dTdT (SEQ ID NO:17) | +- |
| | anti-sense strand | CCCACUCGUAGCCCCUCUG-dTdT (SEQ ID NO:98) | |
| B084 | sense strand | CGAGUGGGAUGCGGGAGAU-dTdT (SEQ ID NO:18) | + |
| | anti-sense strand | AUCUCCCGCAUCCCACUCG-dTdT (SEQ ID NO:99) | |
| B136 | sense strand | CCGGGCAUCUUCUCCUCCC-dTdT (SEQ ID NO:19) | +- |
| | anti-sense strand | GGGAGGAGAAGAUGCCCGG-dTdT (SEQ ID NO:100) | |

**[Table 1-b]**

| | | | |
|---|---|---|---|
| B172 | sense strand | CAUCCAGCCGCAUCCCGGG-dTdT (SEQ ID NO:20) | + |
| | anti-sense strand | CCCGGGAUGCGGCUGGAUG-dTdT (SEQ ID NO:101) | |
| B199 | sense strand | GCCAGGACCUCGCCGCUGC-dTdT (SEQ ID NO:21) | + |
| | anti-sense strand | GCAGCGGCGAGGUCCUGGC-dTdT (SEQ ID NO:102) | |
| B207 | sense strand | CUCGCCGCUGCAGACCCCG-dTdT (SEQ ID ND:22) | +- |
| | anti-sense strand | CGGGGUCUGCAGCGGCGAG-dTdT (SEQ ID NO:103) | |
| B253 | sense strand | GCGCUCAGCCCGGUGCCAC-dTdT (SEQ ID NO:23) | + |
| | anti-sense strand | GUGGCACCGGGCUGAGCGC-dTdT (SEQ ID NO:104) | |
| B262 | sense strand | CCGGUGCCACCUCUCCUCC-dTdT (SEQ ID NO:24) | +- |
| | anti-sense strand | GGACCACAGGUGGCACCGG-dTdT (SEQ ID NO:105) | |
| B280 | sense strand | CACCUGACCCUCCGCCAGG-dTdT (SEQ ID ND:25) | +- |
| | anti-sense strand | CCUGGCGGAGGGUCAGGUG-dTdT (SEQ ID NO:106) | |
| B325 | sense strand | CGCCGCGACUUCGCCGAGA-dTdT (SEQ ID NO:26) | + |
| | anti-sense strand | UCUCGGCGAAGUCGCGGCG-dTdT (SEQ ID NO:107) | |
| B352 | sense strand | CAGCUGCACCUGACGCCCU-dTdT (SEQ ID NO:27) | + |
| | anti-sense strand | AGGGCGUCAGGUGCAGCUG-dTdT (SEQ ID NO:108) | |
| B397 | sense strand | GUGGUGGAGGAGCUCUUCA-dTdT (SEQ ID NO:28) | + |
| | anti-sense strand | UGAAGAGCUCCUCCACCAC-dTdT (SEQ ID NO:109) | |
| B433 | sense strand | GGGAGGAUUGUGGCCUUCU-dTdT (SEQ ID NO:29) | ++ |
| | anti-sense strand | AGAAGGCCACAAUCCUCCC-dTdT (SEQ ID ND:110) | |
| B436 | sense strand | AGGAUUGUGGCCUUCUUUG-dTdT (SEQ ID NO:30) | ++ |
| | anti-sense | strand CAAAGAAGGCCACAAUCCU-dTdT SEQ ID NO:111) | |
| B439 | sense strand | AUUGUGGCCUUCUUUGAGU-dTdT (SEQ ID NO:31) | ++ |
| | anti-sense strand | ACUCAAAGAAGGCCACAAU-dTdT (SEQ ID NO:112) | |
| B442 | sense strand | GUGGCCUUCUUUGAGUUCG-dTdT (SEQ ID NO:32) | + |
| | anti-sense strand | CGAACUCAAAGAAGGCCAC-dTdT (SEQ ID NO:113) | |
| B451 | sense strand | UUUGAGUUCGGUGGGGUCA-dTdT (SEQ ID NO:33) | +- |
| | anti-sense strand | UGACCCCACCGAACYCAAA-dTdT (SEQ ID NO:114) | |
| B463 | sense strand | GGGCUCAUGUCUGUGGAGA-dTdT (SEQ ID NO:34) | ++ |
| | anti-sense strand | UCUCCACACACAUGACCCC-dTdT (SEQ ID NO:115) | |
| B466 | sense strand | GUCAUGUGUGUGGAGAGCG-dTdT (SEQ ID NO:35) | +- |
| | anti-sense strand | CGCVCUCCACACAUGAC-dTdT (SEQ ID NO:116) | |
| B469 | sense strand | AUGUGUGUGGAGAGCGUCA-dTdT (SEQ ID NO:36) | ++ |
| | anti-sense strand | UGACGCUCUCCACACACAU-dTdT (SEQ ID NO:117) | |
| B472 | sense strand | UGUGUGGAGAGCGUCAACC-dTdT (SEQ ID NO:37) | + |
| | anti-sense strand | GGUUGACGCUCCACACA-dTdT (SEQ ID NO:118) | |
| B475 | sense strand | GUGGAGAGCGUCAACCGGG-dTdT (SEQ ID NO:38) | +- |
| | anti-sense strand | CCCGGUUGACGCUCUCCAC-dTdT (SE2 ID NO:119) | |
| 8478 | sense strand | GAGAGCGOCAACCGGGAGA-dTdT (SEQ ID NO:39) | + |
| | anti-sense strand | UCUCCCGGUUGACGCUCUC-dTdT (SEQ ID NO:120) | |
| B516 | sense strand | CAUCGCCCUGUGGAUGACU-dTdT (SEQ ID NO:40) | +- |
| | anti-sense strand | AGUCAUCCACAGGGCGAUG-dTdT (SEQ ID NO:121) | |
| B523 | sense strand | CUGUGGAUCUGGAGUACC-dTdT (SEQ ID NO:41) | + |
| | anti-sense strand | GGUACUCAGUCAUCCACAG-dTdT (SEQ ID NO:122) | |

**[Table 1-c]**

| | | | |
|---|---|---|---|
| B531 | sense strand | GACUGAGUACCUGAACCGG-dTdT (SEQ ID NO:42) | + |
| | anti-sense strand | CCGGUUCAGGUACUCAGUC-dTdT (SEQ ID NO:123) | |
| B533 | sense strand | CUGAGUACCUGAACCGGCA-dTdT (SEQ ID NO:43) | ++ |
| | anti-sense strand | UGCCGGUUCAGGUACUCAG-dTdT (SEQ ID NO:124) | |
| B534 | sense strand | UGAGUACCUGAACCGGCAC-dTdT (SEQ ID NO:44) | +- |
| | anti-sense strand | GUGCCGGUUCAGGUACUCA-dTdT (SEQ ID NO:125) | |
| B536 | sense strand | AGURCCUGAACCGGCACCU-dTdT (SEQ ID NO:45) | +- |
| | anti-sense strand | AGGUGCCGGUUCAGGUACU-dTdT (SEQ ID NO:126) | |
| B539 | sense strand | ACCUGAACCGGCACCUGCA-dTdT (SEQ ID NO:46) | ++ |
| | anti-sense strand | UGCAGGUGCCGGUUCAGGU-dTdT (SEQ ID NO:127) | |
| B543 | sense strand | GAACCGGCACCUGCACACC-dTdT (SEQ ID NO:47) | +- |
| | anti-sense strand | GGUGUGCAGGUGCCGGUUC-dTdT (SEQ ID NO:128) | |
| B545 | sense strand | ACCGGCACCUGCACACCUG-dTdT (SEQ ID NO:48, | + |
| | anti-sense strand | CAGGUGUGCAGGUGCCGGU-dTdT (SEQ ID NO:129) | |
| B558 | sense strand | CACCUGGAUCCAGGAUAAC-dTdT (SEQ ID NO:49) | +- |
| | anti-sense strand | GUUAUCCUGGAUCCAGGUG-dTdT (SEQ ID NO:130) | |
| B576 | sense strand | CGGAGGCUGGGAUGCCUUU-dTdT (SEQ ID NO:50) | +- |
| | anti-sense strand | AAAGGCAUCCCAGCCUCCG-dTdT (SEQ ID NO:131) | |
| B586 | sense strand | GAUGCCUUUGUGGAACUGU-dTdT (SEQ ID NO:51) | +- |
| | anti-sense strand | ACAGUUCCACAAAGGCAUC-dTdT (SEQ ID NO:132) | |
| B595 | sense strand | GUGGAACUGUACGGCCCCA-dTdT (SEQ ID NO:52) | +- |
| | anti-sense strand | UGGGGCCGUACAGUUCCAC-dTdT (SEQ ID NO:133) | |
| B604 | sense strand | UACGGCCCCAGCAUGCGGC-dTdT (SEQ ID NO:53) | +- |
| | anti-sense strand | GCCGCAUGCUGGGGCCGUA-dTdT (SEQ ID NO:134) | |
| B613 | sense strand | AGCAUGCGGCCUCUGUUUG-dTdT (SEQ ID NO:54) | +- |
| | anti-sense strand | CAAACAGAGGCCGCAUGCU-dTdT (SEQ ID NO:135) | |
| B614 | sense strand | GCAUGCGGCCUCUGUUUGA-dTdT (SEQ ID NO:55) | ++ |
| | anti-sense strand | UCAAACAGAGGCCGCAUCC-dTdT (SEQ ID NO:136) | |
| B615 | sense strand | CAUGCGGCCUCUGUUUGAU-dTdT (SEQ ID NO:56) | + |
| | anti-sense strand | AUCAAACAGAGGCCGCAUG-dTdT (SEQ ID NO:137) | |
| B616 | sense strand | AUGCGGCCUCUGUUUGAUU-dTdT (SEQ ID NO:57) | + |
| | anti-sense strand | AAUCAAACAGAGGCCGCAU-dTdT (SEQ ID NO:138) | |
| B619 | sense strand | CGGCCUCUGUUUGAUUUCU-dTdT (SEQ ID NO:56) | +÷ |
| | anti-sense strand | AGAAAUCAAACAGAGGCCG-dTdT (SEQ ID NO:139) | |
| B622 | sense strand | CCUCUGUUUGAUUUCUCCU-dTdT (SEQ ID NO:59) | ++ |
| | anti-sense strand | AGGAGAAAYCAAACAGAGG-dTdT (SEQ ID NO:140) | |
| B625 | sense strand | CUGUUUGAUUUCUCCUGGC-dTdT (SEQ ID NO:60) | + |
| | anti-sense strand | GCCAGGAGAAAUCAAACAG-dTdT (SEQ ID NO:141) | |
| B628 | sense strand | UUUGAUUUCUCCUGGCUGU-dTdT (SEQ ID NO:61) | + |
| | anti-sense strand | ACAGCCAGGAGAAAUCAAA-dTdT (SEQ ID NO:142) | |
| B631 | sense strand | GAUUUCUCCUGGCUGUCUC-dTdT (SEQ ID NO:62) | ++ |
| | anti-sense strand | GAGACAGCCAGGAGAAAUC-dTdT (SEQ ID NO:143) | |
| B634 | sense strand | UUCUCCUGGCUGUCUCUGA-dTdT (SEQ ID NO:63) | + |
| | anti-sense strand | UCAGAGACAGCCAGGAGAA-dTdT (SEQ ID NO:144) | |

**[Table 1-d]**

| | | | |
|---|---|---|---|
| B636 | sense strand | CUCCUGGCUGUCUCUGAAG-dTdT (SEQ ID NO:64) | + |
| | anti-sense strand | CUUCAGAGACAGCCAGGAG-dTdT (SEQ ID NO:145) | |
| B642 | sense strand | GCUGUCUCUGAAGACUCUG-dTdT (SEQ ID NO 65) | + |
| | anti-sense strand | CAGAGUCUUCAGAGACAGC-dTdT (SEO ID NO:146) | |
| B649 | sense strand | CUGAAGACUCUGCUCAGUU-dTdT (SEQ ID NO 66) | +- |
| | anti-sense strand | AACUGAGCAGAGUCUUCAG-dTdT (SEQ ID NO:147) | |
| B654 | sense strand | GACUCUGCUCAGUUUGGCC-dTdT (SEQ ID NO 67) | +- |
| | anti-sense strand | GGCCAAACUGAGCAGAGUC-dTdT (SEQ ID NO:148) | |
| B658 | sense strand | CUGCUCAGUUUGGCCCUGG-dTdT (SEQ ID NO:68) | +- |
| | anti-sense strand | CCAGGGCCAAACUGAGCAG-dTdT (SEQ ID NO:149) | |
| B667 | sense strand | UUGGCCCUGGUGGGAGCUU-dTdT (SEQ ID NO:69) | +- |
| | anti-sense strand | AAGCUCCCACCAGGGCCAA-dTdT (SEQ ID NO:150) | |
| B676 | sense strand | GUGGGAGCUUGCAUCACCC-dTdT (SEQ ID NO:70) | +- |
| | anti-sense strand | GGGUGAUGCAAGCUCCCAC-dTdT (SEQ ID NO:151) | |
| B697 | sense strand | GGUGCCUAUCUGGGCCACA-dTdT (SEQ ID NO:71) | +- |
| | anti-sense strand | UGUGGCCCAGAUAGGCACC-dTdT (SEQ ID NO:152) | |
| B700 | sense strand | GCCUAUCUGGGCCACAAGU-dTdT (SEQ ID NO:72) | + |
| | anti-sense strand | ACUUGUGGCCCAGAUAGGC-dTdT (SEQ ID NO:153) | |
| B703 | sense strand | UAUCUGGGCCACAAGUGAA-dTdT (SEQ ID NO:73) | ++ |
| | anti-sense strand | UUCACUUGUGGCCCAGAUA-dTdT (SEQ ID NO:154) | |
| B706 | sense strand | CUGGGCCACAAGUGAAGUC-dTdT (SEQ ID NO:74) | + |
| | anti-sense strand | GACUUCACUUGUGGCCCAG-dTdT (SEQ ID NO:155) | |
| B709 | sense strand | GGCCACAAGUGAAGUCAAC-dTdT (SEQ ID NO:75) | ++ |
| | anti-sense strand | GUUGACUUCACUUGUGGCC-dTdT (SEQ ID NO:156) | |
| B712 | sense strand | SACAAGUGAAGUCAACAUG-dTdT (SEQ ID NO:76) | + |
| | anti-sense strand | CAUGUUGACUUCACUUGUG-dTdT (SEQ ID NO:157) | |
| B717 | sense strand | GUGAAGUCAACAUGCCUGC-dTdT (SEQ ID NO:77) | + |
| | anti-sense strand | GCAGGCAUGUUGACUUCAC-dTdT (SEQ ID NO:158) | |
| B729 | sense strand | GAAGUCAACAUGCCUGCCC-dTdT (SEQ ID NO:78) | +- |
| | anti-sense strand | GGGCAGGCAUGUUGACUUC-dTdT (SEQ ID NO:159) | |
| B721 | sense strand | AGUCAACAUGCCUGCCCCA-dTdT (SEQ ID NO;79) | ++ |
| | anti-sense strand | UGGGGCAGGCAUGUUGACU-dTdT (SEQ ID NO:160) | |
| B724 | sense strand | CAACAUGCCUGCCCCAAAC-dTdT (SEQ ID NO:80) | ++ |
| | anti-sense strand | GUUUGGGGCAGGCAUGUUG-dTdT (SEQ ID NO:161) | |
| B727 | sense strand | CAUGCCUGCCCCAAACAAA-dTdT (SEQ ID NO:81) | ++ |
| | anti-sense strand | UUUGUUUGGGGCAGGCAUG-dTdT (SEQ ID NO:162) | |

| | | | |
|---|---|---|---|
| In the column of "Activity", it is indicated that activity is high in order of the mark of "++", "+" and "+-". | | | |

### Example 2: Evaluation of oligo double-stranded RNAs for cell growth by cell counting assay

The screened oligo double-stranded RNAs were introduced to a cancer cell, for which the ability to inhibit cell growth was evaluated by cell counting assay.

### i) Method

On a 96-well plate, A431 cell (epithelial cancer cell) was seeded at 5×10² cells/well, and incubated in 10% FBS containing DMEM medium (Sigma, D6046) for 24 hours. The culture medium was removed from the plate under suction, and 135 µl of 10% FBS-containing DMEM medium was added. There was added 15 µl of the solution containing the complex of oligo double-stranded RNA-liposome A (oligo double-stranded RNA : liposome A = 1 : 16 by weight) mixed in 10% maltose solution, so that the final volume was 150 µl. This was incubated in a CO₂ incubator for 6 days. 15 µl each of Cell Counting Kit-8 solution (DOJINDO) was added and subjected to color reaction in a CO₂ incubator for 2 hours. The absorbance was measured at 450 nm (reference wavelength: 595 nm) by means of a microplate reader. The rate of relative cell number was evaluated as cell viability when the number of A431 cell which has not been transfected with the oligo double-stranded RNA was regarded as 100 %. As a negative control, the cell which was transfected with an oligo double-stranded RNA for firefly luciferase mRNA was used. As a positive control, the cell which was transfected with one of oligo double-stranded RNAs, i.e., B717, inhibiting the expression of Bcl-2 protein, was used. As for the oligo doubie-stranded RNA having an inhibitory activity on the expression of Bcl-2 protein, B043 and B533 were evaluated.

### ii) Results

Fig. 5 shows the results. In both of the oligo double-stranded RNAs, the cell number began to decrease at a concentration of about 1 nM in transfection and further decreased to about 20% at 10 nM. Thus, it was elucidated that the oligo double-stranded RNA of the present invention inhibited the expression of Bcl-2 protein, and as a result they exhibited an effect inhibiting cell growth. Example 3: Effect of one-point mutant oligo double-stranded RNAs

### i) Effect of one-point mutation

In order to elucidate the influence exerted by mutation of oligo double-stranded RNAs, a one-point mutant was prepared and its activity as an oligo double-stranded RNA was evaluated by Western blotting.

As oligo double-stranded RNAs having one-point mutation, the following 3 mutants were prepared. The nucleotide changed by one-point mutation was underlined.
B043-15A:
   sense strand
      5'-GUGAUGAAGUACAUACAUU-dTdT-3' (SEQ ID NO:163)
   anti-sense strand
      5'-AAUGUAUGUACUUCAUCAC-dTdT-3' (SEQ ID NO:164)
B533-18U:
   sense strand
      5'-CUGAGUACCUGAACCGGUA-dTdT-3' (SEQ ID NO:165)
   anti-sense strand
      5'-UACCGGUUCAGGUACUCAG-dTdT-3' (SEQ ID NO:166)
B717-10U:
   sense strand
      5'-GUGAAGUCAUCAUGCCUGC-dTdT-3' (SEQ ID NO:167)
   anti-sense strand
      5'-GCAGGCAUGAUGAGUUCAC-dTdT-3' (SEQ ID NO:168)

In B043-15A, the 15th base pair from the 5'-terminal of B043 sense strand is converted from C-G into A-U; in B533-18U, the 18th base pair, from the 5'-terminal of B533 sense strand, from C-G into U-A; and in B717-10U, the 10th base pair, from A-U into U-A, respectively.

The results of B043-15A are shown in Fig. 3, and those of B533-18U and B717-10U are shown in Fig. 4. In all of the one-point mutant oligo double-stranded RNAs, it was proved that the inhibitory activity on the expression of Bcl-2 protein was retained in some degree, though the activity was somewhat decreased in comparison with that of normal types.

### ii) Effect of the position of one-point mutation

In order to elucidate the influence of the position of mutation of oligo double-stranded RNAs exerted on the activity, 7 types of one-point mutants of the oligo double-stranded RNA in B533 were prepared, in which the position of mutation was different; the inhibitory activity of the oligo double-stranded RNAs for the expression of Bcl-2 protein was evaluated by means of Western blotting as shown in Example 1.

As one-point mutant oligo double-stranded RNAs, those derived from the oligo double-stranded RNA sense strand of B533 were used, in which one-point mutation was located at 2nd, 5th, 8th, 10th, 12th, 15th, and 18th base from the 5'-terminal of the sense strand. Specific examples of one-point mutants are; in B533-2A, the 2nd base pair from the 5'-terminal of the B533 sense strand, i.e., U-A, was converted into A-U; in B533-5U, the 5th base pair from the 5'-terminal of the B533 sense strand, i.e., G-C, was converted into U-A; in B533-8U, the 8th base pair from the 5'-terminal of the B533 sense strand, i.e., C-G, was converted into U-A; in B533-10A, the 10th base pair from the 5'-terminal of the B533 sense strand, i.e., U-A, was converted into A-U; in 8533-12U, the 12th base pair from the 5'-terminal of the B533 sense strand, i.e., A-U, was converted into U-A; in B533-15U, the 15th base pair from the 5'-terminal of the B533 sense strand, i.e., C-G, was converted into U-A; and in B533-18U, the 18th base pair from the 5'-terminal of the B533 sense strand, i.e., C-G, was converted into U-A, respectively.

The B533 oligo double-stranded RNA used and its one-point mutant oligo double-stranded RNAs are as follows.
B533:
   sense strand
      5'-CUGAGUACCUGAACCGGCA-dTdT-3' (SEQ ID NO:43)
   anti-sense strand
      5'-UGCCGGUUCAGGUACUCAG-dTdT-3' (SEQ ID NO:124)
B533-2A:
   sense strand
      5'-CAGAGUACCUGAACCGGCA-dTdT-3' (SEQ ID NO:169)
   anti-sense strand
      5'-UGCCGGUUCAGGUACUCUG-dTdT-3' (SEQ ID NO:170)
B533-5U:
   sense strand
      5'-CUGAUUACCUGAACCGGCA-dTdT-3' (SEQ ID NO:171)
   anti-sense strand
      5'-UGCCGGUUCAGGUAAUCAG-dTdT-3' (SEQ ID NO:172)
B533-8U:
   sense strand
      5'-CUGAGUAUCUGAACCGGCA-dTdT-3' (SEQ ID NO:173)
   anti-sense strand
      5'-UGCCGGUUCAGAUACUCAG-dTdT-3' (SEQ ID NO:174)
B533-10A:
   sense strand
      5'-CUGAGUACCAGAACCGGCA-dTdT-3' (SEQ ID NO:175)
   anti-sense strand
      5'-UGCCGGUUCUGGUACUCAG-dTdT-3' (SEQ ID NO:176)
B533-12U:
   sense strand
      5'-CUGAGUACCUGUACCGGCA-dTdT-3' (SEQ ID NO:177)
   anti-sense strand
      5'-UGCCGGUACAGGUACUCAG-dTdT-3' (SEQ ID NO:178)
B533-15U:
   sense strand
      5'-CUGAGUACCUGAACUGGCA-dTdT-3' (SEQ ID NO:179)
   anti-sense strand
      5'-UGCCAGUUCAGGUACUCAG-dTdT-3' (SEQ ID NO:180)
B533-18U:
   sense strand
      5'-CUGAGUACCUGAACCGGUA-dTdT-3' (SEQ ID NO:165)
   anti-sense strand
      5'-UACCGGUUCAGGUACUCAG-dTdT-3' (SEQ ID NO:166)
The results are shown in Table 2.

The result are shown in Table 2.

### [Table 2]

**Table 2: Effect of the position of one-point mutation on the oligo double-stranded RNA**

| | oligo double-stranded RNA | activity |
|---|---|---|
| 1 | B533 (SEQ ID NO:43 and 124) | ++ |
| 2 | B533-2A (SEQ ID NO:169 and 170) | ++ |
| 3 | B533-5U SEQ ID NO:171 and 172) | ++ |
| 4 | B533-8U (SEQ ID NO:173 and 174) | + |
| 5 | B533-10A (SEQ ID NO: 175 and 176) | + |
| 6 | B533-12U (SEQ ID NO:177 and 178) | + |
| 7 | B533-15U (SEQ ID NO: 179 and 180) | + |
| 8 | B533-18U (SEQ ID NO:165 and 166) | ++ |

In the column of "Activity", the mark of "++" indicates that activity is very high, and the mark of "+" indicates that activity is high.

The inhibitory activity of the oligo double-stranded RNA for the expression of Bcl-2 protein had a tendency to decrease as one-point mutation was located near the center of a double-strand forming portion. However, the lowest active oligo double-stranded RNAs (B533-8U, B553-10A, B533-12U), which had one-point mutation at 8th, 10th and 12th positions from the sense strand of double-strand forming portion, clearly inhibited the expression of Bcl-2 protein when it was introduced to the cell at a concentration of 10 nM. This suggests that one-point mutants are also effective oligo double-stranded RNAs for inhibiting the expression of Bcl-2 protein.

### Example 4: Activity of oligo double-stranded RNAs shorter than 21 bases

The influence of the base number of nucleic acids constituting the oligo double-stranded RNAs and of the base number of an overhang at the 3'-terminal and 5'-terminal exerted on the inhibitory activity on the expression of Bcl-2 protein was examined. Sense strands and anti-sense strands of 16 to 21 bases onvoloving a part of the double-strand forming portion of B533 were employed. These nucleic acids were combined as shown in Table 3 below to provide oligo double-stranded RNAs in which the double-strand forming portions were composed of 16 to 19 base pairs and the overhang of 0 to 3 bases at the 3'-terminal, or the overhang of 1 to 2 bases at the 5'-terminal. The inhibitory activity of these oligo double-stranded RNAs for the expression of Bcl-2 protein was evaluated by Western blotting as described in Example 1.

### [Table 3]

**Table 3: the combination of the sense strand and the anti-sense strand constituting the oligo double-stranded RNA around B533, and the inhibitory activity on the expression of Bcl-2 protein**

| | sense strand | anti-sense strand | the length of double strand (bp) | the base numberof the double-strand forming portion | activity |
|---|---|---|---|---|---|
| 1 | 8533 (SEO ID NO: 43) | B533 (SEQ ID NO: 124) | 19 | 2-2 | ++ |
| 2 | B533 (SEQ ID NO: 43) | B533-20b (SEQ ID NO:183) | 18 | 3-2 | + |
| 3 | B533 (SEO ID NO: 43) | 8534-20b (SEQ ID NO:184) | 19 | 2-1 | ++ |
| 4 | B533-20b (SEQID NO:181) | B533 (SEQ ID NO: 124) | 19 | 1-2 | ++ |
| 5 | B533-20b (SEQ ID NO:181) | B533-20b (SEQ ID NO:183) | 18 | 2-2 | + |
| 6 | B533-20b (SEQ ID NO:181) | B534-20b (SEQ ID NO:184) | 19 | 1-1 | ++ |
| 7 | B534 (SEQ ID NO: 182) | B533 (SEQ ID NO: 124) | 18 | 2-3 | ++ |
| 8 | B534 (SEQ ID NO: 182) | B533-20b (SEQ ID NO:183) | 17 | 3-3 | + |
| 9 | B534 (SEQ ID NO: 182) | B534-20b (SEQ ID NO:184) | 18 | 2-2 | ++ |
| 10 | B535-19b(SEQ ID NO:198) | B535-19b (SEQ ID NO:199) | 17 | 2-2 | ++ |
| 11 | B536-18b (SEQ ID NO:200) | B536-18b (SEQ ID NO:201) | 16 | 2-2 | + |
| 12 | B537-17b (SEQ ID NO:202) | B537-17b (SEQ ID NO:203) | 15 | 2-2 | + |
| 13 | B533bl-19b (SEQ ID NO:212) | B535-19b (SEQ ID NO:199) | 19 | 0-0 | ++ |
| 14 | B534bl-18b (SEQ ID NO:222) | B536-18b (SEQ ID NO:201) | 18 | 0-0 | ++ |
| 15 | B535bl-17b (SEQ ID NO:204) | B537-17b (SEQ ID NO:203) | 17 | 0-0 | + |
| 16 | B531-20b (SEQ ID NO:205) | B534-20b (SEQ ID NO:184) | 19 | (-1)-(-1) | + |
| 17 | B533-OH-2(SEQ ID NO:206) | B533-OH-2 (SEQ ID NO:207) | 19 | (-2)-(-2) | + |

In the column of "the length of double strand ", the number of base pair forming a complementary portion constituted from sense strand and anti-sense strand is indicated. In the column of "the base number of the double-strand forming portion ", "(a base number of a sense strand) - (a base number of an anti-sense strand)" is indicated and "-" indicates the portion of the 5'-terminal end. In the column of "Activity", the mark of "++" indicates that activity is very high, and the mark of "+" indicates that activity is high.

All of the combined oligo double-stranded RNAs as shown in Table 3 showed an inhibitory activity on the expression of Bcl-2 protein. Thus, it was demonstrated that the length of nucleic acids constituting the oligo double-stranded RNAs had no large influence on the inhibitory activity on the expression of Bcl-2 protein. Specifically, it was demonstrated that the double-strand forming portion of oligo double-stranded RNAs, i.e., the complementary portion of the sense strand and anti-sense strand of oligo double-stranded RNA, had no large influence on the activity as far as the length of 15 to 19 base pairs was kept. It was also suggested that there was no large influence on the activity when the base number of the overhang at the 3'-terminal was 0 to 3 and the overhang at the 5'-terminal had 0 to 2 bases.

Further, the overhang at the 3'-terminal of oligo double-stranded RNAs in B533-20b and B534-20b is not dTdT but has a sequence identical with a part of bcl-2 mRNA following the double-strand forming portion and a sequence complementary to a part of bcl-2 mRNA following the double-strand forming portion; this, however, showed a strong inhibitory activity on the expression of Bcl-2 protein (Table 3). Thus, this indicates that the sequence of the overhang at the 3'-terminal may be a sequence identical with a part of bcl-2 mRNA and/or a sequence complementary to a part of bcl-2 mRNA. This also indicates that the activity of oligo double-stranded RNAs are retained even though the sequence of the overhang at the 3'-terminal is in any form including dTdT.

### Example 5: Effect of partially DNA-substituted derivatives

in ribonucleotides constituting oligo double-stranded RNAs, in order to elucidate an influence on the inhibitory activity of the oligo double-stranded RNA for the expression of Bcl-2 protein by replacement of some ribonucleotides with deoxyribonucleotide, the evaluation was performed using a partially DNA-substituted oligo double-stranded RNA.

The nucleic acids constituting oligo double-stranded RNA as used in evaluation were prepared by replacing some of the respective sense and anti-sense strands of B043 and B533 oligo double-stranded RNAs with deoxyribonucleotides as shown below, in addition to the respective sense strand and anti-sense strand constituting the B043 and B533 oligo double-stranded RNAs. The followings indicate the replaced position and sequence of specific deoxyribonucleotides. B043 sense strand line:
(1) B043 sense:
   5'-GUGAUGAAGUACAUCCAUU-dTdT-3' (SEQ ID NO:11)
(2) B043-4nt-D sense:
   Nucleic acid in which the 4 bases at the 3'-terminal of the B043 sense strand were converted into deoxyribonucleotides (U changed to T).
   5'-GUGAUGAAGUACAUCCAdTdT-dTdT-3' (SEQ ID NO:185)
(3) B043-6nt-D sense:
   Nucleic acid in which 6 bases at the 3'-terminal of the B043 sense strand were converted into deoxyribonucleotides (U changed to T).
   5'-GUGAUGAAGUACAUCdCdAdTdT-dTdT-3' (SEQ ID NO:186)
(4) B043-G-dG sense:
   Nucleic acid in which all G bases in the B043 sense strand were converted from ribonucleotide into deoxyribonucleotides.
   5'-dGUdGAUdGAAdGUACAUCCAUU-dTdT-3' (SEQ ID NO:187)
(5) B043-U-dT sense:
   Nucleic acid in which all of the ribonucleotide U bases in the B043 sense strand were converted into deoxyribonucleotide T bases.
   5'-GdTGAdTGAAGdTACAdTCCAdTdT-dTdT-3' (SEQ ID NO:188)
B533 sense strand line:
(1) B533 sense:
   5'-CUGAGUACCUGAACCGGCA-dTdT-3' (SEQ ID NO:43)
(2) B533-4nt-D sense:
   Nucleic acid in which 4 bases at the 3'-terminal of the B533 sense strand were converted into deoxyribonucleotides.
   5'-CUGAGUACCUGAACCGGdCdA-dTdT-3' (SEQ ID NO:189)
(3) B533-6nt-D sense:
   Nucleic acid in which 6 bases at the 3'-terminal of the B533 sense strand were converted into deoxyribonucleotides.
   5'-CUGAGUACCUGAACCdGdGdCdA-dTdT-3' (SEQ ID NO:190)
(4) B533-G-dG sense:
   Nucleic acid in which all G bases in the B533 sense strand were converted from ribonucleotide into deoxyribonucleotides.
   5'-CUdGAdGUACCUdGAACCdGdGCA-dTdT-3' (SEQ ID NO:191)
(5) B533-U-dT sense:
   Nucleic acid in which all of the ribonucleotide U bases in the B533 sense strand were converted into deoxyribonucleotide T bases.
   5'-CdTGAGdTACCdTGAACCGGCA-dTdT-3' (SEQ ID NO:192)
B043 anti-sense strand line:
(1) B043 anti-sense:
   5'-AAUGGAUGUACUUCAUCAC-dTdT-3' (SEQ ID NO:92)
(2) B043-4nt-D anti-sense:
   Nucleic acid in which 4 bases at the 3'-terminal of the B043 anti-sense strand were converted into deoxyribonucleotides.
   5'-AAUGGAUGUACUUCAUCdAdC-dTdT-3' (SEQ ID NO:193)
(3) B043-2nt2nt-D anti-sense:
   Nucleic acid in which the respective 2 bases at the 5'-terminal and 3'-terminal of the B043 anti-sense strand were converted into deoxyribonucleotides.
   5'-dAdAUGGAUGUACUUCAUCAC-dTdT-3' (SEQ ID NO:194)
B533 anti-sense strand line:
(1) B533 anti-sense:
   5'-UGCCGGUUCAGGUACUCAG-dTdT-3' (SEQ ID NO:124)
(2) B533-4nt-D anti-sense:
   Nucleic acid in which 4 bases at the 3'-terminal of the B533 anti-sense strand were converted into deoxyribonucleotides.
   5'-UGCCGGUUCAGGUACUCdAdG-dTdT-3' (SEQ ID NO:193)
(3) B533-2nt2nt-D anti-sense:
   Nucleic acid in which the respective 2 bases at the 5'-terminal and 3'-terminal of the B533 anti-sense strand were converted into deoxyribonucleotides.
   5'-dTdGCCGGUUCAGGUACUCAG-dTdT-3' (SEQ ID NO:196)

A pair of a sense strand selected from the above B043 sense strand line and an anti-sense strand selected from the B043 anti-sense strand line, or a pair of a sense strand selected from the B533 sense strand line and an anti-sense strand selected from the B533 anti-sense strand line were used as an oligo double-stranded RNA for evaluation. Table 4 indicates a combination of the sense strand and the anti-sense strand constituting the oligo double-stranded RNA used. The inhibitory activity of these oligo double-stranded RNAs for the expression of Bcl-2 protein was evaluated by Western blotting as described in Example 1.

### [Table 4]

**Table 4: the combination of the sense strand and the anti-sense strand constituting the partially DNA-substituted oligo double-stranded RNA**

| | sense strand | anti-sense strand |
|---|---|---|
| 1 | B043 (SEQ ID NO:11) | B043 (SEQ ID NO:92) |
| 2 | B043-4nt-D (SEQ ID NO:185) | B043 (SEQ ID NO:92) |
| 3 | B043-6nt-D (SEQ ID NO:186) | B043 (SEQ ID NO:92) |
| 4 | B043-G-dG (SEQ ID NO:187) | B043 (SEQ ID NO:92) |
| 5 | 8043-U-dT (SEQ ID NO:188) | B043 (SEO ID NO:92) |
| 6 | B043 (SEQ ID NO:11) | B043-4nt-D (SEQ ID NO:193) |
| 7 | B043-4nt-D (SEQ ID NO:185) | B043-4nt-D (SEQ ID NO:193) |
| 8 | B043-6nt-D (SEQ ID NO:186) | B043-4nt-D (SEQ ID NO:193) |
| 9 | B043-G-dG (SEQ ID NO:187) | B043-4nt-D (SEQ ID NO:193) |
| 10 | B043-U-dT (SEQ ID NO:188) | B043-4nt-D(SEQ ID NO:193) |
| 11 | B04 3(SEQ ID NO:11) | B043-2nt2nt-D (SEQ ID NO:194) |
| 12 | B043-4nt-D (SEQ ID NO:185) | B043-2nt2nt-D (SEX ID NO:194) |
| 13 | B043-6nt-D (SEQ ID NO:186) | B043-2nt2nt-D (SEQ ID NO:194) |
| 14 | B043-G-dG (SEQ ID NO:187) | B043-2nt2nt-D (SEQ ID NO:194) |
| 15 | B043-U-dT (SEQ ID NO:188) | B043-2nt2nt-D (SEQ ID NO:194) |
| 16 | B533 (SEQ ID NO:43) | B533 (SEQ ID NO:124) |
| 17 | B533-4nt-D (SEQ ID NO:189) | B533 (SEQ ID NO:124) |
| 18 | B533-6nt-D (SEQ ID NO:190) | 8533 (SEQ ID NO:124) |
| 19 | B533-G-dG (SEQ ID NO:191) | B533 (SEQ ID NO:124) |
| 20 | B533-U-dT (SEQ ID NO:192) | B533 (SEQ ID NO:124) |
| 21 | B533 (SEQ ID NO:43) | B533-4nt-D (SEQ ID NO:195) |
| 22 | B533-4nt-D (SEQ ID NO:189) | B533-4nt-D (SEQ ID NO:195) |
| 23 | B533-6nt-D (SEQ ID NO:190) | B533-4nt-D (SEQ ID NO:195) |
| 24 | B533-G-dG (SEQ ID NO:191) | B533-4nt-D (SEQ ID NO:195) |
| 25 | B533-U-dT (SEQ ID NO:192) | B533-4nt-D (SEQ ID NO:195) |
| 26 | B533 (SEQ ID NO:43) | B533-2nt2nt-D (SEQ ID NO:196) |
| 27 | B533-4nt-D (SEQ ID NO:189) | B533-2nt2nt-D (SEQ ID NO:196) |
| 28 | B533-6nt-D (SEQ ID NO:190) | B533-2nt2nt-D (SEQ ID NO:196) |
| 29 | B533-G-dG (SEQ ID NO:191) | B533-2nt2nt-D (SEQ ID NO:196) |
| 30 | B533-U-dT (SEQ ID NO:192) | B533-2nt2nt-D (SEQ ID NO:196) |

All of the oligo double-stranded RNAs in combination as mentioned in Table 4 showed an inhibitory activity on the expression of Bcl-2 protein. This result demonstrates that the presence of deoxyribonucleotides in some parts of nucleic acids constituting the oligo double-stranded RNAs has no influence on the inhibitory activity on the expression of Bcl-2 protein. Thus, it was elucidated that these partially DNA-substituted oligo double-stranded RNAs can be utilized in inhibition of the expression of Bcl-2 protein.

### Example 6: Effect of modification of overhang at 3'-terminal

In the above-mentioned screening, dTdT was used as an overhang. In this screening, the dTdT was converted into a 2'-methoxyethyl derivative, and the influence thereof on the inhibitory activity of the oligo double-stranded RNA for the expression of Bcl-2 protein was examined.

As an oligo double-stranded RNA, B717-MOE in which the overhang at the 3'-terminal of B717 was 2'-methoxyethylated TT was used.

The results are shown in Fig. 6. The density of bands of Bcl-2 protein on the lane of B717-MOE and that on the lane of B717 were approximately the same. This indicates that even though the overhang is converted into 2'-methoxyethyl derivative, the activity remains in the same degree as an oligo double-stranded RNA containing dTdT in the overhang. Thus, it was proved that the activity as an oligo double-stranded RNA was retained even though the nucleotide of the overhang at the 3'-terminal was modified.

### Example 7: Effect of blunt end oligo double-stranded RNAs

The influence of the presence or absence of the overhang at the 3'-terminal constituting oligo double-stranded RNAs on the inhibitory activity of an oligo double-stranded RNA for the expression of Bcl-2 protein was examined. Evaluation was performed by using a blunt end oligo double-stranded RNA comprising 18 base pairs or 19 base pairs with no overhang at the 3'-terminal. Table 5 shows specifically the examined oligo double-stranded RNAs and their results. The inhibitory activity of these oligo double-stranded RNAs for the expression of Bcl-2 protein was evaluated by Western blotting according to the method as described in Example 1.

### [Table 5]

**Table 5: the combination of the sense strand and the anti-sense strand constituting the blunt end oligo double-stranded RNA, and the inhibitory activity on the expression of Bcl-2 protein**

| | sense strand | anti-sense strand | the length of double strand (bp) | the base number of the double-strand forming portion | activity |
|---|---|---|---|---|---|
| 1 | 6043bl-19b (SEQ ID NO:208) | B043bl-19b (SEQ ID NO:224) | 19 | 0-0 | ++ |
| 2 | B436bl-19b (SEQ ID NO:209) | B436bl-19b (SEQ ID NO:225) | 19 | 0-0 | + |
| 3 | B439bl-19b (SEQ ID NO:210) | B439bl-19b (SEQ ID NO:226) | 19 | 0-0 | + |
| 4 | B469bl-19b (SEQ ID NO:211) | 8469bl-19b (SEQ ID NO:227) | 19 | 0-0 | ++ |
| 5 | B533bl-19b (SEQ ID NO:212) | B533bl-19b (SEQ ID NO:228) | 19 | 0-0 | ++ |
| 6 | B614b1-19b (SEQ ID NO:213) | B614bl-19b (SEQ ID NO:229) | 19 | 0-0 | + |
| 7 | B625bl-19b (SEQ ID NO:214) | 6625bl-19b (SEQ ID NO:230) | 19 | 0-0 | + |
| 8 | B631bl-19b (SEQ ID NO:215) | B631bl-19b (SEQ ID NO:231) | 19 | 0-0 | + |
| 9 | B634bl-19b (SEQ ID NO:216) | B634bl-19b (SEQ ID NO:232) | 19 | 0-0 | + |
| 10 | B717bl-19b (SEQ ID NO:217) | B717bl-19b (SEQ ID NO:233) | 19 | 0-0 | + |
| 11 | B043b1-18b (SEQ ID NO:218) | B043bl-18b (SEQ ID NO:234) | 18 | 0-0 | + |
| 12 | B044b1-18b (SEQ ID NO:219) | B044bl-18b (SEQ ID NO:235) | 18 | 0-0 | + |
| 13 | B469bl-18b (SEQ ID NO:220) | B469bl-18b (SEQ ID NO:236) | 18 | 0-0 | + |
| 14 | B470b1-18b (SEQ ID NO:221) | B470bl-18b (SEQ ID NO:237) | 18 | 0-0 | + |
| 25 | B534b1-18b (SEQ ID NO:222) | B534bl-18b (SEQ ID NO:238) | 18 | 0-0 | ++ |
| 26 | B718b1-18b B718bl-18b (SEQ ID NO:223) | B718bl-18b (SEQ ID NO:239) | 18 | 0-0 | + |

In the column of "the length of double strand ", the number of base pair forming a complementary portion constituted from sense strand and anti-sense strand is indicated. In the column of "the base number of the double-strand forming portion ", "(a base number of a sense strand) - (a base number of an anti-sense strand)" is indicated. In the column of "Activity", the mark of "++" indicates that activity is very high, and the mark of "+" indicates that activity is high.

All of the oligo double-stranded RNAs in combination as mentioned in Table 5 showed an inhibitory activity on the expression of Bcl-2 protein. This suggests that the blunt end oligo double-stranded RNAs can also retain approximately the same activity as the overhanging type at the 3'-terminal.

### Example 8: In vivo effect with oligo double-stranded RNAs (1)

A549 cells (10⁶ cells/mouse) were inoculate to the spleen of nude mice (BALB/c, nu/nu, male, 5 weeks of age), and after 10 minutes the spleen was taken out. During 6th to 45th days after inoculation, the complex of oligo double-stranded RNA -liposome A (oligo double-stranded RNA : liposome A = 1 : 16 by weight) containing bcl-2 oligo double-stranded RNA B717 (SEQ ID NO:77 and 158) prepared in the method as shown in Example 1, was administered intravenously in a frequency of once a week (a total of 6 times) or 3 times a week (a total of 18 times). The complex was administered at a dose of 10 mg/kg (body weight) as the oligo double-stranded RNA. To a control group, 10% maltose solution was administered 3 times a week (a total of 18 times). The survival number up to 100 days after inoculation is shown in Fig. 7.

A Kaplan-Meier curve between a respective group to which B717 was administered and a control group was applied to a generalized Wilcoxon's test, indicating that there was a statistically significant difference (P<0.01).

### Example 9: In vivo effect with oligo double-stranded RNAs (2)

A549 cells (10⁶ cells/mouse) were inoculated to the spleen of nude mice (BALB/c, nu/nu, male, 5 weeks of age), and after 10 minutes the spleen was taken out. During 6th to 44th days after inoculation, the complex of oligo double-stranded RNA- liposome A (oligo double-stranded RNA : liposome A = 1 : 16 by weight) containing bcl-2 oligo double-stranded RNA B043 (SEQ ID NO:11 and 92) prepared in the method as shown in Example 1, was administered intravenously in a frequency of twice a week (a total of 12 tinies). The complex was administered at a dose of 10 mg/kg (body weight) as the oligo double-stranded RNA. To a control group, 10% maltose solution was administered twice a week (a total of 12 times). The survival number up to 100 days after transplatation is shown in Fig. 8.

A Kaplan-Meier curve between a group to which B043 was administered and a control group was applied to a generalized Wilcoxon's test, indicating that there was a statistically significant difference (P<0.01).

### Example 10: In vivo effect with oligo double-stranded RNAs (3) - Dose Dependency -

A549 cells (10⁶ cells/mouse) were inoculated to the spleen of nude mice (BALB/c, nu/nu, male, 5 weeks of age), and after 10 minutes the spleen was taken out. During 61h to 44th days after inoculation, the complex of oligo double-stranded RNA- liposome A (oligo double-stranded RNA : liposome A = 1 : 16 by weight) containing bcl-2 oligo double-stranded RNA B043 (SEQ ID NO:11 and 92) prepared in the method as shown in Example 1, was administered intravenously in a frequency of twice a week (a total of 12 times). The complex was administered at a dose of 1 mg/kg (body weight), 3 mg/kg (body weight) and 10 mg/kg (body weight), respectively, as the oligo double-stranded RNA. To a control group, 10% maltose solution was administered twice a week (a total of 12 times). The results are shown in Fig. 9.

A Kaplan-Meier curve among a respective group to which 1 mg/kg (body weight), 3 mg/kg (body weight) or 10 mg/kg (body weight) of B043 was administered and a control group was applied to a generalized Wilcoxon's test, indicating that there was a statistically significant difference (P<0.05, P<0.01, P<0.01, respectively).

### Example 11: In vivo effect with oligo double-stranded RNAs (4) - Investigation of continuous administration schedule -

A549 cells (10⁶ cells/mouse) were inoculated to the spleen of nude mice (BALB/c, nu/nu, male, 5 weeks of age), and after 10 minutes the spleen was taken out. During 5th to 10th days or 5th to 20th days after inoculation, the complex of oligo double-stranded RNA-liposome A (oligo double-stranded RNA : liposome A=1 : 16 by weight) containing bcl-2 oligo double-stranded RNA B043 (SECT ID NO:11 and 92) prepared in the method as shown in Example 1, was administered intravenously in a frequency of 5 times a week (a total of 5 times or 12 times). The complex was administered at a dose of 10 mg/kg (body weight) as the oiigo double-stranded RNA. To a control group, 10% maltose solution was administered 5 times a week (a total of 12 times). The results are shown in Fig. 10.

Sixty-nine days after inoculation of cancer, all of 10 mice survived in both groups to which the complex was administered 5 times and 12 times, respective. A Kaplan-Meier curve between a respective group to which B043 was administered and a control group was applied to a generalized Wilcoxon's test, indicating that there was a statistically significant difference (P<0.01).

### Example 12: Effect of local administration using oligo double-stranded RNAs

To nude mice (BALB/c, nu/nu, male, 5 weeks of age) was inoculated 100 µL of PC-3 cells (2.5×10⁶ cells) suspension in PBS subcutaneously at the right side. During 7th to 18th days after inoculation, the complex of an oligo double-stranded RNA-liposome A (oligo double-stranded RNA : liposome A = 1 : 16 by weight) containing bcl-2 oligo double-stranded RNA B043 (SEQ ID NOs:11 and 92) or B717 (SEQ ID NOs:77 and 158) prepared in the method as shown in Example 1, was administered subcutaneously around cancer in a frequency of 5 times a week (a total of 10 times). The complex was administered at a dose of 0.1 mg/mouse as the oligo double-stranded RNA. To a control group, 10% maltose solution was administered 5 times a week (a total of 10 times), Tumor volume was calculated from a formula: "Volume = minor axis × minor axis × major axis ÷ 2", where the tumor was regarded as oval. The results are shown in Fig. 11.

Increase of the tumor volume, in both groups to which B043 and B717 were administered respectively and in the control group, was statically significantly reduced over 14th to 36th days after cancer inoculation (P<0.01, Dunnett's test).

### Example 13: Evaluation of an inhibitory activity on the expression of Bcl-2 protein in the peripheral sequence of bcl-2 oligo double-stranded RNA obtained by screening

On oligo double-stranded RNAs having sequences around the 4 oligo double-stranded RNAs (B436, B469, B533, B631) which showed a high inhibitory activity on the expression of Bcl-2 protein in the secondary screening and that around B717 (an oligo double-stranded RNA used as a positive control in screening), the inhibitory activities on the expression of Bcl-2 protein were evaluated by Western blotting as shown in Example 1. The oligo double-stranded RNAs having the sequences around B436, B469, B533, B631, or B717, are composed of oligo double-stranded RNAs comprising a double-strand forming portion consisting of a sense RNA strand and its complementary RNA strand, in which the sense strand consisted of 19 bases beginning from the point distant by 1 to 3 bases toward upstream or downstream along the bcl-2 mRNA from the 5'-terminal of the sense strand of the respective original oligo double-stranded RNAs. Specific examples of sequences around B436, B469, B533, B631 and B717 are shown in Table 6 below.

Table 6 shows the results of evaluation of the inhibitory activity on the expression of Bcl-2 protein.

**[Table 6-a]**

| Table 6-a: the sequences around B436 | | | |
|---|---|---|---|
| oligo double-stranded RNA | sense strand / anti-sense strand | sequence (SEQ ID NO:) | activity |
| B434 | sense strand | GGAGGAUUGUGGCCUUCUU-dTdT (SEQ ID NO:240) | ++ |
| | anti-sense strand | AAGAAGGCCACAAUCCUCC-dTdT (SEQ ID NO:257) | |
| B435 | sense strand | GAGGAUUGUGGCCUUCUUU-dTdT (SEQ ID NO:241) | ++ |
| | anti-sense strand | AAAGAAGGCCACAAUCCUC-dTdT (SEQ ID NO:258) | |
| B436 | sense strand | AGGAUUGUGGCCUUCUUUG-dTdT (SEQ ID NO:30) | ++ |
| | anti-sense strand | CAAAGAAGGCCACAAUCCL3-dTdT (SEQ ID NO:111) | |
| B437 | sense strand | GGAUUGUGGCCUUCUUUGA-dTdT (SEQ ID NO:242) | ++ |
| | anti-sense strand | UCAAAGAAGGCCACAAUCC-dTdT (SEQ ID NO:259) | |
| B438 | sense strand | GAUUGUGGCCUUCUUUGAG-dTdT (SEQ ID NO:243) | ++ |
| | anti-sense strand | CUCAAAGAAGGCCACAAUC-dTdT (SEQ ID NO:260) | |

**[Table 6-b]**

| Table 6-b: the sequences around B469 | | | |
|---|---|---|---|
| oligo double-stranded RNA | sense strand / anti-sense strand | sequence (SEQ ID NO:) | activity |
| B467 | sense strand | UCAUGUGUGUGGAGAGCGU-dTdT (SEQ ID NO:244) | ++ |
| | anti-sense strand | ACGCUCUCCACACACAUGA-dTdT (SEQ ID NO:261) | |
| B468 | sense strand | CAUGUGUGUGGAGAGCGUC-dTdT (SEQ ID NC:245) | ++ |
| | anti-sense strand | GACGCUCUCCACACACAUG-dTdT (SEQ ID NO:262) | |
| B469 | sense strand | AUGUGUGUGGAGAGCGUCA-dTdT (SEQ ID NO:36) | ++ |
| | anti-sense strand | UGACGCUCUCCACACACAU-dTdT (SEQ ID NO:117) | |
| B470 | sense strand | UGUGUGUGGAGAGCGUCAA-dTdT (SEQ ID NO:246) | ++ |
| | anti-sense strand | UUGACGCUCUCCACACACA-dTdT (SEQ ID NO:263) | |
| B471 | sense strand | GCGUGUGGAGAGCGUCAAC-dTdT (SEQ ID NO:247) | ++ |
| | anti-sense strand | GUUGACGCUCUCCACACAC-dTdT (SEQ ID NO:264) | |

**[Table 6-c]**

| Table 6-c: the sequences around B533 | | | |
|---|---|---|---|
| oligo double-stranded RNA | sense strand / anti-sense strand | sequence (SEQ ID NO:) | activity |
| B533 | sense strand | CUGAGOACCUGAACCGGCA-dTdT (SEQ ID NO:43) | ++ |
| | anti-sense strand | UGCCGGUUCAGGUACUCAG-dTdT (SEQ ID NO:124) | |
| B535 | sense strand | GAGUACCUGAACCGGCACC-dTdT (SEQ ID NO:248) | ++ |
| | anti-sense strand | GGUGCCGGOUCAGGUACOC-dTdT (SEQ ID NO:265) | |

**[Table 6-d]**

| Table 6-d: the sequences around B631 | | | |
|---|---|---|---|
| oligo double-stranded RNA | sense strand / anti-sense strand | sequence (SEQ ID NO:) | activity |
| B629 | sense strand | UUGAUUUCUCCUGGCUGUC-dTdT (SEQ ID NO:249) | + |
| | anti-sense strand | GACAGCCAGGAGAAAUCAA-dTdT (SEQ ID NO:266) | |
| B630 | sense strand | UGAUUUCUCCUGGCUGUCU-dTdT (SEQ ID NO:250) | + |
| | anti-sense strand | AGACAGCCAGGAGAAAUCA-dTdT (SEQ ID NO:267) | |
| B631 | sense strand | GAUUUCUCCUGGCUGUCUC-dTdT (SEQ ID NO:62) | ++ |
| | anti-sense strand | GAGACAGCCAGGAGAAAUC-dTdT (SEQ ID NO:143) | |
| B632 | sense strand | AUUUCUCCUGGCUGUCUCU-dTdT (SEQ ID NO:251) | ++ |
| | anti-sense strand | AGAGACAGCCAGGAGAAAU-dTdT (SEQ ID NO:268) | |
| B633 | sense strand | UUUCUCCUGGCUGUCUCUG-dTdT (SEQ ID NO:252) | ++ |
| | anti-sense strand | CAGAGACAGCCAGGAGAAA-dTdT (SEQ ID NO:269) | |

**[Table 6-e]**

| Table 6-e: the sequences around B717 | | | |
|---|---|---|---|
| oligo double-stranded RNA | sense strand / anti-sense strand | sequence (SEQ ID NO:) | activity |
| B714 | sense strand | CAAGUGAAGUCAACAUGCC-dTdT (SEQ ID NO:253) | + |
| | anti-sense strand | GGCAUGUUGACUUCACUUG-dTdT (SEQ ID NO:270) | |
| B715 | sense strand | AAGUGAAGUCAACAUGCCU-dTdT (SEQ ID NO:254) | + |
| | anti-sense strand | AGGCAUGUUGACUUCACUU-dTdT (SEQ ID NO:271) | |
| B716 | sense strand | AGUGAAGUCAACAUGCCUG-dTdT (SEQ ID NO:255) | + |
| | anti-sense strand | CAGGCAUGUUGACUUCACU-dTdT (SEQ ID NO:272) | |
| B717 | sense strand | GUGAAGUCAACAUGCCUGC-dTdT (SEQ ID NO:77) | ++ |
| | anti-sense strand | GCAGGCAUGUUGACUUCAC-dTdT (SEQ ID NO:153) | |
| B718 | sense strand | UGAAGUCAACAUGCCUGCC-dTdT (SEQ ID NO:256) | +- |
| | anti-sense strand | GGCAGGCAUGUUGACUUCA-dTdT (SEQ ID NO:273) | |

It was elucidated that the oligo double-stranded RNAs having the above-mentioned peripheral sequences showed an inhibitory activity on the expression of Bcl-2 protein.

### Example 14: Evaluation of other oligo double-stranded RNAs regarding the inhibitory activity on the expression of Bcl-2 protein

The inhibitory activity of oligo double-stranded RNAs (B521, B557, B584, B635, B734. B735, B736), of which the sequences are specifically shown in Table 7 below, for the expression of Bcl-2 protein was evaluated by Western blotting as shown in Example 1. Table 7 indicates the results.

**[Table 7]**

| Table 7 | | | |
|---|---|---|---|
| oligo double-stranded RNA | sense strand / anti- | sequence (SEQ ID NO:) | activity |
| B521 | sense strand | CCCUGUGGAUGACUGAGUA-dTdT (SEQ ID NO:274) | ++ |
| | anti-sense strand | UACUCAGUCAUCCACAGGG-dTdT SEQ ID NO:291) | |
| B557 | sense strand | ACACCUGGAUCCAGGAUAA-dTdT (SEQ ID NO:275) | ++ |
| | anti-sense strand | UUAUCCUGGAUCCAGGUGU-dTdT (SEQ ID NO:282) | |
| B5B4 | sense strand | GGGAUGCCUUUGUGGAACU-dTdT (SEQ ID NO:276) | +- |
| | anti-sense strand | AGUUCCACAAAGGCAUCCC-dTdT (SEQ ID NO:283) | |
| B635 | sense strand | UCUCCUGGCUGUCUCUGAA-dTdT (SEQ ID NO:277) | ++ |
| | anti-sense strand | UUCAGAGACAGCCAGGAGA-dTdT (SEQ ID NO:284) | |
| B734 | sense strand | GCCCCAAACAAAUAUGCAA-dTdT (SEQ ID NO:278) | +- |
| | anti-sense strand | UUGCAUAUUUGUUUGGGC-dTdT (SEQ ID NO:285) | |
| B735 | sense strand | CCCCAAACAAAUAUGCAAA-dTdT (SEQ ID NO:279) | +- |
| | anti-sense strand | UUUGCAUAUUUGUUUGGGG-dTdT (SEQ ID NO:286) | |
| B736 | sense strand | CCCAAACAAAUAUGCAAAA-dTdT (SEQ ID NO:230) | +- |
| | anti-sense strand | UUUUGCAUAUUUGUUUGGG-dTdT (SEQ ID NO:287) | |

### Example 15: Evaluation of oligo double-stranded RNAs on the inhibitory activity on the expression of Bcl-2 protein (1)

### i) Preparation of oligo double-stranded RNAs

Nucleic acid constituting an oligo double-stranded RNA was synthesized by a standard solid phase phosphoramidite method using an automatic nucleic acid synthesizer. The synthesis was relied on Dharmacon Co, (Colorado, USA) or Japan Bioservice (Saitama Pref., Japan), or achieved by the present inventors.

Briefly, the present inventors performed the synthesis according to the following procedure. Using an automatic DNA synthesizer (Applied Biosystems, Expedite 8909), monomers were condensed one by one by a standard phosphoramidite method to form a desired sequence. Using a concentrated ammonium hydroxide-ethanol (3:1) mixture, the nucleotide chain was cleaved from CPG (controlled pore glass) and deprotected in the same solution kept at 55°C for 18 hours.

The mixture was then treated with 1M tetrabutylammonium fluoride in tetrahydrofuran solution for 20 hours to remove the 2'-silyl group by deprotection. The resulting oligo-ribonucleotide was purified by reverse-phase chromatography. The product was further treated with 80% acetic acid solution at room temperature for 30 minutes to remove the 5'-DMTr group for deprotection, followed by re-purification by ion-exchange chromatography. After desalination, the resulting oligonucleotide was proved to be the objective full length oligonucleotide in 90% or more purity by means of capillary gel electrophoresis.

Thus, a variety of nucleic acids constituting oligo double-stranded RNAs were synthesized. Oligo double-stranded RNAs were prepared by mixing two nucleic acids of which the double-strand forming portion were complementary, in an equimolar amount as mentioned below.

### ii) Method for evaluation of the inhibitory activity on the expression of Bcl-2 protein

An inhibitory activity on the expression of Bcl-2 protein was evaluated by introducing together with a carrier an oligo double-stranded RNA into a various type of cancer cells and determining the amount of expressed protein by Western blotting.

### Preparation of a complex of an oligo double-stranded RNA and a carrier

Using liposome A comprising 2-O-(2-diethylaminoethyl)carbamoyl 1,3-O-dioleoylglycerol and purified egg yolk lecithin as a carrier, a complex with an oligo double-stranded RNA was prepared. One part by weight of oligo double-stranded RNA was mixed with 16 parts by weight of liposome A to give a complex. The followings indicate the preparation of 2 ml of complex solution having a final concentration of 10 µM oligo double-stranded RNA. The concentration of the oligo double-stranded RNA indicates the molar concentration of oligo double-stranded RNA contained in the complex assuming that the oligo double-stranded RNA forms a double-stand completely.

A sense strand and an anti-sense strand were respectively dissolved in water for injection so as to be 300 µM, each of which 66.6 µl was mixed in a test tube. One ml of solution containing each strand in a concentration of 20 µM was prepared by adding 866.8 µl of 10% maltose solution to this mixture. This was used as an oligo double-stranded RNA solution. One ml of a liposome A-dispersed solution at 4.3 mg/ml was prepared by adding 732 µl of 10% maltose solution to 268 µl of the liposome A-dispersed solution of 16 mg/ml. One ml of the above oligo double-stranded RNA-containing solution was slowly added to 1 ml of the liposome A-dispersed solution with stirring. By the above procedure, a solution containing a complex of liposome A with an oligo double-stranded RNA in which the final concentration of oligo double-stranded RNA was 10 µM was prepared. The particles of this complex were homogenized by dispersing with a 600 W bath-type ultra-sonicator for 2 minutes.

### Western Blotting

Using the above-mentioned complex of liposome A with oligo double-stranded RNA, it was evaluated whether the expression of Bcl-2 protein was inhibited by transfection of the cells with the oligo double-stranded RNA, by evaluating change of the amount of Bcl-2 protein by Western blotting.

On a Petri dish of 6 cm in diameter, A431 cell (epithelial cancer cell), A375 cell (melanoma cell), MDA-MB-231 cell (breast cancer), or A549 cell (lung cancer) was seeded at 2×10⁵ cells/dish, and incubated in a DMEM medium (Sigma, D6046) containing 10% FBS (fetal bovine serum) overnight at 37°C under 5% CO₂. Next day, the culture medium was removed from the dish under suction, and 2.7 ml of 10% FBS-DMEM medium (Sigma, D6046) was added for substitution of the medium. There was added 0.3 ml of the complex-containing solution of oligo double-stranded RNA-liposome A (oligo double-stranded RNA : liposome A = 1 : 16 by weight) mixed in 10% maltose solution, so that the final volume was 3 ml. At this point, the final concentration of oligo doubie-stranded RNA is 3 nM, 10 nM or 100 nM. This was incubated at 37°C in a 5% CO₂ incubator for 72 hours. The cells were washed twice with PBS (phosphate buffered saline) and moved with a cell scraper into a 1.5 ml tube. After centrifugation at 1000×g for 2 minutes and removal of the supernatant, the cells were dissolved in 20-100 µl of lysis buffer (50 mM Tris-HCI, pH 7.5, 150 mM NaCl, 1% NP-40). The mixture was allowed to stand on ice for 30 minutes, centrifuged at 100,000xg for 15 minutes, and the supernatant was moved into a fresh tube and kept as a sample for electrophoresis.

Electrophoresis was carried out on polyacrylamide gel (ATTO NPG-520L), on which a sample was applied at 15 µg of total protein for one lane. After termination of electrophoresis, the protein in the gel was transferred on a polyvinylidene fluoride (PVDF) membrane, and blocked in 5% skim milk-containing PBST (PBST-MLK; herein, the composition of PBST was 0.1% Tween 20-containing PBS) at room temperature for 1 hour. First, Bcl-2 protein was detected. The PVDF membrane after blocking was soaked in a mouse anti-human Bcl-2 monoclonal antibody (DAKO M0887) diluted 500 times with PBST-MLK and shaken overnight at 4°C for binding with a primary antibody. The PVDF membrane was washed with PBST, and shaken with HRP (horse radish peroxidase) labeled anti-mouse Ig antibody (DAKO P0260) diluted 2000 times with PBST-MLK at room temperature for 2 hours to bind a secondary antibody. After washing with PBST, light was emitted from the membrane with Western Lightening Chemiluminescence Reagent Plus (Perkin Elmer) and the amount of Bcl-2 protein was detected by ChemiDoc (BioRad).

After detection of Bcl-2 protein, the same PVDF membrane was washed with distilled water, and Actin protein was detected in the same manner as in Bcl-2. Goat anti-human Actin antibody (Santa Cruz SC-1616) was used as a primary antibody, and HRP-labeled anti-goat Ig antibody (DAKO P0449) was used as a secondary antibody. The antibodies were diluted 500 times and 1500 times, respectively, with PBST-MLK.

Comparison with them was determined visually by using the density of band of Bcl-2 protein as a negative control, where transfection with an oligo double-stranded RNA (GL3) inhibiting the expression of luciferase was made. When the double-strand forming portion was an oligo double-stranded RNA of 25 or more base pairs, the case where the density was much lighter than that of the negative control in a treating concentration of 10 nM was designated as '++"; slightly lighter, "+"; and the case where the density in a treating concentration of 100 nM was slightly lighter than that of the negative control was designated as "+/-". Evaluation of an inhibitory activity on the expression of Bcl-2 protein of an oligo double-stranded RNA in which the complementary portion is 25 base pairs

The oligo double-stranded RNAs used in evaluation of an inhibitory activity on the expression of Bcl-2 protein were provided as the following oligo double-stranded RNAs based on the sequence (hereinafter, referred to as bcl-2 mRNA; SEQ ID NO.197) of GenBank Accession NO. BC027258.

In order to evaluate whether an oligo double-stranded RNA having a double-strand forming portion of 25 base pairs had a strong inhibitory activity on the expression of Bcl-2 protein, the present inventors prepared the B043-25 anti-sense strand and the B043-25 sense strand which was complementary to the B043-25 anti-sense strand, and prepared the oligo double-stranded RNA of B043-25. In addition, the anti-sense strands of B042-25, B041-25, B040-25, B039-25, B038-25 and B037-25, which had a sequence of 25 bases shifted by 1 to 6 bases toward the 3'-terminal along the sequence complementary to bcl-2 mRNA from the sequence of 25 bases of the B043-25 anti-sense strand with the exclusion of 2 dT bases from the 3'-terminal and had 2 additional dT bases to the shifted 3'-terminal; and the sense strands of B042-25, B041-25, B040-25, B039-25, B038-25 and B037-25, which had a sequence of 25 bases shifted by 1 to 6 bases toward the 5'-terminal along the sequence of bcl-2 mRNA from the sequence of 25 bases of the B043-25 sense strand with the exclusion of 2 dT bases from the 3'-terminal and had 2 additional dT bases to the shifted 3'-terminal were prepared, and the respective oligo double-stranded RNAs were prepared.

In this situation, the oligo double-stranded RNAs used in evaluation of the inhibitory activity on the expression of Bcl-2 protein were represented by "BXXX-25", wherein "XXX" was indicated the position number from the initiation point of translation in bcl-2 mRNA of the 5'-teminal base of sense strand in the oiigo double-stranded RNA. The inhibitory activity on the expression of Bcl-2 protein of the above 7 oligo double-stranded RNAs (B037-25, B038-25, B039-25, B040-25, B041-25, B042-35 and B043-25) were evaluated by Western blotting.

The results are shown in Table 8

**[Table 8]**

| Table 8: the inhibitory activity on the expression of Bcl-2 protein of the oligo double-stranded RNA in which the complementary portion is 25 base pairs | | | |
|---|---|---|---|
| oligo double-stranded RNA | sense strand / anti-sense strand | sequence (SEQ ID NO:) | activity |
| B037-25 | sense strand | GAG AUA GUG AUG AAG UAC AUC CAU U -dTdT (SEQ ID NO:288) | ++ |
| | anti-sense strand | AAU GGA UGU ACU UCA UCA CUA UCU C -dTdT (SEQ ID NO:319) | |
| B038-25 | sense strand | AGA UAG UGA UGA AGU ACA UCC AUU A -dTdT (SEQ ID NO:289) | ++ |
| | anti-sense strand | UAA UGG AUG UAC UUC AUC ACU AUC U -dTdT (SEQ ID NO:320) | |
| B039-25 | sense strand | GAU AGU GAU GAA GUA CAU CCA UUA U -dTdT(SEQ ID NO:290) | ++ |
| | anti-sense strand | AUA AUG GAU GUA CUU CAU CAC UAU C -dTdT (SEQ ID NO:321) | |
| B040-25 | sense strand | AUA GUG AUG AAG UAC AUC CAU UAU A -dTdT(SEQ ID NO:291) | ++ |
| | anti-sense strand | UAU AAU GGA UGU ACU UCA UCA CUA U -dTdT (SEQ ID NO:322) | |
| B041-25 | sense strand | UAG UGA UGA AGU ACA UCC AUU AUA A -dTdT(SEQ ID NO:292) | ++ |
| | anti-sense strand | UUA UAA UGG AUG UAC UUC AUC ACU A -dTdT (SEQ ID NO:323) | |
| B042-25 | sense strand | AGU GAU GAA GUA CAU CCA UUA UAA G -dTdT(SEQ ID NO:293) | ++ |
| | anti-sense strand | CUU AUA AUG GAU GUA CUU CAU CAC U -dTdT (SEQ ID NO:324) | |
| B043-25 | sense strand | GUG AUG AAG UAC AUC CAU UAU AAG C -dTdT(SEQ ID NO:294) | ++ |
| | anti-sense strand | GCU UAU AAU GGA UGU ACU UCA UCA C -dTdT (SEQ ID NO:325) | |

It was elucidated that Bcl-2 protein was almost completely inhibited by the oligo nucleic acid RNAs as shown in Table 8 at 100 nM of transfection, and further the expression of Bcl-2 protein was almost completely inhibited even in transfection at 10 nM (Figs. 12 and 13).

Thus, in addition to B043-25 oligo double-stranded RNA, it was demonstrated that the oligo double-stranded RNAs consisting of an anti-sense strand, which had a sequence of 25 bases shifted by 1 to 6 bases toward the 3'-terminal along the sequence complementary to bcl-2 mRNA from the sequence of 25 bases of the B043-25 anti-sense strand with the exclusion of 2 dT bases from the 3'-terminal and had 2 additional dT bases to the shifted 3'-terminal: and a sense strand complementary to the sequence of 25 bases of the shifted anti-sense strand with the exclusion of 2 dT bases from the 3'-terminal and had 2 additional dT bases to the shifted 3'-terminal, showed an inhibitory activity on the expression of Bcl-2 protein.

### Example 16: Evaluation of oligo double-stranded RNAs for the inhibitory activity on the expression of Bcl-2 protein (2)

The present inventors investigated that the effect of an oligo double-stranded RNA, in which the other complementary strand was composed of 25 base pairs, exerted on an inhibitory activity on the expression of Bcl-2 protein. The oligo double-stranded RNAs specifically investigated include those of B430-25, 8463-25, B527-25, B608-25, B625-25 and B711-25. The inhibitory activity of these oligo double-stranded RNAs for the expression of Bcl-2 protein was evaluated by Western blotting in the method as described in Example 15.

The results are shown in Table 9.

**[Table 9]**

| Table 9: the inhibitory activity on the expression of Bcl-2 protein of the oligo double-stranded RNA in which the complementary portion is 25 base pairs | | | |
|---|---|---|---|
| oligo double-stranded RNA | sense strand / anti-sense strand | sequence (SEQ ID NO:) | activity |
| B430-25 | sense strand | UGG GGG AGG AUU GUG GCC UUC UUU G -dTdT (SEQ ID NO:295) | + |
| | anti-sense strand | CAA AGA AGG CCA CAA UCC UCC CCC A -dTdT (SEQ ID NO:326) | |
| B463-25 | sense strand | GGG GUC AUG UGU GUG GAG AGC GUC A -dTdT (SEQ ID NO:296) | ++ |
| | anti-sense strand | UGA CGC UCU CCA CAC ACA UGA CCC C -dTdT (SEQ ID NO:327) | |
| B527-25 | sense strand | GGA UGA CUG AGU ACC UGA ACC GGC A -dTdT (SEQ ID NO:297) | ++ |
| | anti-sense strand | UGC CGG UUC AGG UAC UCA GUC AUC C -dTdT (SEQ ID NO:328) | |
| B608-25 | sense strand | GCC CCA GCA UGC GGC CUC UGU UUG A -dTdT (SEQ ID NO:298) | ++ |
| | anti-sense strand | UCA AAC AGA GGC CGC AUG CUG GGG C -dTdT (SEQ ID NO:329) | |
| B625-25 | sense strand | CUG UUU GAU UUC UCC UGG CUG UCU C -dTdT (SEQ ID NO:299) | ++ |
| | anti-sense strand | GAG ACA GCC AGG AGA AAU CAA ACA G -dTdT (SEQ ID NO:330) | |
| B711-25 | sense strand | CCA CAA GUG AAG UCA ACA UGC CUG C -dTdT (SEQ ID NO:300) | ++ |
| | anti-sense strand | GCA GGC AUG UUG ACU UCA CUU GUG G -dTdT (SEQ ID NO:331) | |

It was elucidated that Bcl-2 protein was almost completely inhibited by the oligo double-stranded RNAs as shown in Table 9 when transfected at 100 nM, and even in transfection at 10 nM, further the expression of Bcl-2 protein was almost completely inhibited (Fig. 13).

Thus, the oligo double-stranded RNAs of which the complementary portion was composed of 25 base pairs showed a strong inhibitory activity on the expression of Bci-2 protein.

### Example 17: Effect of the overhang at the 3'-terminal in oligo double-stranded RNAs in which the complementary portion is composed of 25 base pairs

An oligo double-stranded RNA in which the overhang at the 3'-terminal was deoxyribonucleotides dTdT and capable of forming a complementary strand with bcl-2 mRNAfollowing the double-strand forming portion, was investigated on whether there was an influence on an inhibitory activity on the expression of Bcl-2 protein.

Nucleic acids constituting oligo double-stranded RNAs used in evaluation, in addition to the sense and anti-sense strands constituting B037-25 oligo double-stranded RNA, were obtained by preparing nucleic acids having as an overhang a sequence capable of forming a complementary strand with bcl-2 mRNA following the double-strand forming portion instead of the 3'-terminal dTdT of the sense and anti-sense strands of B037-25 oligo double-stranded RNA. The followings are specific examples of the sequences.
B037-25 sense strand line:
(1) B037-25 sense:
   5'-GAGAUAGUGAUGAAGUACAUCCAUU-dTdT-3' (SEQ ID NO:288)
(2) B037SSOH25 sense:
   5'-GAGAUAGUGAUGAAGUACAUCCAUUAU-3' (SEQ ID NO:301)
B037-25 anti-sense strand line:
(1) B037-25 anti-sense:
   5'-AAUGGAUGUACUUCAUCACUAUCUC-dTdT-3'(SEQ ID NO:319)
(2) B037SSOH25 anti-sense:
   5°-AAUGGAUGUACUUCACUUACUAUCUCCC-3` (SEQ ID NO:332)

Specifically, the examined oligo double-stranded RNAs and the results are shown in Table 10. The inhibitory activity of these oligo double-stranded RNAs for the expression of Bcl-2 protein was evaluated by Western blotting as described in Example 15.

**[Table 10]**

| Table 10: influence of 3'-terminal overhang of oligo double-stranded RNA to the inhibitory activity on the expression of Bcl-2 protein | | | |
|---|---|---|---|
| oligo double-stranded RNA | sense strand / anti-sense strand | sequence (SEQ ID NO:) | activity |
| B037-25 | sense strand | GAG AUA GUG AUG AAG UAC AUC CAU U -dTdT (SEQ ID NO:288) | ++ |
| | anti-sense strand | AAU GGA UGU ACU UCA UCA CUA UCU C -dTdT (SEQ ID NO:319) | |
| B037SSOH25 | sense strand anti-sense strand | GAG AUA GUG AUG AAG UAC AUC CAU UAU (SEQ ID NO:301) | ++ |
| | | AAU GGA UGU ACU UCA UCA CUA UCU CCC (SEQ ID NO:332) | |

It was elucidated that Bcl-2 protein was almost completely inhibited by the oligo nucleic acid RNAs as shown in Table 10 when transfected at 100 nM, and even in transfection at 10 nM, further the expression of Bcl-2 protein was almost completely inhibited (Fig. 14).

These results indicate that the 3'-overhang which is a sequence capable of forming a complementary strand with bcl-2 mRNA following the double-strand forming portion of the nucleic acid constituting the oligo double-stranded RNA has no large influence on the inhibitory activity on the expression of Bcl-2 protein. Thus, it was elucidated that these oligo double-stranded RNAs could be utilized in inhibiting the expression of Bcl-2 protein. This indicates that the activity of the oligo double-stranded RNAs is retained even though the 3'-overhang has any type of sequences other than dTdT.

### Example 18: Effect of blunt end oligo double-stranded RNAs in which the complementary strand portion comprises 25 base pairs

The influence of the presence or absence of the 3'-terminal overhang of a nucleic acid constituting an oligo double-stranded RNA exerted on the inhibitory activity on the expression of Bcl-2 protein by the oligo double-stranded RNA, was investigated. Blunt end oligo double-stranded RNAs of 25 base pairs with the elimination of the 3'-overhang were used for evaluation. Specifically, the examined oligo double-stranded RNAs and their results were shown in Table 11. The inhibitory activity of these oligo double-stranded RNAs for the expression of Bcl-2 protein was evaluated by Western blotting as described in Example 15.

**[Table 11]**

| Table 11: the inhibitory activity on the expression of Bcl-2 protein of the blunt end oligo double-stranded RNA | | | |
|---|---|---|---|
| oligo double-stranded RNA | sense strand / anti-sense strand | sequence (SEQ ID NO:) | activity |
| B037-25 | sense strand | GAG AUA GUG AUG AAG UAC AUC CAU U -dTdT (SEQ ID NO:288) | ++ |
| | anti-sense strand | AAU GGA UGU ACU UCA UCA CUA UCU C -dTdT (SEQ ID NO:319) | |
| B037b125 | sense strand | GAG AUA GUG AUG AAG UAC AUC CAU U (SEQ ID NO:302) | ++ |
| | anti-sense strand | AAU GGA UGU ACU UCA UCA CUA UCU C (SEQ ID NO:333) | |
| B035b127 | sense strand | GGG AGA UAG UGA UGA AGU ACA UCC AUU (SEQ ID NO:303) | ++ |
| | anti-sense strand anti-sense strand | AAU GGA UGU ACU UCA UCA CUA UCU CCC (SEQ ID NO:332) | |
| B039b127 | sense strand | GAU AGU GAU GAA GUA CAU CCA UUA UAA (SEQ ID NO:304) | ++ |
| | anti-sense stand anti-sense strand | UUA UAA UGG AUG UAC UUC AUC ACU AUC (SEQ ID NO:334) | |

It was elucidated that Bcl-2 protein was almost completely inhibited by the oligo nucleic acid RNAs as shown in Table 11 when transfected at 100 nM, and even in transfection at 10 nM, further the expression of Bcl-2 protein was almost completely inhibited (Fig. 15).

From these results, it was suggested that all of the blunt end oligo double-stranded RNAs showed an inhibitory activity on the expression of Bcl-2 protein, and approximately the same activity as that of the 3'-terminal overhang type was retained even in the blunt end-type oligo double-stranded RNAs.

### Example 19: Effect of sense stranded DNA derivatives

It was evaluated whether replacing all of the ribonucleotides of the sense strand constituting an oligo double-stranded RNA with deoxyribonucleotides affected the inhibitory activity of the oligo double-stranded RNA for the expression of Bcl-2 protein by using an oligo double-stranded RNA in which all the sense strands were DNA.

The nucleic acids constituting oligo double-stranded RNAs used in evaluation were prepared by replacing the respective sense strands constituting the oligo doubie-stranded RNAs B037-25, B038-25, B039-25, B040-25, B041-25, B042-25 and B043-25 with deoxyribonucleotides as shown below.

The followings are specific examples of the sequences.
B037-25 sense strand line:
(1) B037-25 sense:
   5'-GAGAUAGUGAUGAAGUACAUCCAUU-dTdT-3' (SEQ ID NO:288)
(2) B037DNA25 sense:
   Nucleic acid in which all bases of the B037 sense strand were converted into deoxyribonucleotides (U changed to T).
   5'-dGdAdGdAdTdAdGdTdGdAdTdGdAdAdGdTdAdCdAdTdCdCdAdTdT-dTdT-3' (SEQ ID NO:311)
B038-25 sense strand line:
(1) B038-25 sense:
   5'-AGAUAGUGAUGAAGUACAUCCAUUA-dTdT-3' (SEQ ID NO:289)
(2) B038DNA25 sense:
   Nucleic acid in which all bases of the B038 sense strand were converted into deoxyribonucleotides (U change to T).
   5'-dAdGdAdTdAdGdTdGdAdTdGdAdAdGdTdAdCdAdTdCdCdAdTdTdA-dTdT-3' (SEQ ID NO:312)
B039-25 sense strand line:
(1) B039-25 sense:
   5'-GAUAGUGAUGAAGUACAUCCAUUAU-dTdT-3' (SEQ ID NO:290)
(2) B039DNA25 sense:
   Nucleic acid in which all bases of the B039 sense strand were converted into deoxyribonucleotides (U changed to T).
   5'-dGdAdTdAdGdTdGdAdTdGdAdAdGdTdAdCdAdTdCdCdAdTdTdAdT-dTdT-3' (SEQ ID NO:313)
B040-25 sense strand line:
(1) B040-25 sense:
   5'-AUAGUGAUGAAGUACAUCCAUUAUA-dTdT-3' (SEQ ID NO:291)
(2) B040DNA25 sense:
   Nucleic acid in which all bases of the B040 sense strand were converted into deoxyribonucleotides (U changed to T).
   5'-dAdTdAdGdTdGdAdTdGdAdAdGdTdAdCdAdTdCdCdAdTdTdAdTdA-dTdT-3' (SEQ ID NO:314)
B041-25 sense strand line:
(1) B041-25 sense:
   5'-UAGUGAUGAAGUACAUCCAUUAUAA-dTdT-3' (SEQ ID NO:292)
(2) B041 DNA25 sense:
   Nucleic acid in which all bases of the B041 sense strand were converted into deoxyribonucleotides (U changed to T).
   5'-dTdAdGdTdGdTdGdAdTdGdAdAdGdAdTdCdCdAdTdTdAdTdAdA-dTdT-3' (SEQ ID NO:315)
B042-25 sense strand line:
(1) B042-25 sense:
   5'-AGUGAUGAAGUACAUCCAUUAUAAG-dTdT-3' (SEQ ID NO:293)
(2) B042DNA25 sense:
   Nucleic acid in which all bases of the B042 sense strand were converted into deoxyribonucleotides (U changed to T).
   5'-dAdGdTdGdAdTdGdAdAdGdTdAdCdAdTdCdCdAdTdTdAdTdAdAdG-dTdT-3' (SEQ ID NO:316)
B043-25 sense strand line;
(1) B043-25 sense:
   5'-GUGAUGAAGUACAUCCAUUAUAAGC-dTdT-3' (SEQ ID NO:294)
(2) B043DNA25 sense:
   Nucleic acid in which all bases of the B043 sense strand were converted into deoxyribonucleotides (U changed to T).
   5'-dGdTdGdAdTdGdAdAdGdTdAdCdAdTdCdCdAdTdTdAdTdAdAdGdC-dTdT-3' (SEQ ID NO:317)

Specifically, the examined oligo double-stranded RNAs and the results are shown in Table 12. The inhibitory activity of these oligo double-stranded RNAs for the expression of Bcl-2 protein was evaluated by Western blotting as described in Example 15.

**[Table 12]**

| Table 12: the inhibitory activity on the expression of Bcl-2 protein of the oligo double-stranded RNA, wherein all of the ribonucleotides of the sense strand are replaced with deoxyribonucleotides | | | |
|---|---|---|---|
| oligo double-stranded RNA | sense strand / anti-sense strand | sequence (SEQ ID NO:) | activity |
| B037-25 | sense strand | GAG AUA GUG AUG AAG UAC AUC CAU UdTdT (SEQ ID NO:288) | ++ |
| | anti-sense strand | AAU GGA UGU ACU UCA UCA CUA UCU CdTdT (SEQ ID NO:319) | |
| B037DNA25 | sense strand | dGdAdG dAdTdA dGdTdG dAdTdG dAdAdG dTdAdC dAdTdC dCdAdT dTdTdT (SEQ ID NO:311) | +- |
| | anti-sense strand | AAU GGA UGU ACU UCA UCA CUA UCU CdTdT (SEQ ID NO:319) | |
| B038DNA25 | sense strand | dAdGdA dTdAdG dTdGdA dTdGdA dAdGdT dAdCdA dTdCdC dAdTdT dAdTdT (SEQ ID NO:312) | +- |
| | anti-sense strand | UAA UGG AUG UAC UUC AUC ACU AUC UdTdT (SEQ ID NO:320) | |
| B039DNA25 | sense strand | dGdAdT dAdGdT dGdAdT dGdAdA dGdTdA dCdAdT dCdCdA dTdTdA dTdTdT (SEQ ID NO:313) | +- |
| | anti-sense strand | AUA AUG GAU GUA CUU CAU CAC UAU CdTdT (SEQ ID NO:321) | |
| B040DNA25 | sense strand | dAdTdA dGdTdG dAdTdG dAdAdG dTdAdC dAdTdC dCdAdT dTdAdT dAdTdT (SEQ ID NO:314) | +- |
| | anti-sense strand | UAU AAU GGA UGU ACU UCA UCA CUA UdTdT (SEQ ID NO:322) | |
| B041DNA25 | sense strand | dTdAdG dTdGdA dTdGdA dAdGdT dAdCdA dTdCdC dAdTdT dAdTdA dAdTdT (SEQ ID NO:315) | +- |
| | anti-sense strand | UUA UAA UGG AUG UAC UUC AUC ACU AdTdT (SEO ID NO:323) | |
| B042DNA25 | sense strand | dAdGdT dGdAdT dGdAdA dGdTdA dCdAdT dCdCdA dTdTdA dTdAdA dGdTdT (SEQ ID NO:316) | +- |
| | anti-sense strand | CUU AUA AUG GAU GUA CUU CAU CAC UdTdT (SEQ ID NO:324) | |
| B043DNA25 | sense strand | dGdTdG dAdTdG dAdAdG dTdAdC dAdTdC dCdAdT dTdAdT dAdAdG dCdTdT (SEQ ID NO:317) | +- |
| | anti-sense strand | GCU UAU AAU GGA UGU ACU UCA UCA CdTdT (SEQ ID NO:325) | |

It was elucidated that Bcl-2 protein was slightly inhibited by the oligo nucleic acid RNAs as shown in Table 12 when transfected at 100 nM, and even in transfection at 10 nM, further the expression of Bcl-2 protein was slightly inhibited (Fig. 16).

On the basis of these results, it was confirmed that the oligo double-stranded RNAs of which their constituting sense strand was entirely replaced with deoxyribonucleotides also showed an inhibitory activity on the expression of Bcl-2 protein. Thus, it was found that the oligo double-stranded RNAs of which their constituting sense strand was entirely replaced with deoxyribonucleotides could also be utilized in inhibiting the expression of Bcl-2 protein.

### Example 20: Effect of the partially DNA-substituted sense strands

It was evaluated whether partial substitution of the ribonucleotides in the sense strand of oligo double-stranded RNA with deoxyribonucleotides affected the inhibitory activity of an oligo double-stranded RNA for the expression of Bcl-2 protein by using partially DNA-substituted RNAs.

The nucleic acids constituting oligo double-stranded RNAs used in evaluation were prepared by replacing the respective sense strands constituting B037-25 oligo double-stranded RNAs with deoxyribonucleotides as shown below. The followings are specific examples of the sequences.
B037-25 sense strand line:
(1) B037-25 sense:
   5'-GAGAUAGUGAUGAAGUACAUCCAUU-dTdT-3' (SEQ ID NO:288)
(2) B037DNA25 sense:
   Nucleic acid in which all bases of the B037 sense strand were converted into deoxyribonucleotides (U change to T).
   5'-dGdAdGdAdTdAdGdTdGdAdTdGdAdAdGdTdAdCdAdTdCdCdAdTdT-dTdT-3' (SEQ ID NO:311)
(3) B037-25-dC-S sense:
   Nucleic acid in which all ribonucleotide C bases of the B037 sense strand were converted into deoxyribonucleotides.
   5'-GAGAUAGUGAUGAAGUAdCAUdCdCAUU-dTdT-3' (SEQ ID NO:305)
(4) B037-25-dG-S sense:
   Nucleic acid in which all ribonucleotide G bases of the B037 sense strand were converted into deoxyribonucleotides.
   5'-dGAdGAUAdGUdGAUdGAAdGUACAUCCAUU-dTdT-3' (SEQ ID NO:306)
(5) B037-25-dGC-S sense:
   Nucleic acid in which all ribonucleotide G and C bases of the B037 sense strand were converted into deoxyribonucleotides.
   5'-dGAdGAUAdGUdGAUdGAAdGUAdCAUdCdCAUU-dTdT-3' (SEQ ID NO:307)
(6) B037-25-10D sense:
   Nucleic acid in which 10 bases at the 3'-terminal of the B037 sense strand were converted into deoxyribonucleotides (U change to T).
   5'-GAGAUAGUGAUGAAGUAdCdAdTdCdCdAdTdT-dTdT-3' (SEQ ID NO:308)
(7) B037-25-14D sense:
   Nucleic acid in which 14 bases at the 3'-terminal of the B037 sense strand were converted into deoxyribonucleotides (U change to T).
   5'-GAGAUAGUGAUGAdAdGdTdAdCdAdTdCdCdAdTdT-dTdT-3' (SEQ ID NO:309)
(8) B037-25-18D sense:
   Nucleic acid in which 18 bases at the 3'-terminal of the B037 sense strand were converted into deoxyribonucleotides (U change to T).
   5'-GAGAUAGUGdAdTdGdAdAdGdTdAdCdAdTdCciCdAdTdT-dTdT-3' (SEQ ID NO:310)
B037-25 anti-sense strand line:
(1) B037-25 anti-sense:
   5'-AAUGGAUGUACUUCAUCACUAUCUC-dTdT-3' (SEQ ID NO:319)

The pairs of the sense strands selected from the above B037-25 sense strand line and the B037-25 anti-sense strands were used as oligo double-stranded RNAs for evaluation,

The combinations of the sense strands and the anti-sense strands constituting the oligo double-stranded RNAs used are shown in Table 13.

The inhibitory activity of these oligo double-stranded RNAs for the expression of Bcl-2 protein was evaluated by Western blotting as described in Example 1.

**[Table 13]**

| Table 13: the inhibitory activity on the expression of Bcl-2 protein of the oligo double-stranded RNA, wherein parts of the ribonucleotides of the sense strand are replaced with deoxyribonucleotides | | | |
|---|---|---|---|
| oligo double-stranded RNA | sense strand / anti-sense strand | sequence (SEQ ID NO:) | activity |
| B037-25 | sense strand | GAG AUA GUG AUG AAG UAC AUC CAU UdTdT (SEQ ID NO:288) | ++ |
| | anti-sense strand | AAU GGA UGU ACU UCA UCA CUA UCU CdTdT (SEQ ID NO:319) | |
| B037-25-dC-S | sense strand | GAG AUA GUG AUG AAG UAdC AUdC dCAU UdTdT (SEQ ID NO:305) | ++ |
| | anti-sense strand | AAU GGA UGU ACU UCA UCA CUA UCUCdTdT (SEQ ID NO:319) | |
| B037-25-dG-S | sense strand | dGAdG AUA dGUdG AUdG AAdG UAC AUC CAU UdTdT (SEQ ID NO:306) | ++ |
| | anti-sense strand | AAU GGA UGU ACU UCA UCA CUA UCU CdTdT (SEQ ID NO:319) | |
| B037-25-dGC-S | sense strand | dGAdG AUA dGUdG AUdG AAdG UAdC AUdC dCAU UdTdT (SEQ ID NO:307) | ++ |
| | anti-sense strand | AAU GGA UGU ACU UCA UCA CUA OCU CdTdT (SEQ ID NO:319) | |
| B037-25-10D | sense strand | GAG AUA GUG AUG AAG UAdC dAdTdC dCdAdT dTdTdT (SEQ ID NO:308) | ++ |
| | anti-sense strand | AAU GGA UGU ACU UCA UCA CUA UCU CdTdT (SEQ ID NO:319) | |
| B037-25-14D | sense strand | GAG AUA GUG AUG AdAdG dTdAdC dAdTdC dCdAdT dTdTdT (SEQ ID NO:309) | ++ |
| | anti-sense strand | AAU GGA UGU ACU UCA UCA CUA UCU CdTdT (SEQ ID NO:319) | |
| B037-25-18D | sense strand | GAG AUA GUG dAdTdG dAdAdG dTdAdC dAdTdC dCdAdT dTdTdT (SEQ ID NO:310) | + |
| | anti-sense strand | AAU GGA UGU ACU UCA UCA CUA UCU CdTdT (SEQ ID NO:319) | |
| B037DNA25 | sense strand | dGdAdG dAdTdA dGdTdG dAdTdG dAdAdG dTdAdC dAdTdC dCdAdT dTdTdT (SEQ ID NO:311) | +- |
| | anti-sense strand | AAU GGA UGU ACU UCA UCA CUA UCU CdTdT (SEQ ID NO:319) | |

It was elucidated that Bcl-2 protein was almost completely inhibited by the oligo double-stranded RNAs as shown in Table 13 when transfected at 100 nM, and even in transfection at 10 nM, further the expression of Bcl-2 protein was almost completely inhibited (Fig. 17).

These results indicate that the substitution of a part of the sense strand constituting oligo double-stranded RNAs with deoxyribonucleotides has no large influence on the inhibitory activity on the expression of Bcl-2 protein. Thus, it was found that these oligo double-stranded RNAs in which the sense strand was partially substituted by DNA could also be utilized in inhibiting the expression of Bcl-2 protein.

### Example 21: Effect of oligo double-stranded RNAs in which all of the phosphoric diester linkages are phosphorothioates

Using oligo double-stranded RNAs having the sense strand in which all of ribonucleotides were replaced with deoxyribonucleotides and all of the phosphoric diester linkages were converted into phosphorothioate linkages, the influence of the oligo double-stranded RNAs on an inhibitory activity on the expression of Bcl-2 protein was evaluated.

The nucleic acids constituting oligo double-stranded RNAs used in evaluation were prepared by replacing all ribonucleotides constituting the sense strands constituting B037-25 oligo double-stranded RNAs with deoxyribonucleotides and converting all of the phosphoric diester linkages into phosphorothioate linkages.

The followings show specific examples of the sequences.
B037-25 sense strand line:
(1) B037-25 sense:
   5'-GAGAUAGUGAUGAAGUACAUCCAUU-dTdT-3' (SEQ ID NO:288)
(2) B037DNA25 sense:
   Nucleic acid in which all bases of the B037 sense strand were converted into deoxyribonucleotides (U change to T).
   5'-dGdAdGdAdTdAdGdTdGdAdTdGdAdAdGdTdAdCdAdTdCdCdAdTdT-dTdT-3' (SEQ ID NO:311)
(3) B037DNA25PS sense:
   Nucleic acid in which all bases of the B037 sense strand were converted into deoxyribonucleotides and all of the phosphoric diester linkages were phosphorothioates (U change to T).
   5'-dGdAdGdAdTdAdGdTdGdAdTdGdAdAdGdTdAdCdAdTdCdCdAdTdT-dTdT-3' (SEQ ID NO:318)
B037-25 anti-sense strand line:
(1) B037-25 anti-sense:
   5'-AAUGGAUGUACUUCAUCACUAUCUC-dTdT-3'' (SEQ ID NO:319)

Specifically, the examined oligo double-stranded RNAs and the results are shown in Table 14. The inhibitory activity of these oligo double-stranded RNAs for the expression of Bcl-2 protein was evaluated by Western blotting as described in Example 15.

**[Table 14]**

| Table 14: the inhibitory activity on the expression of Bcl-2 protein of the oligo double-stranded RNA in which the bases and the phosphoric diester linkages are converted. | | | |
|---|---|---|---|
| oligo double-stranded RNA | sense strand / anti-sense strand | sequence (SEQ ID NO:) | activity |
| B037-25 | sense strand anti-sense strand | GAG AUA GUG AUG AAG UAC AUC CAU UdTdT (SEQ ID NO:288) | ++ |
| | | AAU GGA UGU ACU UCA UCA CUA UCU CdTdT (SEQ ID NO:319) | |
| B037DNA25 | sense strand | dGdAdG dAdTdA dGdTdG dAdTdG dAdAdG dTdAdC dAdTdC dCdAdT dTdTdT (SEQ ID NO:311) | +- |
| | anti-sense strand | AAU GGA UGU ACU UCA UCA CUA UCU CdTdT (SEQ ID NO:319) | |
| B037DNA25PS | sense strand | dGdAdG dAdTdA dGdTdG dAdTdG dAdAdG dTdAdC dAdTdC dCdAdT dTdTdT (SEQ ID NO:318)*1 | +- |
| | anti-sense strand | AAU GGA UGU ACU UCA UCA CUA UCU CdTdT (SEQ ID NO:319) | |

| | | | |
|---|---|---|---|
| *1: all of the phosphoric diester linkages are phosphorothioates. | | | |

It was elucidated that Bcl-2 protein was slightly inhibited by the oligo nucleic acid RNAs as shown in Table 14 when transfected at 100 nM, and even in transfection at 10 nM, further the expression of Bcl-2 protein was slightly inhibited (Fig. 18).

On the basis of these results, it was proved that the oligo double-stranded RNAs of which their constituting sense strand was entirely replaced with deoxyribonucleotides and all of the phosphoric diester linkages were converted into phosphorothioate linkages, also showed an inhibitory activity on the expression of Bcl-2 protein. Thus, it was found that the oligo double-stranded RNAs of which the constituent sense strand was entirely replaced by deoxyribonucleotides and all of the phosphoric diester linkages were converted into phosphorothioate linkages could also be utilized in inhibiting the expression of Bcl-2 protein.

### Industrial Applicability

The present invention contributes to provide oligo double-stranded RNAs which have a high inhibitory activity on the expression of Bcl-2 protein. The present invention contributes to provide pharmaceutical compositions for treatment and/or prevention of diseases caused by over-expression of Bcl-2 protein, such as cancer, and further contributes to the development of a method for treating and/or preventing these diseases.
Furthermore, the present application is directed to the following embodiments:
**1.** An oligo double-stranded RNA, which comprises a double-strand forming portion of 15 to 19 base pairs which is a pair of a sense RNA strand of one sequence selected from SEQ ID NO:1 to SEQ ID NO:81. SEQ ID NO:240 to SEQ ID NO:256, and SEQ ID NO:274 to SEQ ID NO:280 from which 2 dT bases at the 3'-terminal are excluded, and a complementary anti-sense RNA strand of one sequence selected from SEQ ID NO:82 to SEQ ID NO:162, SEQ ID NO:257 to SEQ ID NO:273, and SEQ ID NO:281 to SEQ ID NO:287 from which 2 dT bases at the 3'-terminal are excluded; of which pair a total of up to 4 base pairs may further be excluded from either one or both terminals.
**2.** The oligo double-stranded RNA according to 1, which contains one or more bases of deletion, substitution, insertion or addition in the sequence of at least one of the RNA strands in the double-strand forming portion, and retains an inhibitory activity on the expression of Bcl-2 protein.
**3.** The oligo double-stranded RNA according to 1, wherein a part of ribonucleotides constituting at least one of the RNA strands or the whole sense RNA strand in the double-strand forming portion is replaced with deoxyribonucleotides or modified nucleotides, said oligo double-stranded RNA retaining an inhibitory activity on the expression of Bcl-2 protein.
**4.** The oligo double-stranded RNA according to any one of 1 to 3, wherein the double-strand forming portion is a pair of RNAs of SEQ ID NO;11 and SEQ ID NO:92, SEQ ID NO:30 and SEQ ID NO:111, SEQ ID NO:36 and SEQ ID NO:117, SEQ ID NO:43 and SEQ ID NO:124, SEQ ID NO:55 and SEQ ID NO:136, SEQ ID NO:62 and SEQ ID NO:143, or SEO ID NO:77 and SEQ ID NO:158 from which 2 dT bases at the respective 3'-terminals are excluded.
**5.** The oligo double-stranded RNA according to any one of 1 to 4, wherein a 1-base to 4-base nucleotide as an overhang is added to the 3'-terminal of at least one of the RNA strands or 5'-terminal of at least one of the RNA strands in the double-strand forming portion.
**6.** The oligo double-stranded RNA according to 5, wherein the 1-base to 4-base nucleotide added to the 3'-terminal or 5'-terminal as the overhang is a deoxyribonucleotide.
**7.** The oligo double-stranded RNA according to 5, wherein the nucleotide added to the 3'-terminal as the overhang is dTdT.
**8.** The oligo double-stranded RNA according to any one of 1 to 7, wherein at least a part of riboses or a phosphate backbone constituting the nucleotide of at least one of the RNA strands are modified.
**9.** The oligo double-stranded RNA according to 8, wherein the modification of the ribose or phosphate backbone comprises one or more modifications selected from a modification of a 2'-hydroxyl group of the ribose with substitution by a substituent selected, from H, OR, R, R', OR, SH, SR, NH₂, NHR, NR₂, N₃, ON, F, Cl, Br, and I, wherein R represents alkyl or aryl, and R' represents alkylene; a modification of the ribose to a 4'-thio derivative: and a modification of the phosphate backbone to a phosphorothioate, a phosphorodithioate, an alkyl phosphonate, or a phosphoroamidate.
**10.** The oligo double-stranded RNA according to any one of 1 to 9, which is capable of inhibiting the expression of Bcl-2 protein in comparison with the case in which the oiigo double-stranded RNA is not present, after transfection of a cell.
**11.** A nucleic acid, which comprises an RNA of one sequence selected from SEQ ID NO:1 to SEQ ID NO:162 and SEQ ID NO:240 to SEQ ID NO:287 from which 2 dT bases at the 3'-terminal are excluded.
**12.** The nucleic acid according to 11, which contains one or more bases of deletion, substitution, insertion or addition in the sequence of RNA, and retains an inhibitory activity on the expression of Bcl-2 protein when the nucleic acid constitutes an oligo double-stranded RNA.
**13.** The nucleic acid according to 11, wherein a part of the ribonucleotide of the RNA is replaced with deoxyribonucleotides or modified nucleotides.
**14.** The nucleic acid according to any one of 11 to 13, to which a 1-base to 4-base nucleotide is added at the 3'-terminal or 5'-terminal of the RNA.
**15.** The nucleic acid according to 14, wherein the 1-base to 4-base nucleotide added to the 3'-terminaf or 5'-terminal is a deoxyribonucleotide.
**16.** The nucleic acid according to 14, wherein the nucleotide added to the 3'-terminal is dTdT.
**17.** A nucleic acid which has one sequence selected from SEQ ID NO:163 to SEQ ID NO:196 and SEQ ID NO:198 to SEQ ID NO:239.
**18.** The nucleic acid according to any one of 11 to 17, wherein a part or all of riboses or a phosphate backbone constituting a nucleotide strand are modified.
**19.** A nucleic acid, which has one sequence selected from SEQ ID NO:1 to SEQ ID NO:162 from which 2 dT bases at the 3'-terminal are excluded and in which uridine (U) is thymine (T), as a deoxyribonucleotide.
**20.** The nucleic acid according to 19, which contains one or more bases of deletion, substitution, insertion or addition in the sequence, said nucleic acid expressing an RNA having an inhibitory activity on the expression of Bcl-2 protein when the nucleic acid is used as a template to prepare the oligo double-stranded RNA.
**21.** A pharmaceutical composition which comprises a complex comprising a carrier which is effective in introducing an oligo double-stranded RNA into a cell and the oligo double-stranded RNA according to any one of 1 to 9.
**22.** The pharmaceutical composition according to 21, wherein the carrier which is effective in introducing an oligo double-stranded RNA into a cell is a cationic carrier.
**23.** The pharmaceutical composition according to 21 or 22, which comprises 1 to 200 parts by weight of the carrier to 1 part by weight of the oligo doubie-stranded RNA.
**24.** The pharmaceutical composition according to 21 or 22, which comprises 2.5 to 100 parts by weight of the carrier to 1 part by weight of the oiigo double-stranded RNA.
**25.** The pharmaceutical composition according to 21 or 22, which comprises 10 to 20 parts by weight of the carrier to 1 part by weight of the oligo double-stranded RNA.
**26.** A pharmaceutical composition, which comprises a nucleic acid for the preparation of an oligo double-stranded RNA comprising the nucleic acid according to 19 or 20.
**27.** The pharmaceutical composition according to any one of 21 to 26, which is used for treating and/or preventing a disease for which inhibition of the expression of Bcl-2 protein is desired.
**28.** The pharmaceutical composition according to 27, which is used for treating and/or preventing a disease for which acceleration of apoptosis is desired.
**29.** The pharmaceutical composition according to 27, which is used for treating and/or preventing a cancer.
**30.** The pharmaceutical composition according to 27, which is used for treating and/or preventing a hematological malignant disease.
**31.** An oligo double-stranded RNA, which comprises as a double-strand forming portion, a double-stranded RNA comprising
   a sense RNA strand of one sequence selected from SEQ ID NO:288 and SEQ ID NO:295 to SEQ ID NO:300 from which 2 dT bases at the 3'-terminal and 6 bases at the 5'-terminal are excluded, and to which subsequently a total of 6 bases are added at the 3'-terminal and/or the 5'-terminal, said sense RNA strand corresponding to a part of bcl-2 mRNA; and
   a complementary anti-sense RNA strand of one sequence selected from SEQ ID NO:319 and SEQ ID NO:326 to SEQ ID NO:331 from which 2 dT bases and the subsequent 6 bases at the 3'-terminal are excluded, and to which subsequently a total of 6 bases are added at the 3'-terminal and/or 5'-terminal, said anti-sense RNA strand being complementary to the part of bcl-2 mRNA.
**32.** An oligo double-stranded RNA, which comprises as a double-strand forming portion, a double-stranded RNA comprising
   a sense RNA strand of one sequence selected from SEQ ID NO:288 to SEQ ID NO:300 from which 2 dT bases at the 3'-terminal are excluded, and to which a total of 2 bases are added at the 3'-terminal and/or 5'-terminal, said sense RNA strand corresponding to a part of bcl-2 mRNA; and
   a complementary anti-sense RNA strand of one sequence selected from SEQ ID NO:319 to SEQ ID NO:331 from which 2 dT bases at the 3'-terminal are excluded, and to which a total of 2 bases are added at the 3'-terminal and/or 5'-terminal, said anti-sense RNA strand being complementary to the part of bcl-2 mRNA.
**33.** The oligo double-stranded RNA according to 31 or 32, which contains one or more bases of deletion, substitution, insertion or addition in the sequence of at least one of the RNA strands of the double-strand forming portion, and retains an inhibitory activity on the expression of Bcl-2 protein.
**34.** The oligo double-stranded RNA according to any of 31 to 33, wherein the double-strand forming portion is a pair of RNAs of SEQ ID NO:302 and SEQ ID NO:333, SEQ ID NO:303 and SEQ ID NO:332, or SEQ ID NO: 304 and SEQ ID NO:334.
**35.** The oligo double-stranded RNA according to 31, wherein a part of ribonucleotides constituting at least one of the RNA strands or the whole sense RNA strand in the double-strand forming portion is substituted by deoxyribonucleotides or modified nucleotides, said oligo double-stranded RNA retaining an inhibitory activity on the expression of Bcl-2 protein.
**36.** The oligo double-stranded RNA according to any of 31 to 35, wherein the double-strand forming portion is a pair of RNAs of SEQ ID NO:288 and SEQ ID NO:319, SEQ ID NO:289 and SEQ ID NO:320, SEQ ID NO:290 and SEO ID NO:321, SEO ID NO:291 and SEQ ID NO:322, SEQ ID NO:292 and SEQ ID NO:323, SEQ ID NO:293 and SEQ ID NO:324, SEQ ID NO:294 and SEQ ID NO:325, SEQ ID NO:295 and SEQ ID NO:326, SEQ ID NO:296 and SEQ ID NO:327, SEQ ID NO:297 and SEQ ID NO:328, SEQ ID NO:298 and SEQ ID NO:329, SEQ ID NO:299 and SEQ ID NO:330, SEQ ID NO:300 and SEQ ID NO:331, SEQ ID NO:305 and SEQ ID NO:319, SEQ ID NO: 306 and SEQ ID NO:319, SEQ ID NO:307 and SEQ ID NO:319, SEQ ID NO:308 and SEQ ID NO:319, SEQ ID NO: 309 and SEQ ID NO:319, SEQ ID NO:310 and SEQ ID NO:319, SEQ ID NO:311 and SEQ ID NO:319, SEQ ID NO: 312 and SEQ ID NO:320, SEQ ID NO:313 and SEQ ID NO:321, SEQ ID NO:314 and SEQ ID NO:322, SEQ ID NO: 315 and SEQ ID NO:323, SEQ ID NO:316 and SEQ ID NO:324, or SEQ ID NO:317 and SEQ ID NO:325 from which 2 dT bases at the respective 3'-terminals are excluded.
**37.** The oligo double-stranded RNA according to any one of 31 or 34 to 36, wherein a 1-base to 4-base nucleotide as an overhang is added to the 3'-terminal or 5'-terminal of at least one RNA of the double-strand forming portion comprising the sense RNA strand and the anti-sense RNA strand.
**38.** The oligo double-stranded RNA according to 37, wherein the 1-base to 4-base nucleotide added to the 3'-terminai or 5'-terminal as the overhang is a deoxyribonucleotide
**39.** The oligo double-stranded RNA according to 37, wherein the nucleotide added to the 3'-terminal as the overhang is dTdT.
**40.** The oligo double-stranded RNA according to 37, wherein the nucleotide added as an overhang at the 3'-terminal of the sense RNA strand has, following the double-strand forming portion, a sequence having 1 to 4 bases identical with the bcl-2 mRNA, and the nucleotide added as an overhang at the 3'-terminal of the anti-sense RNA strand has, following the double-strand forming portion, a sequence having 1 to 4 bases complementary to bcl-2 mRNA.
**41.** The oligo double-stranded RNA according to 40, which is a pairof RNAs of SEQ ID NO:301 and SEQ ID NO:332.
**42.** The oligo double-stranded RNA according to any one of 31 to 40, wherein at least a part or all of riboses or a phosphate backbone constituting the nucleotide of at least one of the RNA strands are modified.
**43.** The oligo double-stranded RNA according to 42, wherein the modification of the riboses or phosphate backbone comprises one or more modifications selected from a modification of a 2'-hydroxyl group of the ribose with substitution by a substituent selected from H, OR, R, R', OR, SH, SR, NH₂, NHR, NR₂, N₃, CN, F, Cl, Br, and I, wherein R represents alkyl or aryl, and R' represents alkylene; a modification of the ribose to a 4'-thio derivative; and a modification of the phosphate backbone to a phosphorothioate, a phosphorodithioate, an alkyl phosphonate, or a phosphoroamidate.
44. The oligo doubie-stranded RNA according to 43, wherein the double-strand forming portion is a pair of RNAs of SEQ ID NO:318 and SEQ ID NO:319.
45. The oligo double-stranded RNA according to any one of 31 to 44, which is capable of inhibiting the expression of the Bcl-2 protein in comparison with the case in which the oligo double-stranded RNA is not present, in the course of transfection of a cell.
46. A nucleic acid having one sequence selected from SEQ ID NO:288 and SEQ ID NO:295 to SEQ ID NO:300 from which 2 dT bases at the 3'-terminal and 6 bases at the 5'-terminal are excluded, and to which a total of 6 bases are added to the 3'-terminal and/or the 5'-terminal, said sequence corresponding to a part of bcl-2 mRNA.
47. A nucleic acid having one sequence selected from SEQ ID NO:319 and SEQ ID NO:326 to SEQ ID NO:331 from which 2 dT bases and subsequent 6 bases at the 3'-terminal are excluded, and to which a total of 6 bases are added to the 3'-terminal and/or 5'-terminal, said sequence being complementary to a part of bcl-2 mRNA.
48. A nucleic acid having one sequence selected from SEQ ID NO:288 to SEQ ID NO:300 from which 2 dT bases at the 3'-terminal are excluded, and to which a total of 2 bases are added to the 3'-terminal and/or 5'-terminal, said sequence corresponding to a part of bcl-2 mRNA.
49. A nucleic acid having one sequence selected from SEQ ID NO:319 to SEQ ID NO:331 from which 2 dT bases at the 3'-terminal are excluded, and to which a total of 2 bases are added to the 3'-terminal and/or 5'-terminal, said sequence being complementary to a part of bcl-2 mRNA.
50. The nucleic acid according to any one of 46 to 49, which contains one or more bases of deletion, substitution, insertion or addition in the sequence of RNA, and retains an inhibitory activity on the expression of Bcl-2 protein when the nucleic acid constitutes an oligo double-stranded RNA.
51. The nucleic acid according to 46. wherein a part of ribonucleotides of the RNA is replaced with deoxyribonucleotides or modified nucleotides.
52. The nucleic acid according to 51, which has one sequence selected from SEQ ID NO:305 to SEQ ID NO:317.
53. The nucleic acid according to any one of 46 to 52, to which a 1-base to 4-base nucleotide is added at the 3'-terminal or 5'-terminal of the RNA.
54. The nucleic acid according to 53, wherein the 1-base to 4-base nucleotide added to the 3'-terminal or 5'-terminal of the RNA is a deoxyribonucieotide.
55. The nucleic acid according to 53, wherein the nucleotide added to the 3'-terminaf of the RNA is dTdT.
56. The nucleic acid according to 53, wherein the nucleotide added to the 3'-terminal of the RNA has, following the double-strand forming portion, a sequence identical with the bcl-2 mRNA, or, following the double-strand forming portion, a sequence complementary to the bcl-2 mRNA.
57. The nucleic acid according to 56, which has a sequence of SEQ ID NO:301 or SEQ ID NO:332.
58. The nucleic acid according to any one of 46 to 57, wherein a part or all of riboses or a phosphate backbone constituting the nucleotide strand are modified.
59. A nucleic acid comprising a sequence selected from SEO ID NO:288 to SEQ ID NO:300 and SEQ ID NO:319 to SEQ ID NO:331 from which 2 dT bases at the 3'-terminal are excluded and in which uridine (U) is thymine (T), as a deoxyribonucleotide.
60. The nucleic acid according to 59, which contains one or more bases of deletion, substitution, insertion or addition in the sequence, said nucleic acid expressing an RNA having an inhibitory activity on the expression of Bcl-2 protein when the nucleic acid is used as a template to prepare the oligo double-stranded RNA.
61. A pharmaceutical composition which comprises a complex comprising a carrier which is effective in introducing an oligo double-stranded RNA into a cell and the oligo double-stranded RNA according to any one of 31 to 45.
62. The pharmaceutical composition according to 61, wherein the carrier which is effective in introducing an oligo double-stranded RNA into a cell is a cationic carrier.
63. The pharmaceutical composition according to 61 or 62, which comprises 1 to 200 parts by weight of the carrier to 1 part by weight of the oligo double-stranded RNA.
64. The pharmaceutical composition according to 61 or 62. which comprises 2.5 to 100 parts by weight of the carrier to 1 part by weight of the oligo double-stranded RNA.
65. The pharmaceutical composition according to 61 or 62, which comprises 10 to 20 parts by weight of the carrier to 1 part by weight of the oligo double-stranded RNA.
66. A pharmaceutical composition which comprises a nucleic acid for the preparation of an oligo double-stranded RNA comprising the nucleic acid according to 59 or 60.
67. The pharmaceutical composition according to 63 or 64, which is used for treating and/or preventing a disease for which inhibition of the expression of Bcl-2 protein is desired.
68. The pharmaceutical composition according to 67, which is used for treating and/or preventing a disease for which promotion of apoptosis is desired.
69. The pharmaceutical composition according to 67, which is used for treating and/or preventing a cancer.
70. The pharmaceutical composition according to 67, which is used for treating and/or preventing a hematological malignant disease.
71. A method for screening an oligo double-stranded RNA having an inhibitory activity on the expression of Bcl-2 protein, comprising:
   1) using as a screening target a group of oligo double-stranded RNAs, each comprising a double-strand forming portion of 25 to 27 base pairs comprising double-strand forming portion of 19 base pairs of an oligo double-stranded RNA composed of SEQ ID NO:11 and SEQ ID NO:92, SEQ ID NO:30 and SEQ ID NO:111, SEQ ID NO:36 and SEQ ID NO:117, SEQ ID NO:43 and SEQ ID NO:124, SEQ ID NO:55 and SEQ ID NO:136, SEQ ID NO: 62 and SEQ ID NO:143, or SEQ ID NO:77 and SEQ ID NO:158; and
   2) evaluating a decrease of the amount of bcl-2 mRNA or an inhibition of the expression of Bcl-2 protein, after transfection of a cell with the oligo double-stranded RNA obtained in 1) above.

## Claims

1. An oligo double-stranded RNA, comprising a double-strand forming portion of 15-19 base pairs which is formed by pairing a sense RNA strand of a base sequence of SEQ ID NO. 11 with the elimination of 2 dT bases from the 3'-terminal, and an anti-sense RNA strand of a base sequence of SEQ ID NO. 92 with the elimination of 2 dT bases from the 3'-terminal, wherein a total of up to 4 base pairs may further be eliminated from either one or both ends of thus formed double strand.

2. An oligo double-stranded RNA according to claim 1, wherein either one or both strands in the double-strand forming portion contain one or more nucleotides of deletion, substitution, insertion, or addition, with a retention of an inhibitory activity on the expression of Bcl-2 protein.

3. An oligo double-stranded RNA according to claim 1, wherein a part(s) of ribonucleotides from either or both strands in the double-strand forming portion or the whole sense RNA strand according to claim 1 are replaced with a deoxyribonucleotide(s) or a modified nucleotide(s), with a retention of an inhibitory activity on the expression of Bcl-2 protein.

4. The oligo double-stranded RNA according to any one of claims 1 to 3, wherein 1 to 4 nucleotides as an overhang are added to the 3'-terminal or 5'-terminal of either one or both strands of the double-strand forming portion.

5. The oligo double-stranded RNA according to claim 4, wherein 1 to 4 nucleotides added as an overhang at the 3'-terminal or 5'-terminal are a deoxyribonucleotide(s).

6. The oligo double-stranded RNA according to claim 4, wherein the added nucleotides as an overhang at the 3'-terminal are dTdT.

7. An oligo double-stranded RNA according to any one of claims 1 to 6, wherein at least a part of riboses or phosphate backbone of the nucleotides from either one or both RNA strands in the double-strand forming portion is modified.

8. The oligo double-stranded RNA according to claim 7, wherein the modification of a ribose or phosphate backbone includes a single or a combined modifications selected from a modification of the 2'-hydroxyl group of ribose by substitution with a substituent selected from H, OR, R, R', OR, SH, SR, NH2, NHR, NR2, N3, CN, F, Cl, Br, and I (wherein "R" represents an alkyl or aryl, and "R"' represents an alkylene), a modification of ribose to 4'-thio derivative, and a modification of phosphate backbone giving phosphorothioate, phosphorodithioate, alkyl phosphonate, or phosphoroamidate.

9. A pharmaceutical composition which comprises a complex comprising a carrier effective in introducing an oligo double-stranded RNA into a cell and an oligo double-stranded RNA according to any one of claims 1 to 8.

10. The pharmaceutical composition according to claim 9, wherein the carrier effective in introducing an oligo double-stranded RNA into a cell is a cationic carrier.

11. The pharmaceutical composition according to claim 9, wherein the carrier effective in introducing an oligo double-stranded RNA into a cell is a cationic carrier comprising 2-O-(2-diethylaminoethyl) carbamoyl-1,3-O-dioleoylglycerol.

12. The pharmaceutical composition according to any one of claims 9 to 11, which comprises 1-200 parts by weight of the carrier to 1 part by weight of oligo double-stranded RNA, 2.5-100 parts by weight of the carrier to 1 part by weight of oligo double-stranded RNA or 10-20 parts by weight of the carrier to 1 part by weight of oligo double-stranded RNA.

13. The pharmaceutical composition according to any one of claims 9 to 12, for use in treating and/or preventing diseases for which inhibition of the expression of Bcl-2 protein is desired.

14. Pharmaceutical composition according to claim 13 for use in treating and/or preventing diseases for which acceleration of apoptosis is desired.

15. Pharmaceutical composition according to claim 13 for use in treating and/or preventing cancers or for use in treating and or preventing hematological malignant diseases.
